# EUROPÄISCHE PATENTANMELDUNG

(11) **EP 4 585 135 A1**
(43) Veröffentlichungstag der Anmeldung: **16.07.2025**
(21) Anmeldenummer: 25151231.5
(22) Anmeldetag: 10.01.2025
(51) Int. Cl.: A61B 1/00, A61B 90/00, G06F 3/01, G16H 40/63

(54) **MEDIZINISCHES SYSTEM, VERFAHREN ZUM BETRIEB EINES MEDIZINISCHEN SYSTEMS UND VERFAHREN ZUM KOPPELN EINES MEDIZINISCHEN BILDGEBUNGSINSTRUMENTS MIT EINER ANZEIGEVORRICHTUNG**

(30) Priorität: 12.01.2024 DE 102024100946
(71) Anmelder: Karl Storz SE & Co. KG, 78532 Tuttlingen (DE)
(72) Erfinder: Wenzler, Sebastian, 78532 Tuttlingen (DE); Schmelzer, Mark, 78532 Tuttlingen (DE); Clemens, Gerald, 78532 Tuttlingen (DE); Bühler, Clemens, 78532 Tuttlingen (DE); Gerlach, Irina, 78532 Tuttlingen (DE)

(57) **Zusammenfassung**

Die vorliegende Erfindung betrifft ein medizinisches System (10) umfassend ein medizinisches Bildgebungsinstrument (12), das dazu eingerichtet ist, Bilddaten zu erzeugen und die Bilddaten kabellos zu übertragen, eine Anzeigevorrichtung (40), die dazu eingerichtet ist, Bilddaten zu empfangen und eine Darstellung (44) für einen Benutzer zu erzeugen, und eine Gestenerkennungseinheit (76), die dazu eingerichtet ist, eine auf die Anzeigevorrichtung (40) gerichtete Geste des Benutzers zu erkennen und nach Maßgabe der Erkennung der Geste eine Kopplung zwischen dem Bildgebungsinstrument (12) und der Anzeigevorrichtung (40) herzustellen Die vorliegende Erfindung betrifft zudem einen Datenübertragungsadapter, ein Verfahren zum Nachrüsten eines medizinischen Bildgebungsinstruments und ein Verfahren zum Übertragen von Bilddaten.

Ferner betrifft die vorliegende Erfindung ein Verfahren zum Betrieb eines medizinischen Systems (10) und ein Verfahren zum Koppeln eines medizinischen Bildgebungsinstruments (12) mit einer Anzeigevorrichtung (40).

## Beschreibung

Die vorliegende Erfindung betrifft ein medizinisches System, ein Verfahren zum Betrieb eines medizinischen Systems und ein Verfahren zum Koppeln eines medizinischen Bildgebungsinstruments mit einer Anzeigevorrichtung.

Ein Endoskop ist ein medizinisches Bildgebungsinstrument, das dazu dient, anatomische Strukturen in einer Kavität eines Patienten zu untersuchen. Lediglich ein verhältnismäßig kleiner bildgebender Abschnitt des Endoskops muss dazu über einen Zugang der Kavität des Patienten zugeführt werden. Die inneren Strukturen können daraufhin von medizinischem Personal, beispielsweise einem Arzt, betrachtet werden, ohne dass ein invasiver chirurgischer Eingriff erforderlich ist. Auf diese Weise ist es möglich, Körperhöhlen, Organe und andere Hohlstrukturen effizient visuell zu untersuchen.

Mit der Zeit wurden unterschiedliche Arten von Endoskopen entwickelt, die den Bedürfnissen verschiedener medizinischer Fachbereiche gerecht werden. Beispielsweise gibt es spezielle Endoskope für das Atemweg-Management zur Sicherstellung der Atemwege, in der Gastroenterologie zur Untersuchung von Magen, Darm und anderen Teilen des Verdauungstrakts, für die Bronchoskopie zur Untersuchung der Atemwege, für die Laparoskopie, um zur Durchführung minimalinvasiver Chirurgie einen Einblick in den Bauchraum zu bekommen, und für die Orthopädie zur Untersuchung von Gelenken und Sehnen.

Typischerweise verfügen Endoskope über eine Kamera, die die anatomischen Strukturen abbildet und Bilddaten erzeugt. Diese Bilddaten werden an eine Anzeigevorrichtung übertragen, welche eine Darstellung anhand der erfassten Bilddaten erzeugt. Dadurch kann das medizinische Personal die anatomische Struktur über die Anzeigevorrichtung einsehen oder die Behandlung kann zu Dokumentationszwecken aufgezeichnet werden.

Damit die Kamera einer Kavität des Patienten zugeführt und in dieser positioniert werden kann, weisen viele Endoskope einen länglichen dünnen Schaft auf. Der Schaft wird über den Zugang der Kavität teilweise zugeführt. Beispielsweise wird ein Bronchoskop, ein Endoskop zur Untersuchung der Lunge, über den Mund oder die Nase entlang der Trachea der Lunge zugeführt und innerhalb dieser positioniert. An seinem distalen Ende kann der Schaft eine Optik aufweisen, die Licht von der zu betrachtenden anatomischen Struktur sammelt. Dieses gesammelte Licht wird zu einem Bildsensor der Kamera weitergeleitet, der sich etwa in einem distalen Abschnitt des Schafts oder in einem proximalen Handgriff des Endoskops, an dem der Schaft befestigt ist, befindet. Der Bildsensor wandelt das gesammelte Licht in elektrische Signale um und erzeugt die Bilddaten.

Üblicherweise werden für die Übertragung der Bilddaten von dem Bildsensor zur Anzeigevorrichtung Datenkabel verwendet. Allerdings haben sich Kabel im Einsatz von Endoskopen oft als störend erwiesen. Beispielsweise sind in einem Operationssaal oder einem Rettungswagen viele medizinische Instrumente und Geräte im Einsatz, die jeweils Kabel zur Datenübertragung oder Stromversorgung aufweisen können. Dadurch kann eine Unordnung entstehen und die Kabel sich gegenseitig behindern. In der Folge kann das medizinische Personal in ihrer Bewegungsfreiheit eingeschränkt werden und sich eine verminderte Behandlungsqualität ergeben. Zudem kann sich der Komfort für das medizinische Personal beim Einsatz von Endoskopen reduzieren, wodurch sich eine geringere Akzeptanz für den Einsatz von Endoskopen ergeben kann.

Um diese Einschränkungen zu umgehen, werden vermehrt kabellose Endoskope eingesetzt. Solch ein Endoskop überträgt die Bilddaten kabellos an eine Anzeigevorrichtung, welche eine Darstellung anhand der Bilddaten erzeugt. Allerdings ergeben sich durch den Einsatz kabelloser Endoskope neue Probleme.

Endoskope und auch Anzeigevorrichtungen sind in der Regel einige Jahre lang im Einsatz, um wirtschaftlich betrieben zu werden. In vielen Gesundheitseinrichtungen sind beispielsweise seit mehreren Jahren funktionstüchtige kabelgebundene Endoskope im Einsatz. Unter Umständen können sich Kompatibilitätsprobleme ergeben, wenn beispielsweise eine neue Anzeigevorrichtung angeschafft wird. Die Erfinder haben erkannt, dass ein älteres kabelgebundenes Endoskop möglicherweise nicht mit der neuen Anzeigevorrichtung betrieben werden kann. Dadurch kann sich ergeben, dass mehrere Anzeigevorrichtungen angeschafft werden müssen oder alte noch funktionstüchtige Endoskope ausgemustert werden müssen.

Ferner können sich dadurch Probleme ergeben, dass mehrere verschiedene kabellose Bildgebungsinstrumente gleichzeitig im Einsatz sind oder verschiedene Anzeigevorrichtung vorhanden sind. Die Erfinder haben erkannt, dass ein eingesetztes Bildgebungsinstrument jedoch die Bilddaten an eine bestimmte Anzeigevorrichtung übertragen sollte, die das medizinische Personal für den Einsatz des Bildgebungsinstrument vorgesehen hat. Allerdings kann es vorkommen, dass die Bilddaten an eine andere, falsche Anzeigevorrichtung übertragen werden, wodurch es zu einer Verwechslung kommen kann, welche Anzeigevorrichtung tatsächlich eine Darstellung anhand der Bilddaten des eingesetzten Bildgebungsinstruments erzeugt. Beispielsweise können Bilddaten an eine Anzeigevorrichtung eines benachbarten Operationssaals einer Gesundheitseinrichtung übertragen werden. Dadurch kann der Fall eintreten, dass dem medizinischen Personal eine Darstellung einer anatomischen Struktur eines falschen Patienten angezeigt wird. Die Erfinder haben erkannt, dass dies ein Sicherheitsrisiko darstellen und zu schweren Behandlungsfehlern führen kann. Zudem können in einem Rettungswagen verschiedene Anzeigevorrichtungen zur Verfügung stehen. Dadurch kann sich in Notfallsituationen ergeben, dass zunächst jene Anzeigevorrichtung gesucht werden muss, an die die Bilddaten tatsächlich übertragen werden. Daraus kann sich ein kritischer Zeitverlust ergeben.

Ferner haben die Erfinder erkannt, dass sich durch das Vorhandensein mehrerer Anzeigevorrichtung und kabelloser Instrumente zudem Probleme für den Vorgang des Koppelns eines Bildgebungsinstruments an eine Anzeigevorrichtung oder das Aktivieren von Funktionen des Bildgebungsinstruments ergeben können. Es kann schwierig sein, bestimmte kabellose Instrumente und bestimmte Anzeigevorrichtungen einander zuzuordnen. Insbesondere wenn viele verschiedene kabellose Instrumente im Einsatz sind, kann sich ein fehlerhaftes Koppeln, ein nicht beabsichtigtes Aktivieren von Funktionen und eine falsche Zuordnung ergeben. Das Koppeln und Aktivieren können auch in zeitkritischen Situationen problematisch sein. In solchen Situationen ist es wichtig, zuverlässig, schnell und bediensicher eine Kopplung herstellen zu können und eine Aktivierung von Funktionen durchzuführen, damit ein Patient möglichst schnell behandelt werden kann.

Ferner kann es vorkommen, dass ein Behandler während des Durchführens einer Aktion am Patienten eine Kopplung herstellen möchte. Die Erfinder haben erkannt, dass der Behandler in derartigen Situation womöglich gerade keine freie oder saubere Hand frei hat, um einen Kopplungsvorgang durchführen zu können. Dieses Problem kann auch auftreten, wenn der Behandler beispielsweise eine Anzeigevorrichtung wechseln und/oder hinzuschalten möchte.

Ausgehend vom Stand der Technik sollen Verbesserungen im Bereich der medizinischen Bildgebung ermöglicht werden.

Ausgehend vom Stand der Technik kann der Erfindung die Aufgabe zugrunde liegen, eine Einsatzmöglichkeit und Flexibilität von medizinischen Bildgebungsinstrumenten zu verbessern.

Ausgehend vom Stand der Technik kann der Erfindung ferner die Aufgabe zugrunde liegen, eine Bediensicherheit und/oder eine Betriebssicherheit eines, insbesondere kabellosen, medizinischen Bildgebungsinstruments zu erhöhen.

Ausgehend vom Stand der Technik kann der Erfindung ferner die Aufgabe zugrunde liegen, einen Betrieb eines medizinischen Bildgebungsinstruments sicherer und/oder komfortabler zu gestalten.

Ausgehend vom Stand der Technik kann der Erfindung ferner die Aufgabe zugrunde liegen, einen Betrieb eines medizinischen Bildgebungsinstruments zu vereinfachen.

Zumindest eine dieser Aufgabe wird erfindungsgemäß durch ein medizinisches System, ein Verfahren zum Betrieb eines medizinischen Systems und ein Verfahren zum Koppeln eines medizinischen Bildgebungsinstruments mit einer Anzeigevorrichtung gelöst, wie sie hierin beschrieben und in den Ansprüchen definiert sind. Zudem sind weitere Aspekte der Erfindung beschrieben, die die Erfindung erklären.

Gemäß einem Aspekt kann die vorliegende Erfindung vorsehen, ein medizinisches System bereitzustellen. Das medizinische System kann ein medizinisches Bildgebungsinstrument, das dazu eingerichtet ist, Bilddaten zu erzeugen und das einen Steckeranschluss zum Anschließen eines Datenkabels umfasst, und einen Datenübertragungsadapter, der lösbar mit dem Steckeranschluss verbindbar ist und dazu eingerichtet ist, die Bilddaten über den Steckeranschluss zu empfangen und auf den Bilddaten beruhende Daten kabellos zu übertragen, umfassen.

Gemäß einem Aspekt kann die vorliegende Erfindung zudem vorsehen, einen Datenübertragungsadapter für ein erfindungsgemäßes medizinisches System bereitzustellen.

Gemäß einem Aspekt kann die vorliegende Erfindung zudem vorsehen, einen Datenübertragungsadapter bereitzustellen, insbesondere für ein erfindungsgemäßes medizinisches System. Der Datenübertragungsadapter kann ein Anschlusselement, das lösbar mit einem Steckeranschluss eines medizinischen Bildgebungsinstruments verbindbar ist, eine Steuereinheit, die mit dem Anschlusselement verbunden ist und die dazu eingerichtet ist, Bilddaten von dem Anschlusselement zu empfangen, und eine Datenschnittstelle, die mit der Steuereinheit verbunden ist und die dazu eingerichtet ist, Daten von der Steuereinheit zu empfangen und Daten kabellos zu übertragen, umfassen.

Gemäß einem Aspekt kann die vorliegende Erfindung zudem vorsehen, ein Verfahren zum Nachrüsten eines medizinischen Bildgebungsinstruments, insbesondere mittels eines erfindungsgemäßen medizinischen Systems und/oder mittels eines erfindungsgemäßen Datenübertragungsadapters, bereitzustellen. Das Verfahren zum Nachrüsten kann die Schritte eines Bereitstellens eines medizinisches Bildgebungsinstruments, das dazu eingerichtet ist, Bilddaten zu erzeugen und das einen Steckeranschluss zum Anschließen eines Datenkabels umfasst, eines Bereitstellens eines Datenübertragungsadapters, der lösbar mit dem Steckeranschluss verbindbar ist und dazu eingerichtet ist, die Bilddaten über den Steckeranschluss zu empfangen und kabellos zu übertragen, und eines Verbindens des Datenübertragungsadapters mit dem Steckeranschluss des Bildgebungsinstruments umfassen.

Gemäß einem Aspekt kann die vorliegende Erfindung zudem vorsehen, ein Verfahren zum Übertragen von Bilddaten, insbesondere mittels eines erfindungsgemäßen medizinischen Systems und/oder mittels eines erfindungsgemäßen Datenübertragungsadapters, bereitzustellen. Das Verfahren zum Übertragen von Bilddaten kann die Schritte eines Bereitstellens eines medizinischen Bildgebungsinstruments, das dazu eingerichtet ist, Bilddaten zu erzeugen und das einen Steckeranschluss zum Anschließen eines Datenkabels umfasst, eines Bereitstellens eines Datenübertragungsadapters, der lösbar mit dem Steckeranschluss verbindbar ist und dazu eingerichtet ist, die Bilddaten über den Steckeranschluss zu empfangen und kabellos zu übertragen, eines Verbindens des Datenübertragungsadapters mit dem Steckeranschluss des Bildgebungsinstruments, eines Erzeugens von Bilddaten mittels des Bildgebungsinstruments, eines Übertragens der Bilddaten über den Steckeranschluss zu dem Datenübertragungsadapter und eines kabellosen Übertragens der Bilddaten mittels des Datenübertragungsadapters umfassen.

Gemäß einem Aspekt kann die vorliegende Erfindung zudem vorsehen, ein Verfahren zum Betrieb eines erfindungsgemäßen medizinischen Systems und/oder eines erfindungsgemäßen medizinischen Bildgebungsinstruments bereitzustellen.

Durch diese Merkmale kann eine Einsatzmöglichkeit und Flexibilität eines medizinischen Bildgebungsinstruments verbessert werden. Insbesondere ist durch diese Merkmale ein medizinisches Bildgebungsinstrument zur kabellosen Datenübertragung einrichtbar. Dadurch kann das medizinische Bildgebungsinstrument von einem kabelgebundenen Bildgebungsinstrument in ein kabelloses Bildgebungsinstrument wandelbar sein. Das bedeutet, dass das Bildgebungsinstrument modernisierbar ist. Ein Operationssaal kann beispielsweise mit kabellosen Anzeigevorrichtungen ausgestattet werden und trotzdem können alte, teure Bildgebungsinstrumente weiter betrieben werden. Ein wirtschaftlicher Betrieb einer Gesundheitseinrichtung kann dadurch verbessert werden. Alte Bildgebungsinstrumente können demzufolge mit neuen Anzeigevorrichtungen kompatibel sein und eine Vorwärts- sowie Rückwärtskompatibilität erreicht werden. Das Bildgebungsinstrument bedarf für das Umrüsten zu einem kabellosen Bildgebungsinstrument keiner baulichen oder konstruktiven Änderung und/oder Anpassung. Der Datenübertragungsadapter kann mit dem vorhandenen Steckeranschluss verbunden und/oder betrieben werden. Ferner können auch neue Bildgebungsinstrumente bereitgestellt werden, die zur wahlweise kabellosen und/oder kabelgebundenen Bilddatenübertragung vorsehbar sind. Beispielsweise kann der Datenübertragungsadapter modulhaft vorsehbar sein, wodurch Produktionskosten eingespart werden können.

Gemäß einem weiteren Aspekt kann die vorliegende Erfindung vorsehen, ein medizinisches System bereitzustellen. Das medizinische System kann ein medizinisches Bildgebungsinstrument, das dazu eingerichtet ist, Bilddaten zu erzeugen, eine Anzeigevorrichtung, die dazu eingerichtet ist, Bilddaten zu empfangen und eine Darstellung für einen Benutzer zu erzeugen, eine Kopplungseinheit, die dazu eingerichtet ist, eine Kopplung des Bildgebungsinstruments und der Anzeigevorrichtung herzustellen, wobei in einem gekoppelten Zustand die Bilddaten von dem Bildgebungsinstrument an die Anzeigevorrichtung übertragbar sind, und eine Sicherheitseinheit, die dazu eingerichtet ist, eine Bestätigung der Kopplung durch den Benutzer zu erkennen, wobei die Sicherheitseinheit dazu eingerichtet ist, bei einem Fehlen der Bestätigung der Kopplung durch den Benutzer die Übertragung der Bilddaten von dem Bildgebungsinstrument an die Anzeigevorrichtung zu verhindern, umfassen.

Gemäß einem Aspekt kann die vorliegende Erfindung zudem vorsehen, ein medizinisches Bildgebungsinstrument für ein erfindungsgemäßes medizinisches System bereitzustellen.

Gemäß einem Aspekt kann die vorliegende Erfindung zudem vorsehen, ein medizinisches Bildgebungsinstrument, insbesondere für ein erfindungsgemäßes medizinisches System, bereitzustellen. Das medizinische Bildgebungsinstrument kann eine Kopplungseinheit, die dazu eingerichtet ist, eine Kopplung des Bildgebungsinstruments mit einer Anzeigevorrichtung herzustellen, wobei in einem gekoppelten Zustand die Bilddaten von dem Bildgebungsinstrument an die Anzeigevorrichtung übertragbar sind, und eine Sicherheitseinheit, die dazu eingerichtet ist, eine Bestätigung der Kopplung durch den Benutzer zu erkennen, wobei die Sicherheitseinheit dazu eingerichtet ist, bei einem Fehlen der Bestätigung der Kopplung durch den Benutzer die Übertragung der Bilddaten von dem Bildgebungsinstrument an die Anzeigevorrichtung zu verhindern, umfassen.

Gemäß einem Aspekt kann die vorliegende Erfindung zudem vorsehen, ein Verfahren zum Betrieb eines erfindungsgemäßen medizinischen Systems und/oder eines erfindungsgemäßen medizinischen Bildgebungsinstruments bereitzustellen.

Gemäß einem Aspekt kann die vorliegende Erfindung zudem vorsehen, ein Verfahren zur Verbindung eines medizinischen Bildgebungsinstruments mit einer Anzeigevorrichtung, insbesondere durchgeführt mittels eines erfindungsgemäßen medizinischen Systems und/oder eines erfindungsgemäßen medizinischen Bildgebungsinstruments, bereitzustellen. Das medizinische Bildgebungsinstrument kann dazu eingerichtet sein, Bilddaten zu erzeugen, und die Anzeigevorrichtung, kann dazu eingerichtet sein, Bilddaten zu empfangen und eine Darstellung für einen Benutzer zu erzeugen. Das Verfahren zur Verbindung kann die Schritte eines Herstellens einer Kopplung des Bildgebungsinstruments und der Anzeigevorrichtung, wobei in einem gekoppelten Zustand die Bilddaten von dem Bildgebungsinstrument an die Anzeigevorrichtung übertragbar sind, eines Erkennens einer Bestätigung der Kopplung durch den Benutzer und eines Verhinderns der Übertragung der Bilddaten von dem Bildgebungsinstrument an die Anzeigevorrichtung bei einem Fehlen der Bestätigung der Kopplung durch den Benutzer umfassen.

Durch diese Merkmale kann eine Bediensicherheit und/oder eine Betriebssicherheit eines, insbesondere kabellosen, medizinischen Bildgebungsinstruments erhöht werden. Es kann eine gezielte Paarung eines Bildgebungsinstruments und einer Anzeigevorrichtung erreicht werden. Zudem kann sichergestellt werden, dass das für einen Einsatz vorgesehene Bildgebungsinstrument tatsächlich mit einer bestimmten Anzeigevorrichtung gekoppelt wird. Insbesondere wenn viele verschiedene kabellose Geräte und/oder Instrumente im Einsatz sind, kann trotzdem die gezielte Paarung hergestellt werden. Die Paarung kann durch diese Merkmale zudem benutzerfreundlich und effizient hergestellt werden. In weiterer Folge können dadurch Behandlungsfehler verhindert werden und in Notfallsituationen schnell und zuverlässig eine sichere Paarung herstellbar sein. Die Bestätigung durch den Benutzer gibt dem Benutzer eine Rückmeldung darüber, dass eine richtige, gewollte Kopplung eines bestimmten Bildgebungsinstruments mit einer bestimmten Anzeigevorrichtung hergestellt wird. Der Benutzer kann sich also im Vergleich zu einem herkömmlichen Koppeln sicherer sein, dass die richtige Paarung hergestellt wurde. Dadurch, dass bei einem Ausbleiben der Bestätigung die Übertragung der Bilddaten verhindert wird, kann erreicht werden, dass ein ungewolltes Übertragen von Bilddaten an eine nicht vorgesehene Anzeigevorrichtung ausbleibt. Beispielsweise kann verhindert werden, dass ungewollt Bilddaten an eine Anzeigevorrichtung eines benachbarten Operationssaals übertragen werden. Generell wird dadurch die Betriebssicherheit bei einem, insbesondere gleichzeitigem, Betrieb mehrerer medizinischer Bildgebungsinstrumente erhöht.

Gemäß einem weiteren Aspekt kann die vorliegende Erfindung vorsehen, ein medizinisches System bereitzustellen. Das medizinische System kann ein Objekt, das ein Erkennungsmerkmal aufweist und das anhand des Erkennungsmerkmals identifizierbar ist, ein medizinisches Bildgebungsinstrument mit einer Kamera, mittels derer Bilder erfassbar und Bilddaten erzeugbar sind, wobei mittels der Kamera ein Bild des Erkennungsmerkmals des Objekts erfassbar ist, eine Erkennungseinheit, die dazu eingerichtet ist, das Vorhandensein des Erkennungsmerkmals in dem mittels der Kamera aufgenommenen Bild des Erkennungsmerkmals zu erkennen und nach Maßgabe der Erkennung des Vorhandenseins des Erkennungsmerkmals ein Erkennungssignal zu erzeugen, und eine Steuereinheit, die dazu eingerichtet ist, das Erkennungssignal zu verarbeiten und zumindest eine auf das Objekt bezogene Funktion des medizinischen Systems nach Maßgabe des Erkennungssignals zu aktivieren, umfassen.

Gemäß einem Aspekt kann die vorliegende Erfindung zudem vorsehen, ein medizinisches Bildgebungsinstrument für ein erfindungsgemäßes medizinisches System bereitzustellen.

Gemäß einem Aspekt kann die vorliegende Erfindung zudem vorsehen, ein medizinisches Bildgebungsinstrument, insbesondere für ein erfindungsgemäßes medizinisches System, insbesondere Endoskop, bereitzustellen. Das medizinische Bildgebungsinstrument kann eine Kamera, mittels derer Bilder erfassbar und Bilddaten erzeugbar sind, eine Erkennungseinheit, die dazu eingerichtet ist, das Vorhandensein eines Erkennungsmerkmals in einem mittels der Kamera aufgenommenen Bilds des Erkennungsmerkmals zu erkennen und in Abhängigkeit des Erkennungsmerkmals ein Erkennungssignal zu erzeugen, und eine Steuereinheit, die dazu eingerichtet ist, das Erkennungssignal zu verarbeiten und zumindest eine Funktion des medizinischen Bildgebungsinstruments nach Maßgabe des Erkennungssignals zu aktivieren, umfassen.

Gemäß einem Aspekt kann die vorliegende Erfindung zudem vorsehen, ein Verfahren zum Betrieb eines erfindungsgemäßen medizinischen Systems und/oder eines erfindungsgemäßen medizinischen Bildgebungsinstruments bereitzustellen.

Gemäß einem Aspekt kann die vorliegende Erfindung zudem vorsehen, ein Verfahren mit einem, insbesondere erfindungsgemäßen, medizinischen System bereitzustellen. Das medizinische System kann ein Objekt, das ein Erkennungsmerkmal aufweist und das anhand des Erkennungsmerkmals identifizierbar ist, und ein medizinisches Bildgebungsinstrument mit einer Kamera, mittels derer Bilder erfassbar und Bilddaten erzeugbar sind, wobei mittels der Kamera ein Bild des Erkennungsmerkmals des Objekts erfassbar ist, umfassen. Das Verfahren kann den Schritt eines Erfassens eines Bilds des Erkennungsmerkmals des Objekts mittels der Kamera umfassen.

Durch diese Merkmale kann ein Betrieb eines medizinischen Bildgebungsinstruments sicherer und/oder komfortabler gestaltet werden. Ferner kann eine Bediensicherheit und/oder eine Betriebssicherheit des, insbesondere kabellosen, medizinischen Bildgebungsinstruments erhöht werden. Beispielsweise kann erreicht werden, dass eine Funktion berührungsfrei aktivierbar ist. Es kann zudem sichergestellt werden, dass lediglich das Bildgebungsinstrument gezielt aktiviert werden kann. Das Aktivieren anhand des Erkennungsmerkmals kann als eine kostengünstige, einfache und effiziente Sicherheitsfunktion implementiert werden. Ferner können bestehende Bildgebungsinstrumente unter Bezugnahme des Erkennungsmerkmals betrieben werden. Es kann vorteilhaft ausgenutzt werden, dass Bildgebungsinstrumente zumindest häufig bereits eine Kamera aufweisen. Das bedeutet, dass für das Vorsehen des Erkennungsmerkmals und die Implementierung der Erkennungseinheit keine bauliche Anpassung bereits bestehender oder herkömmlicher Bildgebungsinstrumente notwendig ist. Es kann die ohnehin vorhandene Kamera verwendet werden. Ferner kann der Benutzer selbst das Aktivieren vornehmen. Dadurch hat er Gewissheit, dass eine bestimmte Funktion des Bildgebungsinstrument nicht ungewollt aktiviert wird und diese erst nach der Maßgabe des Erkennungsmerkmals aktiviert wird. Beispielsweise kann das Aktivieren einer Funktion in bestimmten Situationen rechtlich bedenklich sein. Umfasst die Funktion etwa eine scharfe Bilderfassung eines Objektbereichs, kann es ungewollt sein, dass die Bilderfassung ohne vorherige Zustimmung eines Patienten durchgeführt wird. Ferner kann es gesetzliche Bestimmungen geben, die es verbieten, bestimmte Merkmale eines Patienten wie etwa das Gesicht auf erfassten Bildern erkenntlich sind und diese Bilder insbesondere auf zentralen Datenspeichern gespeichert werden.

Gemäß einem weiteren Aspekt kann die vorliegende Erfindung vorsehen, ein medizinisches System bereitzustellen. Das medizinische System kann ein medizinisches Bildgebungsinstrument, das dazu eingerichtet ist, Bilddaten zu erzeugen und die Bilddaten kabellos zu übertragen, eine Anzeigevorrichtung, die dazu eingerichtet ist, Bilddaten zu empfangen und eine Darstellung für einen Benutzer zu erzeugen, und eine Gestenerkennungseinheit, die dazu eingerichtet ist, eine auf die Anzeigevorrichtung gerichtete Geste des Benutzers zu erkennen und nach Maßgabe der Erkennung der Geste eine Kopplung zwischen dem Bildgebungsinstrument und der Anzeigevorrichtung herzustellen, umfassen.

Gemäß einem Aspekt kann die vorliegende Erfindung zudem vorsehen, ein Verfahren zum Betrieb eines erfindungsgemäßen medizinischen Systems bereitzustellen.

Gemäß einem Aspekt kann die vorliegende Erfindung zudem vorsehen, ein Verfahren zum Koppeln eines medizinischen Bildgebungsinstruments mit einer Anzeigevorrichtung, insbesondere mit einem erfindungsgemäßen medizinischen System, bereitzustellen. Das medizinische Bildgebungsinstrument kann dazu eingerichtet sein, Bilddaten zu erzeugen und die Bilddaten kabellos zu übertragen, und die Anzeigevorrichtung kann dazu eingerichtet sein, Bilddaten zu empfangen und eine Darstellung für einen Benutzer zu erzeugen. Das Verfahren kann die Schritte eines Erkennens einer auf die Anzeigevorrichtung gerichteten Geste des Benutzers und eines Herstellens einer Kopplung zwischen dem Bildgebungsinstrument und der Anzeigevorrichtung nach Maßgabe der Erkennung der Geste umfassen.

Durch diese Merkmale kann ein Betrieb eines medizinischen Bildgebungsinstruments vereinfacht werden. Insbesondere kann eine Kopplung eines medizinischen Bildgebungsinstruments mit einer Anzeigevorrichtung vereinfacht werden. In einer zeitkritischen Situation in einem Operationssaal kann ein Betrieb des Bildgebungsinstruments einfach sein und eine Darstellung auf einer bevorzugten Anzeigevorrichtung schnell veranlassbar sein. Zudem kann der Benutzer die Kopplung veranlassen, ohne dass er dafür ein Objekt berühren muss. Er muss beispielsweise nicht mittels seiner Hände eine berührungssensitive Benutzerschnittstelle bedienen. Dadurch kann der Benutzer kann intuitiv und komfortabel das Herstellen der Kopplung veranlassen. Durch die Gestenerkennung kann ein Hinzuschalten oder Einschalten einer Anzeigevorrichtung besonders schnell durchführbar sein. Der Benutzer muss nicht dazu vorgesehene Bedienungsmittel verwenden oder über Einstellungen von Anzeigevorrichtungen eine Kopplung veranlassen. Es kann beispielsweise der Fall auftreten, dass der Benutzer eine bestimmte Anzeigevorrichtung hinzuschalten möchte, sodass weiteres medizinisches Personal einen Eingriff auf dieser verfolgen kann. Dies kann der Benutzer komfortabel und schnell mittels der Geste veranlassen. Dadurch, dass die Geste auf die Anzeigevorrichtung gerichtet ist, ist Gefahr einer Fehlbedienung gering. Eine spontane, ungewollte Kopplung kann zumindest im Wesentlichen verhindert werden.

Durch die unterschiedlichen obengenannten Aspekte können Verbesserungen im Bereich der medizinischen Bildgebung ermöglicht werden.

Wenn eine Vorrichtung, Einheit und/oder eine Komponente, insbesondere ein medizinisches System und/oder ein Bildgebungsinstrument, im Zusammenhang mit verschiedenen Aspekten der vorliegenden Erfindung beschrieben ist, kann es sich grundsätzlich um dieselbe Vorrichtung, Einheit und/oder eine Komponente, insbesondere dasselbe medizinisches System und/oder dasselbe Bildgebungsinstrument, handeln. Das kann beispielsweise bedeuten, dass ein medizinisches System Merkmale verschiedener hierin beschriebener Aspekte aufweisen kann.

Das medizinische System kann ein medizinisches Bildgebungssystem, insbesondere ein medizinisches Endoskopie-System, sein. Das kann bedeuten, dass das medizinische System zur endoskopischen Bildgebung eingerichtet ist. Alternativ oder zusätzlich kann das medizinische System ein medizinisches Exoskopie-System sein, das zur exoskopischen Bildgebung eingerichtet ist. Die medizinischen Systeme gemäß den mehreren Aspekten der vorliegenden Erfindung können dasselbe medizinische System umfassen. Ein medizinisches System kann Merkmale gemäß mehreren Aspekten der vorliegenden Erfindung aufweisen. Insbesondere können in einigen Ausführungsformen Merkmale der mehreren Aspekte zusammenwirken. Generell können Einheiten und/oder Baugruppen, die dieselbe Bezeichnung haben, dieselbe Einheit und/oder Baugruppe darstellen. Alternativ oder zusätzlich können auch verschiedene Einheiten und/oder Baugruppen gemeint sein.

Zu dem medizinischen System zugehörig kann jede Komponente sein, die im Zusammenhang mit einem Einsatz des medizinischen Bildgebungsinstruments und/oder der Anzeigevorrichtung vorgesehen ist. Beispielsweise kann das medizinische System Werkezuge, beispielsweise endoskopische Zangen, Absaugschläuche, Trachealtuben, Sonden und/oder dergleichen, umfassen. Ferner kann das medizinische System zumindest eine Datenverarbeitungsvorrichtung umfassen. Die Datenverarbeitungsvorrichtung kann einen Prozessor, einen Datenspeicher, Arbeitsspeicher, einen Grafikprozessor und/oder dergleichen umfassen. Die Datenverarbeitungsvorrichtung kann eine zentrale Datenverarbeitungsvorrichtung und/oder eine, insbesondere zentrale, Recheneinheit umfassen, die dazu eingerichtet ist, Daten mehrerer Komponenten, beispielsweise mehrerer Bildgebungsvorrichtungen und/oder Anzeigevorrichtungen, zu verarbeiten. Insbesondere kann die Datenverarbeitungsvorrichtung zur Verarbeitung von Bilddaten eingerichtet sein. Auf der Datenverarbeitungsvorrichtung können hierin beschriebene Einheiten und/oder Komponenten implementierbar und/oder implementiert sein. Die Datenverarbeitungsvorrichtung kann in einer Arbeitsumgebung des medizinischen Bildgebungsinstruments angeordnet sein. Das kann bedeuten, dass die Datenverarbeitungsvorrichtung in einem Operationssaal oder Behandlungszimmer angeordnet ist, in dem das Bildgebungsinstrument eingesetzt wird. In diesem Zusammenhang kann die Datenverarbeitungsvorrichtung einen Rechner und/oder einen Laptop umfassen. Ferner kann eine zentrale Recheneinheit zumindest ein Teil einer Datenverarbeitungsvorrichtung sein.

Ferner kann die Datenverarbeitungsvorrichtung eine cloudbasierte Datenverarbeitungsvorrichtung umfassen. Eine cloudbasierte Datenverarbeitungsvorrichtung kann eine Datenverarbeitungsvorrichtung sein, die geteilte Datenverarbeitungskapazitäten, beispielsweise in Form von Servern und/oder Datenspeichern, bereitstellt.

Das medizinische Bildgebungsinstrument kann ein Exoskop und/oder ein Endoskop umfassen. Das medizinische Bildgebungsinstrument kann dazu eingerichtet sein, in eine Körperhöhle des Patienten eingeführt zu werden. Eine Körperhöhle kann eine beliebige Körperhöhle und/oder Kavität des Patienten umfassen. Beispielsweise kann die Körperhöhle die Nasenhöhle, die Mundhöhle, die Trachea, die Lunge, den Magen, den Darm, eine Bauchhöhle, eine Thoraxhöhle und/oder einen Gelenkraum umfassen.

Generell kann das medizinische Bildgebungsinstrument dazu eingerichtet sein, einen anatomischen Objektbereich abzubilden und Bilddaten des Objektbereichs zu erzeugen. Der anatomische Objektbereich kann sich auf einen Teil des Körpers des Patienten beziehen. Der anatomische Objektbereich kann eine anatomische Struktur, beispielsweise eine Innenwand eines Darms, eine Innenseite eines Lungenflügels, Knorpelspangen einer Luftröhre, eine Innenwand eines Gefäßes und/oder dergleichen eines Patienten umfassen.

Das Bildgebungsinstrument, insbesondere das Endoskop kann einen Basisabschnitt, und/oder einen Schaft umfassen, wobei der Schaft sich distal von dem Basisabschnitt erstreckt. Der Basisabschnitt kann einen Handgriff umfassen. Der Schaft kann länglich und/oder dünn sein. Insbesondere kann der Schaft ein großes Seitenverhältnis aufweisen. Der Schaft kann einen Außendurchmesser von beispielsweise bis zu 2 cm, insbesondere bis zu 1 cm, in einigen Ausführungsformen bis zu 0,5 cm aufweisen. Der Schaft kann eine Länge von beispielsweise bis zu 300 cm, insbesondere bis zu 150 cm, bevorzugt bis zu 50 cm, aufweisen. Der Schaft kann zumindest abschnittsweise starr und/oder flexibel sein. Ferner kann der Schaft einen beweglichen Abschnitt, insbesondere einen beweglichen distalen Endabschnitt, aufweisen. "Beweglich" kann bedeuten, dass der Benutzer den beweglichen Abschnitt gezielt biegen kann, beispielsweise mittels eines Kabelzugs und/oder mittels eines Motors. Der Schaft kann dazu eingerichtet sein, zumindest teilweise der Körperhöhle des Patienten zugeführt zu werden.

Zum Abbilden des Objektbereichs kann das Bildgebungsinstrument, insbesondere der Schaft und/oder der distale Endabschnitt, eine Eingangsoptik, beispielsweise eine oder mehrere Linsen, aufweisen, die dazu eingerichtet ist, Licht, insbesondere Objektlicht, zu sammeln. Das Licht kann auf einen Bildsensor führbar sein. Der Bildsensor kann dazu eingerichtet sein, Bilddaten anhand des Lichts zu erzeugen. Der Bildsensor kann in dem Schaft, insbesondere in dem distalen Endabschnitt, angeordnet sein. In diesem Fall können die Bilddaten entlang des Schafts zu dem Basisabschnitt führbar sein, beispielsweise durch eine elektronische und/oder optische Datenleitung. Alternativ oder zusätzlich kann ein Bildsensor in dem Basisabschnitt und/oder in einer externen Bildgebungsvorrichtung angeordnet sein.

Das Bildgebungsinstrument kann gemäß einigen Ausführungsformen eine Einrichtung zum kabellosen Datenübertragen umfassen. Insbesondere kann das Bildgebungsinstrument dazu eingerichtet sein, Daten, die auf den Bilddaten beruhen, kabellos zu übertragen. Alternativ oder zusätzlich kann der Datenübertragungsadapter an das Bildgebungsinstrument angeschlossen werden. Mittels des angeschlossenen Datenübertragungsadapters kann das Bildgebungsinstrument zum kabellosen Übertragen von Daten eingerichtet sein. Das Bildgebungsinstrument kann gemeinsam mit dem Datenübertragungsadapter ein Bildgebungsinstrument gemäß einigen Aspekten der Erfindung definieren.

Ferner kann das Bildgebungsinstrument eine Beleuchtungsvorrichtung zur Beleuchtung des Objektbereichs aufweisen. Beispielsweise kann die Beleuchtungsvorrichtung ein Beleuchtungsmittel in dem distalen Endabschnitt umfassen. Alternativ oder zusätzlich kann die Beleuchtungsvorrichtung eine Lichtschnittstelle umfassen, mittels derer externes Beleuchtungslicht, das von einer Lichtquelle erzeugt werden kann, in das Bildgebungsinstrument, insbesondere den Schaft, einkoppelbar ist. Die Beleuchtungsvorrichtung kann ferner eine Lichtauskopplungsoptik, beispielsweise eine Zerstreuungslinse, aufweisen, mittels derer Beleuchtungslicht aus dem Bildgebungsinstrument auskoppelbar ist. Alternativ oder zusätzlich kann das Bildgebungsinstrument, insbesondere die Beleuchtungsvorrichtung, ein Leuchtmittel umfassen. Das Leuchtmittel kann in dem distalen Endabschnitt angeordnet sein. Das Leuchtmittel kann zumindest eine LED umfassen.

Komponenten des Bildgebungsinstruments, die zur Erzeugung von Bilddaten mitwirken, beispielsweise die Eingangsoptik, der Bildsensor, das Leuchtmittel und/oder dergleichen, können gemeinsam eine Kamera definieren. Die Kamera ist dazu eingerichtet, Bilder, insbesondere eines Objektbereichs, zu erfassen und Bilddaten zu erzeugen. Der Begriff "Bild" kann Synonym mit dem Begriff "Abbildung" verwendet werden.

Die Bilddaten können analoge und/oder digitale Signale und/oder digitale Information umfassen, die eine Darstellung, insbesondere des anatomischen Objektbereichs, repräsentieren. Insbesondere können die Bilddaten, insbesondere visuelle, Information über einen Objektbereich tragen. Die Bilddaten können in mehrere Teilbereiche, insbesondere Pixel, unterteilt sein, die jeweils beispielsweise Farbwerte, Helligkeitswerte und/oder Intensitätswerte tragen. Die Bilddaten können kabelgebunden und/oder kabellos übertragbar sein. Ferner können die Bilddaten in verschiedenen Formaten formatierbar sein und/oder sie können verschlüsselbar sein.

Der Steckeranschluss kann eine Schnittstelle umfassen, mittels derer das Bildgebungsinstrument mit anderen Geräten und/oder System verbunden werden kann. Mittels des Steckeranschlusses können Bilddaten und/oder auf den Bilddaten beruhende Information, Steuersignale, Statussignale, Energie und/oder dergleichen übertragbar sein. Ferner kann der Steckeranschluss eine mechanische Funktion erfüllen. Das kann bedeuten, dass der Steckeranschluss eine Steckerbuchse umfassen kann. Das Datenkabel kann an dem Steckeranschluss lösbar befestigbar sein. "Anschließen" kann in dem Zusammenhang auch "befestigen" umfassen. Das Datenkabel kann beispielsweise mittels eines Bajonettmechanismus, eines Klemmmechanismus, eines Steckmechanismus und/oder dergleichen befestigbar und/oder anschließbar sein. Der Steckeranschluss kann herstellerspezifische Spezifikationen und/oder Merkmale aufweisen. Beispielsweise kann der Steckeranschluss eine bestimmte Anzahl an Pins aufweisen, die jeweils eine, insbesondere herstellerspezifische, Anordnung aufweisen können. Den einzelnen Pins kann eine, insbesondere herstellerspezifische, Funktion zuordenbar sein. Beispielsweise kann ein Pin für eine Energieversorgung und/oder eine Datenübertragung vorgesehen sein.

Mit "lösbar" kann gemeint sein, dass das Datenkabel nicht fest mit dem Bildgebungsinstrument verbaut ist. Ein Beispiel eines nicht lösbaren Datenkabels kann ein Datenkabel sein, das an eine Platine eines Bildgebungsinstruments gelötet ist. In einigen Ausführungsformen kann vorgesehen sein, dass das Datenkabel permanent an das Bildgebungsinstrument angeschlossen ist, jedoch auch "lösbar" an das Bildgebungsinstrument angeschlossen ist. Das Datenkabel kann gemäß einer solchen Ausführungsform von dem Bildgebungsinstrument gelöst werden, ohne dass eine dauerhafte Verbindung, etwa eine Lötverbindung, gelöst werden muss. Das Datenkabel kann etwa mittels einer Schraubverbindung an das Bildgebungsinstrument lösbar angeschlossen sein. Ferner kann der Steckeranschluss von einem Gehäuse des Bildgebungsinstruments eingehaust sein. Für ein Lösen des Datenkabels kann es notwendig sein, das Gehäuse zumindest teilweise zu öffnen. Mit "permanent" kann gemeint sein, dass das Datenkabel nicht vor zumindest im Wesentlichen jedem Einsatz des Bildgebungsinstruments an das Bildgebungsinstrument angeschlossen wird.

Das Datenkabel kann vorrangig zur Datenübertragung, insbesondere Bilddatenübertragung, vorgesehen sein. Ferner kann das Datenkabel zur Energieversorgung und/oder zur Signalübertragung vorgesehen sein. Das Datenkabel kann beispielsweise einen Stecker umfassen, der kompatibel mit dem Steckeranschluss ist. Ferner kann das Datenkabel einen Stecker umfassen, der kompatibel mit einem Anschluss der Datenverarbeitungsvorrichtung und/oder der Anzeigevorrichtung ist. Der Stecker kann etwa einen USB-Stecker umfassen. Ferner kann der Stecker herstellerspezifisch und/oder speziell für das medizinische System ausgebildet sein.

Der Steckeranschluss kann an dem Basisabschnitt des Bildgebungsinstruments angeordnet sein. Gemäß einigen Ausführungsformen ist der Basisabschnitt an einem proximalen Abschnitt, insbesondere Ende, des Basisabschnitts angeordnet. Herkömmliche, kabelgebundene Bildgebungsinstrumente weisen häufig einen Steckeranschluss auf. Der Datenübertragungsadapter kann dazu eingerichtet sein, an einen Steckeranschluss eines herkömmlichen, kabelgebundenen Bildgebungsinstruments angeschlossen zu werden.

Der Datenübertragungsadapter ist insbesondere ein eigenständiges und vollständig separat handhabbares Gerät. Der Datenübertragungsadapter ist insbesondere von dem medizinischen Bildgebungsinstrument, mit dessen Steckeranschluss er lösbar verbunden ist, separat ausgebildet. Der Datenübertragungsadapter ist insbesondere kein Teil des besagten medizinischen Bildgebungsinstruments. Der Datenübertragungsadapter kann ein Adaptergehäuse umfassen. Das Adaptergehäuse kann die Komponenten des Datenübertragungsadapters vollständig einhausen. Das besagte Bildgebungsinstrument kann ein Instrumentengehäuse aufweisen. Das Instrumentengehäuse kann die Komponenten des Bildgebungsinstruments vollständig einhausen. Das Adaptergehäuse und das Instrumentengehäuse können separate Bauteile sein.

Der Datenübertragungsadapter kann das Anschlusselement, das lösbar mit dem Steckeranschluss verbindbar ist, die Steuereinheit, die mit dem Anschlusselement verbunden ist und die dazu eingerichtet ist, die Bilddaten von dem Anschlusselement zu empfangen, und die Datenschnittstelle, die mit der Steuereinheit verbunden ist und die dazu eingerichtet ist, Daten von der Steuereinheit zu empfangen und Daten kabellos zu übertragen, umfassen. Die Datenschnittstelle kann beispielsweise ein WLAN-Modul, ein Bluetooth-Modul und/oder dergleichen umfassen. Grundsätzlich kann die Datenschnittstelle dem Datenübertragungsadapter eine Fähigkeit zur kabellosen Datenübertragung verleihen.

Der Datenübertragungsadapter kann gemeinsam mit dem Bildgebungsinstrument in einem angeschlossenen Zustand ein Bildgebungsinstrument definieren. Ein "Bildgebungsinstrument" kann ein Bildgebungsinstrument ohne angeschlossenem Datenübertragungsadapter und/oder ein Bildgebungsinstrument mit angeschlossenem Datenübertragungsadapter umfassen. Ein Bildgebungsinstrument ohne angeschlossenem Datenübertragungsadapter kann ein kabelgebundenes und/oder ein kabelloses Bildgebungsinstrument umfassen. Ein kabelloses Bildgebungsinstrument kann beispielsweise eine Baugruppe umfassen, die Merkmale des Datenübertragungsadapters aufweisen kann. Beispielsweise kann ein kabelloses Bildgebungsinstrument dazu eingerichtet sein, auf den Bilddaten beruhende Daten kabellos zu übertragen. Der Datenübertragungsadapter kann einem kabelgebundenen Bildgebungsinstrument Funktionen verleihen, die ein kabelloses Bildgebungsinstrument hat.

Der Datenübertragungsadapter kann grundsätzlich ähnlich wie ein USB-WLAN Adapter funktionieren. Ein USB-WLAN Adapter kann in einen USB-Anschluss eines Rechners gesteckt werden, um eine kabellose Datenübertragung zu ermöglichen. Dadurch erweitert dieser die kabellose Datenübertragungsfähigkeit des Rechners. In ähnlicher Weise erweitert der Datenübertragungsadapter die kabellose Datenübertragungsfähigkeit des Bildgebungsinstruments. Insbesondere die Datenschnittstelle kann dazu vorgesehen sein, die kabellose Datenübertragungsfähigkeit vorzusehen.

Der Datenübertragungsadapter kann dazu eingerichtet sein, ein Datenkabel zu simulieren und/oder zu ersetzen und/oder funktionsmäßig abzubilden. Das kann bedeuten, dass eine Datenübertragung bildgebungsinstrumentenseitig derart durchführbar ist, als sei ein Datenkabel angeschlossen, obwohl der Datenübertragungsadapter angeschlossen ist. Der Datenübertragungsadapter kann also die Bilddaten über den Steckeranschluss insbesondere datenkabelartig empfangen. Der Datenübertragungsadapter kann das Anschlusselement, insbesondere ein Steckerabschnitt, umfassen, der mit dem Steckeranschluss kompatibel ist. Das Anschlusselement kann ähnlich wie der Stecker eines Datenkabels ausgebildet sein. Beispielsweise kann das Anschlusselement den Befestigungsmechanismus aufweisen, der dazu eingerichtet ist, das Datenkabel zu befestigen. Der Datenübertragungsadapter kann wie das Datenkabel anschließbar und/oder befestigbar sein. Alternativ oder zusätzlich kann der Datenübertragungsadapter einen Adapterbefestigungsmechanismus umfassen, der dazu eingerichtet ist, den Datenübertragungsadapter an dem Bildgebungsinstrument zu befestigen. Um das medizinische Bildgebungsinstrument zur kabellosen Datenübertragung einzurichten, kann ein Benutzer ein an dem Steckeranschluss angeschlossenes Datenkabel lösen und den Datenübertragungsadapter in den freien Steckeranschluss stecken. Der Datenübertragungsadapter kann in der Folge alle Funktionen aufweisen, die eine kabellose Datenübertragung ermöglichen, wobei der Datenübertragungsadapter die Bilddaten über den Steckeranschluss empfängt.

Mit "kabellos übertragen" kann jede Datenübertragung gemeint sein, bei der keine auf die Datenübertragung gerichtete physische Verbindung zwischen dem Sender und dem Empfänger besteht. Eine physische Verbindung kann jedoch zusätzlich, beispielsweise in Form einer Stromversorgung, vorliegen. Die Datenschnittstelle des Datenübertragungsadapters kann dazu vorgesehen sein. Auf der Empfängerseite kann eine passende Datenschnittstelle vorgesehen sein. Beispielsweise kann der Datenübertragungsadapter in ein kabelloses Netzwerk, beispielsweise ein WLAN-Netzwerk, eingebunden sein. Die Bilddaten, allgemein Daten und/oder die auf den Bilddaten beruhende Daten können an einen Router übertragbar sein. Ferner können die Bilddaten, allgemein Daten und/oder die auf den Bilddaten beruhende Daten kabellos über eine Bluetooth-Verbindung, eine NFC-Verbindung, eine Infrarot-Verbindung, eine kabellose USB-Verbindung und/oder dergleichen übertragbar sein.

Die auf den Bilddaten beruhende Daten können ebenfalls Bilddaten sein. Diese Daten können Bilddaten in einem anderen Format umfassen. Die auf den Bilddaten beruhenden Daten können insbesondere die auf den Objektbereich bezogene visuelle Information der Bilddaten umfassen. Beispielsweise können die Bilddaten gesammelt als Daten kabellos übertragbar sein. Gibt es beispielsweise kurze Datenübertragungsunterbrechungen können die Daten gesammelt übertragen werden. Ferner können die Bilddaten gefiltert und/oder bearbeitet werden, bevor sie übertragen werden. Mit Bilddaten können insbesondere Bildrohdaten gemeint sein. Die auf den Bilddaten beruhenden Daten können bearbeitete Bilddaten sein.

Die Steuereinheit kann eine Recheneinheit, insbesondere einen Prozessor und/oder einen Mikroprozessor, einen Datenspeicher, einen Arbeitsspeicher, einen Grafikprozessor und/oder dergleichen umfassen. Die Steuereinheit kann als ein integrales Elektronikbauteil, beispielsweise auf einer gemeinsamen Platine, ausgebildet sein. Die Datenschnittstelle kann gemeinsam mit der Steuereinheit ausgebildet sein. Beispielsweise kann die Datenschnittstelle auf der gemeinsamen Platine angeordnet sein, beziehungsweise dauerhaft mit dieser verbunden sein. Die Datenschnittstelle kann beispielsweise ein WLAN-Modul und/oder ein Bluetooth-Modul umfassen. Die Steuereinheit kann dazu eingerichtet sein, die Bilddaten zu verarbeiten. Generell kann die Steuereinheit die Bilddaten derart verarbeiten, dass Daten beruhend auf den Bilddaten durch die Datenschnittstelle kabellos übertragbar sind. Der Bildsensor kann in einigen Ausführungsformen ebenfalls integral mit der Steuereinheit ausgebildet sein und/oder dauerhaft mit dieser verbunden sein. Gemäß einigen Ausführungsformen kann das medizinische Bildgebungsinstrument die Steuereinheit und die Datenschnittstelle umfassen. Solche Bildgebungsinstrumente können ohne den Datenübertragungsadapter zur kabellosen Datenübertragung eingerichtet sein.

Bildgebungsinstrumente, die nicht zur kabellosen Datenübertragung eingerichtet sind, können mittels des Datenübertragungsadapters zur kabellosen Datenübertragung eingerichtet werden. Das kann bedeuten, dass diese Bildgebungsinstrumente nachrüstbar sind. Mittels des Datenübertragungsadapters nachgerüstete Bildgebungsinstrumente können zur kabellosen Datenübertragung eingerichtet sein. Der Datenübertragungsadapter kann permanent mit dem Bildgebungsinstrument verbunden sein. Das kann bedeuten, dass der Adapter nicht nach erfolgten Einsätzen des Bildgebungsinstruments von diesem gelöst werden muss.

Das Nachrüsten kann ein Verbinden des Datenübertragungsadapters umfassen. Das Verbinden kann ein permanentes Verbinden umfassen. Beispielsweise kann der Datenübertragungsadapter mit einem Gehäuse des Bildgebungsinstruments verbunden, insbesondere verschraubt und/oder verklemmt, werden und/oder zumindest teilweise innerhalb des Gehäuses angeordnet werden. In einigen Ausführungsformen kann das Adaptergehäuse and das Instrumentengehäuse anbindbar und/oder mechanisch ankoppelbar sein. Ferner kann mit "Verbinden" ein Einstecken des Datenübertragungsadapters in den Steckeranschluss umfassen.

Unter einer Anzeigevorrichtung kann grundsätzlich ein Gerät verstanden werden, das einen Bildschirm umfasst. Ein Bild des Objektbereichs kann auf der Anzeigevorrichtung, insbesondere dem Bildschirm, dargestellt werden. Der Benutzer kann beispielsweise anhand der Darstellung der Bilddaten den Objektbereich auf der Anzeigevorrichtung sehen und/oder bewerten. Die Anzeigevorrichtung kann einen Computerbildschirm, einen Bildschirm in einem Operationssaal, ein mobiles Endgerät und/oder dergleichen umfassen. Ein mobiles Endgerät kann ein Tablet, ein Laptop, ein Smartphone und/oder einen tragbaren Bildschirm umfassen. Der tragbare Bildschirm kann beispielsweise zum Einsatz in einem Rettungswagen vorgesehen sein, wobei verschiedene Bildgebungsinstrumente mit dem Bildschirm koppelbar sind.

Die Darstellung kann, insbesondere visuelle, Information über den Objektbereich abbilden und für den Benutzer verfügbar machen. Die Darstellung kann auf den Bilddaten beruhen. Die Bilddaten können zur Darstellung bearbeitbar sein. Beispielsweise können analoge und/oder digitale Bildfilter, insbesondere Glättungsfilter, Farbfilter, Passfilter, auf die Bilddaten anwendbar sein.

Unter "Koppeln" kann ein Prozess verstanden werden, bei dem zwei Geräte miteinander verbunden werden, um Information und/oder Daten auszutauschen und/oder sich eine Funktion zu teilen. In einem ungekoppelten Zustand können Daten nicht von dem Bildgebungsinstrument, insbesondere an die Anzeigevorrichtung, übertragen werden. In einem gekoppelten Zustand können Daten ausgetauscht werden und insbesondere Daten, die auf den Bilddaten beruhen, übertragen werden. Ferner kann eine Funktion des Bildgebungsinstruments in einem gekoppelten Zustand gesteuert werden. Weist das Bildgebungsinstrument etwa ein Beleuchtungsmittel auf, kann dieses in einem gekoppelten Zustand über eine externe Benutzerschnittstelle aktiviert werden. In dem gekoppelten Zustand können Betriebszustandsdaten austauschbar sein. Beispielsweise kann eine Information übertragbar sein, die eine Einsatzbereitschaft einer Kamera des Bildgebungsinstruments betrifft. Ist eine Einsatzbereitschaft etwa nicht gegeben, kann ein Erzeugen von Bilddaten und/oder ein Übertragen von Bilddaten verhindert sein.

Das Bildgebungsinstrument, die Anzeigevorrichtung und/oder eine Datenverarbeitungsvorrichtung kann die Sicherheitseinheit umfassen. Die Sicherheitseinheit kann eine virtuelle Komponente des medizinischen Systems umfassen und/oder dazu eingerichtet sein, eine ungewollte und/oder unbeabsichtigte Kopplung eines Bildgebungsinstruments mit einer Anzeigevorrichtung zu erkennen. Das Bildgebungsinstrument, die Anzeigevorrichtung und/oder eine Datenverarbeitungsvorrichtung kann zumindest eine Steuerung und/oder zumindest eine Recheneinheit umfassen, welche die Sicherheitseinheit implementiert. Die Sicherheitseinheit kann dedizierte Schaltkreise aufweisen und/oder zumindest teilweise als Teil einer Programmierung in allgemeinen Schaltkreisen des Bildgebungsinstruments, der Anzeigevorrichtung und/oder der Datenverarbeitungsvorrichtung ausgeführt sein. Das Bildgebungsinstrument und/oder die Anzeigevorrichtung kann die Sicherheitseinheit zumindest teilweise umfassen. Ferner können das Bildgebungsinstrument und die Anzeigevorrichtung die Sicherheitseinheit gemeinsam umfassen. Mittels der Sicherheitseinheit kann sichergestellt werden, dass lediglich eine gewünschte und/oder gewollte Kopplung hergestellt wird. Das bedeutet, dass lediglich eine vorgesehene Paarung einer Anzeigevorrichtung und eines Bildgebungsinstrument hergestellt wird. Erkennt die Sicherheitseinheit eine ungewollte Paarung eines Bildgebungsinstruments und einer Anzeigevorrichtung, kann die Sicherheitseinheit eine Datenübertragung, insbesondere eine Bilddatenübertragung, verhindern.

Die Sicherheitseinheit kann anhand einer Maßgabe der Bestätigung der Kopplung durch den Benutzer entscheiden, ob eine vorliegende Paarung eine gewollte Paarung ist. Bleibt die Bestätigung aus, kann die Sicherheitseinheit entscheiden, dass die vorliegende Paarung eine ungewollte Paarung ist und das Übertragen der Bilddaten verhindern. Mit dem Ausbleiben der Bestätigung kann insbesondere das Fehlen der Bestätigung gemeint sein. Das kann bedeuten, dass der Benutzer aktiv eine Kopplung und/oder eine Paarung bestätigen muss, damit Bilddaten übertragbar sind. Gemäß einigen Ausführungsformen können trotz des Fehlens der Bestätigung Informationen und/oder Daten übertragen werden. Beispielsweise können Gerätezustandsdaten und/oder Geräteidentifikationsdaten übertragen werden.

Bleibt die Bestätigung aus, kann an der Anzeigevorrichtung etwa der Benutzer über einen Kopplungsversuch und/oder eine nicht erfolgreiche Kopplung informiert werden. Wird eine Bilddatenübertragung verhindert, können die Bilddaten trotzdem erzeugt werden. Diese können auf einer Speichereinheit des Bildgebungsinstruments zwischengespeichert werden. Zwischengespeicherte Bilddaten können bei einer späteren Bestätigung einer Kopplung an die Anzeigevorrichtung und/oder an die Datenverarbeitungsvorrichtung übertragen werden.

Die Bestätigung der Kopplung kann eine Benutzereingabe umfassen. Die Bestätigung kann beispielsweise das Erzeugen eines Bestätigungssignals und/oder das Ändern des Zustands eines Bestätigungssignals bedingen. Die Sicherheitseinheit kann beispielsweise anhand des Bestätigungssignals entscheiden, ob eine Bestätigung vorliegt. Ändert sich das Bestätigungssignal, kann die Sicherheitseinheit entscheiden, dass eine Bestätigung vorliegt.

Die Sicherheitseinheit kann ferner eine Benutzerschnittstelle, insbesondere eine Eingabevorrichtung, umfassen. Beispielsweise kann die Anzeigevorrichtung einen berührungssensitiven Bildschirm umfassen. Auf dem Berührungssensitiven Bildschirm kann ein bedienbares Feld anzeigbar sein, das, wenn es betätigt wird, das Bestätigen der Kopplung bedingt.

Die Kopplung kann eine vorübergehende Verbindung zweier Geräte sein. Die Kopplung kann herstellbar sein und/oder lösbar sein. Bei einem Herstellen der Kopplung kann die Verbindung das erste Mal und/oder wieder hergestellt werden. Wird die Kopplung wieder hergestellt, kann bei dem Herstellen der Kopplung auf Information einer vorangegangenen Kopplung zurückgegriffen werden. Bei einem Lösen der Kopplung kann die Verbindung derart getrennt werden, dass keine Daten mehr austauschbar sind. Das Lösen der Kopplung kann ein Speichern von Information über die Kopplung und/oder über die gekoppelten Geräte umfassen. Beispielsweise kann eine Gerätekennung, insbesondere eine Geräteidentifikationsnummer, und/oder ein Verbindungsprotokoll abgespeichert werden.

Das Objekt kann einen beliebigen, insbesondere beweglichen, Gegenstand und/oder eine beliebige Anordnung umfassen. In manchen Ausführungsformen kann das Objekt im Zusammenhang mit dem medizinischen Bildgebungsinstrument verwendbar sein. Das Objekt kann etwa gemeinsam mit dem Bildgebungsinstrument bei der Durchführung einer diagnostischen und/oder therapeutischen Aktion verwendbar sein. Beispielsweise kann das Objekt ein medizinisches Instrument, insbesondere ein Skalpell, eine Pinzette, eine Schere, einen Haken und/oder dergleichen, ein weiteres Bildgebungsinstrument und/oder eine Anzeigevorrichtung umfassen. Ferner kann das Objekt beispielsweise ein Datenkabel, eine Datenverarbeitungsvorrichtung, ein Datenblatt, ein Formular, einen Rettungswagen und/oder dergleichen umfassen. Zudem kann das Objekt einem Bereich einer Gesundheitseinrichtung, beispielsweise einem Operationssaal und/oder einem Behandlungszimmer, zuzuordnen sein. Beispielsweise kann in einem Operationssaal eine diagnostische und/oder therapeutische Aktion durchgeführt werden, bei dem das Bildgebungsinstrument verwendet wird. Das Objekt kann eine Wand, eine Türe, ein Behandlungstisch, ein Schild und/oder dergleichen umfassen. Wurde das Erkennungsmerkmals des Objekts identifiziert, kann beispielsweise darauf geschlossen werden, dass eine diagnostische und/oder therapeutische Aktion in dem entsprechenden Operationssaal durchgeführt wird und/oder wurde. Grundsätzlich kann das Objekt als Zuordnungshilfe fungieren, sodass etwa ein Bereich eines Gebäudes identifizierbar ist. Alternativ oder zusätzlich ist vorgesehen, dass das Objekt als solches identifizierbar ist.

Das Erkennungsmerkmal kann eine bewusst und/oder willentlich erzeugte und/oder vorgesehene Charakteristik aufweisen. Es kann sich von einem Merkmal eines Objekts unterscheiden, das integral zu diesem Objekt gehört und/oder bereits ohne das Vorsehen des Erkennungsmerkmals vorhanden wäre. Das kann bedeuten, dass das Erkennungsmerkmal dem Objekt hinzufügbar sein kann. Beispielsweise kann das Erkennungsmerkmal unterschiedlich zu einer auffälligen Farbe, Formgebung, Oberflächenstruktur und/oder dergleichen des Objekts sein. Das Erkennungsmerkmal kann in diesem Zusammenhang beispielsweise ein Muster, eine Farbe, eine Farbabfolge, ein Schriftzug, eine Zahl und/oder eine Zahlenkombination und/oder dergleichen sein, das zielgerichtet zum Zweck des Erkennens und/oder Identifizierens des Objekts dem Objekt hinzufügbar ist. Beispielsweise kann ein KFZ-Kennzeichen ein Erkennungsmerkmal des Objekts KFZ sein, jedoch nicht die Form, ein vorhandener Schaden und/oder eine Farbe des KFZ. Ferner kann das Erkennungsmerkmal eine Darstellung umfassen, die zum Zweck des Erkennens und/oder Identifizierens des Objekts erzeugbar und/oder anzeigbar ist. Das Erkennungsmerkmal kann in einigen Ausführungsformen für einen Menschen nicht erkennbar und/oder erfassbar sein. Beispielsweise kann das Erkennungsmerkmal eine schnelle Bildabfolge umfassen, wobei einzelne Bilder für einen Menschen zumindest im Wesentlichen nicht verarbeitbar sind.

Unter "identifizierbar" kann verstanden werden, dass das Objekt spezifisch und/oder unspezifisch identifizierbar ist. Spezifisch kann bedeuten, dass genau das Objekt identifizierbar ist, das das Erkennungsmerkmal aufweist. Unspezifisch kann bedeuten, dass eine Art des Objekts identifizierbar ist. Ist das Objekt beispielsweise eine Zange, kann genau diese Zange spezifisch identifizierbar sein und/oder eine Zange der Art der vorliegenden Zange. Ferner kann das Objekt durch das medizinische System, insbesondere durch die Erkennungseinheit und/oder die Datenverarbeitungsvorrichtung, identifizierbar sein. Das System kann erkennen, dass das Objekt vorliegt.

Die Anzeigevorrichtung, die Datenverarbeitungsvorrichtung und/oder das Bildgebungsinstrument kann zumindest teilweise die Erkennungseinheit umfassen. Die Erkennungseinheit kann beispielsweise zumindest teilweise auf einer Recheneinheit des Bildgebungsinstruments implementiert sein. Alternativ oder zusätzlich kann die Erkennungseinheit zumindest teilweise auf der Anzeigevorrichtung und/oder in der Cloud implementiert sein. Beispielsweise kann für das Erkennen des Vorhandenseins des Erkennungsmerkmals auf Rechenleistung eines Servers zurückgegriffen werden. Das Bildgebungsinstrument, die Anzeigevorrichtung und/oder eine Datenverarbeitungsvorrichtung kann zumindest eine Steuerung und/oder zumindest eine Recheneinheit umfassen, welche die Erkennungseinheit implementiert. Die Erkennungseinheit kann dedizierte Schaltkreise aufweisen und/oder zumindest teilweise als Teil einer Programmierung in allgemeinen Schaltkreisen des Bildgebungsinstruments, der Anzeigevorrichtung und/oder der Datenverarbeitungsvorrichtung ausgeführt sein.

Die Erkennungseinheit kann das Vorhandensein des Erkennungsmerkmals mittels Mustererkennung, Texterkennung und/oder Bilderkennung erkennen. Das Erkennen kann auf einer mathematischen Rechenvorschrift und/oder einem Algorithmus basieren, beispielsweise unter Anwendung künstlicher Intelligenz, maschinellen Lernens, Deep Learnings und/oder eines neuronalen Netzes, insbesondere eines offenen, geschlossen, einschichtigen und/oder rückgekoppelten neuronalen Netzes und/oder einer Kombination dieser. Ferner können digitale Filter auf zumindest ein Bild des Erkennungsmerkmals anwendbar sein, um das Erkennungsmerkmal zu erkennen. Eine Helligkeit, eine Polarisation, eine Wellenlänge und/oder dergleichen kann filterbar sein, um das Erkennungsmerkmal zu erkennen.

Das Erkennungssignal kann ein analoges und/oder ein digitales Signal umfassen. Das Erkennungssignal kann ein Schaltsignal umfassen, das zwischen zwei Werten, etwa Null und Eins, schaltbar ist. Nimmt das Schaltsignal den Wert Eins ein, kann das das Vorhandensein des Erkennungsmerkmals signalisieren.

Die Erkennungseinheit kann mit der Steuereinheit verbunden sein. In manchen Ausführungsformen kann die Erkennungseinheit und die Steuereinheit gemeinsam auf einer Recheneinheit, die insbesondere an dem Bildgebungsinstrument angeordnet sein kann, implementiert sein. Die Steuereinheit kann grundsätzlich eine Funktion des Bildgebungsinstruments auf Grundlage von Steuersignalen und/oder dem Erkennungssignal steuern. Beispielsweise kann die Steuereinheit eine Funktion der Kamera, etwa ein einen Bildaufnahmevorgang, und/oder eine Funktion des Bildgebungsinstruments, beispielsweise ein lokales Speichern und/oder ein Übertragen von Bilddaten, steuern. Mit "steuern" kann auch ein Aktivieren der Funktion gemeint sein. Das kann bedeuten, dass ein Vorgang eingeleitet wird und/oder eine Komponente eingeschaltet wird. Beispielsweise kann das Leuchtmittel, insbesondere die LED, eingeschaltet werden.

Die Steuereinheit kann dazu eingerichtet sein, analoge und/oder digitale Signale zu verarbeiten. Die Steuereinheit kann beispielsweise Entscheidungen auf Grundlage von diesen Signalen treffen. Wenn die Steuereinheit etwa das Erkennungssignal verarbeitet, kann die Steuereinheit das Erkennungssignal in Verbindung mit anderen Signalen, insbesondere Steuersignalen und/oder Zustandssignalen, setzen und eine Entscheidung treffen, ob und wann ein Aktivieren der auf das Objekt bezogenen Funktion durchzuführen ist. Zustandssignale können etwa eine Einsatzbereitschaft anderer Komponenten des medizinischen Systems betreffen. Beispielsweise kann es gewollt sein, dass die auf das Objekt bezogene Funktion in bestimmten Situationen zeitverzögert aktiviert wird. In diesem Fall kann die Steuereinheit ein entsprechendes Zustandssignal gemeinsam mit der Erkennungssignal verarbeiten.

Die auf das Objekt bezogene Funktion des medizinischen Systems kann eine Funktion sein, die in einem direkten Zusammenhang mit dem Objekt steht. Es kann in Abhängigkeit von einem Vorhandensein des Objekts die Funktion aktivierbar sein. Die Funktion kann etwa eine Funktion des Objekts selbst sein. Alternativ oder zusätzlich kann die Funktion das Objekt mit einbeziehen und/oder die Funktion durch das Objekt zuordenbar sein. Kennzeichnet das Objekt beispielsweise einen bestimmten Operationssaal, kann die auf das Objekt bezogene Funktion ein Hinterlegen einer Information über den Operationssaal umfassen. Beispielsweise kann dadurch nachvollzogen werden, welche medizinischen Instrumente in diesem Operationssaal eingesetzt wurden und/oder es kann registriert werden, wann und/oder wo eine bestimmte Aktion durchgeführt wurde. Ferner kann das Vorhandensein des Objekts hinterlegt werden. Beispielsweise kann Information über medizinische Instrumente hinterlegt werden. Zudem kann auch Information über Benutzer hinterlegt werden. Das Objekt kann in einem solchen Fall etwa ein Namensschild und/oder ein anderes Erkennungsmerkmal des Benutzers sein. Ferner kann, wenn das Objekt einschaltbar ist, dieses nach Maßgabe des Erkennungssignals eingeschaltet werden. Beispielsweise kann eine Lichtquelle zur Erzeugung externen Beleuchtungslichts eingeschaltet werden. Zudem kann ein Bildgebungsmodus des Bildgebungsinstruments nach Maßgabe des Erkennungssignals umschaltbar sein. In einem Bildgebungsmodus kann etwa eine Auflösung größer sein. In diesen Bildgebungsmodus kann nach dem Erkennen eines Erkennungsmerkmals auf einem chirurgischen Instrument umschaltbar sein. Wird ein bestimmtes chirurgisches Instrument erkannt, kann also der Bildgebungsmodus mit größerer Auflösung aktivierbar sein, damit der Benutzer einen Objektbereich, in dem ein Eingriff durchzuführen ist, detailreicher betrachten kann.

Eine Funktion des Bildgebungsinstruments kann eine Helligkeit der Beleuchtungsvorrichtung, eine Aufnahmefunktion, eine Speicherfunktion, eine Bilderfassungsrate, eine Datenübertragungsfunktion und/oder dergleichen umfassen. Beispielsweise kann eine kabellose Datenübertragung aktiviert werden, eine Aufnahme gestartet werden und/oder ein zusätzliches Leuchtmittel eingeschaltet werden.

Die Anzeigevorrichtung, die Datenverarbeitungsvorrichtung und/oder das Bildgebungsinstrument kann zumindest teilweise die Gestenerkennungseinheit umfassen. Die Gestenerkennungseinheit kann beispielsweise zumindest teilweise auf einer Recheneinheit der Anzeigevorrichtung implementiert sein. Alternativ oder zusätzlich kann die Gestenerkennungseinheit zumindest teilweise in der Cloud implementiert sein. Beispielsweise kann für das Erkennen der Geste auf Rechenleistung eines Servers zurückgegriffen werden. Das Bildgebungsinstrument, die Anzeigevorrichtung und/oder eine Datenverarbeitungsvorrichtung kann zumindest eine Steuerung und/oder zumindest eine Recheneinheit umfassen, welche die Gestenerkennungseinheit implementiert. Die Gestenerkennungseinheit kann dedizierte Schaltkreise aufweisen und/oder zumindest teilweise als Teil einer Programmierung in allgemeinen Schaltkreisen des Bildgebungsinstruments, der Anzeigevorrichtung und/oder der Datenverarbeitungsvorrichtung ausgeführt sein.

Die Gestenerkennungseinheit kann die Geste mittels Mustererkennung und/oder Bilderkennung erkennen. Das Gestenerkennen kann auf einer mathematischen Rechenvorschrift und/oder einem Algorithmus basieren, beispielsweise unter Anwendung künstlicher Intelligenz, maschinellen Lernens, Deep Learnings und/oder eines neuronalen Netzes, insbesondere eines offenen, geschlossen, einschichtigen und/oder rückgekoppelten neuronalen Netzes und/oder einer Kombination dieser.

Die Gestenerkennungseinheit kann eine Kamera umfassen, die dazu eingerichtet ist, einen Objektbereich umfassend den Benutzer und/oder das Bildgebungsinstrument abzubilden und Bilddaten dieses Objektbereichs zu erzeugen. Der Benutzer und/oder das Bildgebungsinstrument kann anhand dieser Bilddaten beobachtbar sein und/oder eine Bewegung des Benutzers und/oder des Bildgebungsinstruments bestimmbar sein. Beispielsweise kann eine Armbewegung, eine Kopfdrehbewegung und/oder eine Körperhaltung eine bestimmbar sein. Es kann außerdem eine Richtung bestimmbar sein, in die das Bildgebungsinstrument gezeigt und/oder gehalten wird. Ferner kann die Gestenerkennungseinheit einen Marker, insbesondere einen optischen Marker und/oder einen elektromagnetischen Marker, umfassen, der an dem Bildgebungsinstrument angeordnet ist. Mittels einer Messeinrichtung, beispielsweise der Kamera und/oder einer elektromagnetischen Messeinrichtung, der Gestenerkennungseinheit kann eine Bewegung des Markers bestimmbar sein und anhand der bestimmten Bewegung eine Geste erkennbar sein. Eine Bewegung kann ähnlich wie bei einem chirurgischen Navigationssystem bestimmbar sein.

Die Geste kann eine willentliche Körperbewegung, die explizit zur Herstellung der Kopplung ausgeführt wird, und/oder Körperhaltung, die explizit zur Herstellung der Kopplung eingenommen wird, umfassen. Ein Verfolgen etwa der Blickrichtung des Benutzers und ein Erstellen einer Darstellung auf einer Anzeigevorrichtung in Blickrichtung des Benutzers ist damit nicht umfasst. Die Geste kann beispielsweise das Zeigen mit einer Hand und/oder einem Finger auf eine Anzeigevorrichtung, das Ausrichten des Bildgebungsinstrument in Richtung der/einer Anzeigevorrichtung und/oder das Drehen und/oder das Neigen des Kopfs in die Richtung der/einer Anzeigevorrichtung umfassen.

Die Gestenerkennungseinheit kann dazu eingerichtet sein, die Geste direkt zu erkennen. Das kann bedeuten, dass die Geste anhand eines Verhaltens des Benutzers erkennbar ist. Alternativ oder zusätzlich kann die Gestenerkennungseinheit dazu eingerichtet sein, die Geste indirekt zu erkennen. Das kann bedeuten, dass die Geste anhand einer Bewegung des Bildgebungsinstruments und/oder eines anderen Objekts erkennbar ist. Anhand der Bewegung des Bildgebungsinstruments und/oder des anderen Objekts kann auch die Bewegung und/oder die Geste des Benutzers rückschließbar sein.

Generell kann es für einige Merkmale der vorliegenden Erfindung unerheblich sein, ob das Bildgebungsinstrument mittels des Datenübertragungsadapters zur kabellosen (Bild-)Datenübertragung eingerichtet ist oder ob das Bildgebungsinstrument bereits ohne angeschlossenem Datenübertragungsadapter zur kabellosen (Bild-)Datenübertragung eingerichtet ist.

Der Datenübertragungsadapter kann einen Energiespeicher umfassen, der dazu eingerichtet ist, das medizinische Bildgebungsinstrument während eines Betriebs über den Steckeranschluss mit Energie zu versorgen. Der Datenübertragungsadapter kann gänzlich kabellos betreibbar sein. Nicht nur die Datenübertragung, sondern auch die Energieversorgung kann kabellos sein. Der Energiespeicher kann beispielsweise eine austauschbare Batterie und/oder einen aufladbaren Akkumulator, insbesondere einen Lithium-Ionen Akkumulator, umfassen. Der Energiespeicher kann eine Kapazität aufweisen, die dafür zumindest im Wesentlichen ausreicht, das Bildgebungsinstrument während eines Betriebs über mehrere Stunden, beispielsweise mehr zumindest im Wesentlichen zwei, drei, vier, fünf und/oder mehr als fünf Stunden zu versorgen.

Zudem kann der Datenübertragungsadapter eine Ladeschnittstelle umfassen, mittels derer der Energiespeicher aufladbar ist. Vorteilhafterweise kann der Datenübertragungsadapter dauerhaft und/oder permanent an den Steckeranschluss angeschlossen sein. Für ein Aufladen muss der Energiespeicher und/oder der Datenübertragungsadapter nicht ausgebaut beziehungsweise abgeschlossen werden. Alternativ oder zusätzlich kann der Datenübertragungsadapter eine Energiespeicherschnellaustauschvorrichtung umfassen, mittels derer im Betrieb schnell und unkompliziert ein neue Energiespeicher vorgesehen werden, falls der Energiespeicher beispielsweise entladen ist.

Zudem kann die Ladeschnittstelle zur induktiven Energieübertragung eingerichtet sein. Eine Aufladung des Energiespeichers kann komfortabler und weniger fehleranfällig durchführbar sein. Es müssen weniger fragile Bauelemente vorgesehen werden, die beschädigt werden könnten. Zudem der Datenübertragungsadapter einfacher fluiddicht und/oder gasdicht vorsehbar sein. Das kann bewirken, dass der Datenübertragungsadapter unter technisch geringerem Aufwand für eine Autoklavierbarkeit und/oder Desinfizierbarkeit vorsehbar ist. Die induktive Energieübertragung kann insbesondere induktives Laden umfassen und/oder ermöglicht eine kabellose elektrische Energieübertragung von einer Energiequelle zu dem Datenübertragungsadapter und/oder dem Bildgebungsinstrument. Es kann ein Sender, insbesondere eine Ladestation, vorgesehen werden, welche insbesondere in einer Aufbewahrungsvorrichtung und/oder Ladevorrichtung für das Bildgebungsinstrument und/oder den Datenübertragungsadapter vorsehbar ist. Der Sender kann dazu eingerichtet sein, ein wechselndes Magnetfeld zu erzeugen. Der Datenübertragungsadapter, insbesondere die Ladeschnittstelle, kann eine Spule umfassen, welche in einer Ladeposition das sich wechselnde Magnetfeld des Senders erfasst. Das sich ändernde Magnetfeld kann in der Spule, insbesondere mittels Induktion, eine elektrische Spannung erzeugen.

Alternativ oder zusätzlich kann die Ladeschnittstelle zum lösbaren Verbinden eines Ladekabels eingerichtet sein. Dadurch kann der Energiespeicher mittels Ladekabel ladbar sein. Das Laden mittels Ladekabel kann als einfache, kostengünstige und/oder platzsparende Lademöglichkeit des Energiespeichers vorgesehen werden. Das Ladekabel kann während des Betriebs angeschlossen werden, sollte der Energiespeicher einen Ladestand in einem kritischen Bereich, beispielsweise unter 20%, insbesondere unter 15%, vorzugsweise unter 10%, aufweisen.

Zudem kann der Datenübertragungsadapter einen Steckeranschluss zum Anschließen eines Datenkabels umfassen. Dadurch kann eine Redundanz erzeugt werden und ein sicherer und zuverlässiger Betrieb bei einem Ausfall eines Netzwerkes und/oder einer kabellosen Kopplung gewährleistet werden. Zudem kann ein Datenkabel angeschlossen werden, das dazu eingerichtet ist, Bilddaten und/oder auf Bilddaten beruhende Daten an eine Anzeigevorrichtung zu übertragen, die nicht zur kabellosen Datenübertragung eingerichtet ist. Dadurch kann etwa eine ältere Anzeigevorrichtung verwendet werden und ein Betrieb des Bildgebungsinstruments mit vor dem Anschließen des Datenübertragungsadapters kompatiblen Anzeigevorrichtungen erreichbar sein. Der Datenübertragungsadapter kann also lediglich dem Bildgebungsinstrument eine Funktion hinzufügen, jedoch die ursprüngliche Funktion, insbesondere des kabelgebundenen Datenübertragens, zumindest im Wesentlichen nicht beeinträchtigen.

Außerdem kann der Steckeranschluss des Datenübertragungsadapters in seiner Bauart einer Bauart des Steckeranschlusses des Bildgebungsinstruments entsprechen. Auf besonders einfach Weise kann die Kompatibilität mit der älteren Anzeigevorrichtung erreicht werden. Das Bildgebungsinstrument kann wahlweise kabellos und/oder derart betrieben werden, als sei der Datenübertragungsadapter nicht angeschlossen.

Generell kann durch das Vorsehen des Steckeranschlusses, insbesondere des bauartgleichen Steckeranschlusses, eine Vorwärtskompatibilität mit kabellosen Anzeigevorrichtungen und Rückwärtskompatibilität mit kabelgebundenen Anzeigevorrichtungen erreicht werden.

Zudem kann der Datenübertragungsadapter dazu eingerichtet sein, zumindest teilweise desinfiziert und/oder autoklaviert zu werden. Vorteilhafterweise kann der Datenübertragungsadapter nach einem Einsatz aufbereitet werden und/oder für einen neuen Einsatz vorbereitet werden. Der Datenübertragungsadapter kann, insbesondere in einem angeschlossenen Zustand, fluiddicht und/oder gasdicht ausgebildet sein.

Ferner kann das medizinische System dazu eingerichtet sein, in einem Zustand zumindest teilweise desinfiziert und/oder autoklaviert zu werden, in dem der Datenübertragungsadapter mit dem medizinischen Bildgebungsinstrument verbunden ist. Vorteilhafterweise muss der Datenübertragungsadapter für ein Aufbereiten des medizinischen Systems nicht von dem Bildgebungsinstrument abgeschlossen und/oder entfernt werden. Der Datenübertragungsadapter kann also permanent und/oder dauerhaft angeschlossen sein. Zumindest teilweise kann bedeuten, dass der Datenübertragungsadapter gemeinsam mit dem medizinischen Bildgebungsinstrument desinfiziert und/oder autoklaviert werden kann.

Außerdem kann der Datenübertragungsadapter eine Dichtvorrichtung umfassen, die dazu eingerichtet ist, in einem Zustand, in dem der Datenübertragungsadapter mit dem medizinischen Bildgebungsinstrument verbunden ist, den Steckeranschluss des Bildgebungsinstruments gegenüber einer Umgebung, insbesondere fluiddicht und/oder gasdicht, abzudichten. Die Dichtvorrichtung kann einfach und/oder kostengünstig, insbesondere gemeinsam mit dem Anschlusselement, vorsehbar sein. Die Dichtvorrichtung kann beispielsweise eine Labyrinthdichtung, eine Membrandichtung, einen Dichtring, insbesondere einen O-Ring, und/oder dergleichen umfassen. Das Anschlusselement und der Steckeranschluss können gemeinsam die Dichtvorrichtung ausbilden. Beispielsweise kann der Steckeranschluss einen Dichtabschnitt aufweisen, mittels dessen das Bildgebungsinstrument in einem Zustand, in dem ein Datenkabel mit dem Bildgebungsinstrument verbunden ist, gegenüber einer Umgebung, insbesondere gasdicht und/oder fluiddicht, abgedichtet ist. Die Dichtvorrichtung des Datenübertragungsadapters kann derart vorsehbar sein, dass sie mit dem Dichtabschnitt des Steckeranschlusses für ein Abdichten zusammenwirkt.

Zudem kann der Datenübertragungsadapter dazu eingerichtet sein, die Bilddaten vor dem kabellosen Übertragen aufzubereiten. Die Datenübertragung kann sicherer, weniger fehlerbehaftet, schneller und/oder effizienter gestaltet werden. Beispielsweise kann mit "aufbereiten" gemeint sein, dass die Bilddaten verschlüsselt, komprimiert, formatiert, verpackt, gestückelt und/oder bereinigt werden. Durch ein Verschlüsseln kann erreicht werden, dass die Datenübertragung sicherer abläuft und sensible Bilddaten vor einem Zugriff Unberechtigter besser geschützt sind. Dies kann insbesondere wichtig für Bilddaten eines Patienten sein, welche in einem medizinischen Umfeld erzeugt wurden. Durch ein Komprimieren und Bereinigen kann eine größere Datenmenge pro Zeiteinheit übertragbar sein. Ferner können fehlerbehaftete Bilddaten erkannt werden und/oder repariert werden. Durch ein Formatieren, Verpacken und/oder Stückeln können die Bilddaten zur kabellosen Übertragung aufbereitbar sein. Insbesondere können Daten erzeugbar sein, die auf den Bilddaten beruhen, wobei die Daten zur kabellosen Übertragung geeignet sind. Beispielsweise kann eine Bluetooth-Verbindung und/oder eine WLAN-Verbindung erfordern, dass Daten in Paketen gestückelt sind, ein bestimmtes Format aufweisen und/oder auf eine bestimme Weise verschlüsselt sind. Die Bilddaten können zumindest diesen Anforderungen entsprechend aufbereitet werden. Daten, die übertragen werden, können die Bilddaten und/oder Daten sein, die auf den Bilddaten beruhen. Die Bezeichnung beider Formen der Datenübertragung kann austauschbar miteinander verwendet werden und/oder dasselbe meinen. Wird etwa gefordert, dass Bilddaten übertragen werden, kann das bedeuten, dass Daten übertragen werden, die auf den Bilddaten beruhen. Es versteht sich, dass nicht zwangsläufig dieselben Bilddaten, die von der Kamera erzeugt wurden, unverändert übertragen werden.

Ferner kann der Datenübertragungsadapter dazu eingerichtet sein, die Bilddaten unmittelbar an ein mobiles Endgerät kabellos zu übertragen. Das Bildgebungsinstrument kann mit dem mobilen Endgerät betreibbar sein. Eine Flexibilität und/oder eine Vielseitigkeit des Bildgebungsinstruments kann verbessert werden. Das mobile Endgerät kann beispielsweise ein Smartphone und/oder ein Tablet umfassen. "Unmittelbar" kann bedeuten, dass die Bilddaten mittels des Datenübertragungsadapters gesendet werden und mittels des mobilen Endgeräts empfangen werden, insbesondere ohne, dass eine weitere Vorrichtung wie etwa die Anzeigevorrichtung zwischengeschaltet ist. Beispielsweise kann eine derartige Datenübertragung mittels Bluetooth und/oder Wi-Fi Direct durchführbar sein.

Außerdem kann der Steckeranschluss herstellerspezifisch sein. Das kann bedeuten, dass eine Anzahl und/oder Anordnung an Anschlusselementen, insbesondere Pins, für einen Hersteller standardisiert ist und sich von Hersteller zu Hersteller unterscheidet. Ferner kann eine Formgebung und/oder ein Befestigungsmechanismus des Steckeranschlusses herstellerspezifisch sein. Das kann bedeuten, dass lediglich herstellerspezifische Stecker mit dem Steckeranschluss kompatibel sind und/oder eine Ansteuerung, beziehungsweise Pinbelegung, herstellerspezifisch ist. Insbesondere können Stecker für bestimmte Steckeranschlüsse zertifiziert sein und lediglich ein zertifizierter Stecker mit einem bestimmten Steckeranschluss für eine medizinische Anwendung rechtlich erlaubt sein. Das Anschlusselement des Datenübertragungsadapters kann eine derartige Zertifizierung aufweisen und/oder eine herstellerspezifische Ausformung und/oder Pinbelegung aufweisen.

Außerdem kann der Datenübertragungsadapter dazu eingerichtet sein, zumindest ein Steuersignal kabellos zu empfangen und das Steuersignal über den Steckeranschluss an das Bildgebungsinstrument zu übertragen. Das Bildgebungsinstrument kann zumindest im Wesentlichen kabellos betreibbar sein. In einigen Ausführungsformen können zumindest im Wesentlichen alle Funktionen des Bildgebungsinstruments kabellos steuerbar und/oder aktivierbar sein. Neben Steuersignalen können weitere Signale, beispielsweise Zustandssignale, kabellos übertragbar sein.

Zudem kann in einem Zustand, in dem der Datenübertragungsadapter mit dem medizinischen Bildgebungsinstrument verbunden ist, der Datenübertragungsadapter und das Bildgebungsinstrument gemeinsam ein von einem Benutzer als einzelnes Instrument handhabbares Bildgebungsinstrument ausbilden. Ein Betrieb des Bildgebungsinstruments kann intuitiv sein. Insbesondere kann der Datenübertragungsadapter ergonomisch ausgeformt sein und/oder eine ergonomische Ausformung und/oder Eigenschaft des ursprünglichen Bildgebungsinstruments, das den Steckeranschluss aufweist und zur kabelgebundenen Datenübertragung vorgesehen ist, zumindest im Wesentlichen nicht beeinträchtigen. Beispielsweise kann der Datenübertragungsadapter vergleichsweise klein sein, insbesondere in einem Verhältnis zu dem proximalen Handgriff des Bildgebungsinstruments. Ferner kann der Datenübertragungsadapter eine Formgebung, insbesondere des Handgriffs, des Datenübertragungsadapters aufnehmen und fortführen. Ein Übergang vom Handgriff zum Datenübertragungsadapter kann zumindest im Wesentlichen harmonisch ausgeformt sein. Der Datenübertragungsadapter kann beispielsweise den Handgriff fortführen und/oder verlängern. Das kann bedeuten, dass der Datenübertragungsadapter einen Handgriffabschnitt aufweist. In dem verbundenen Zustand kann der Benutzer das Bildgebungsinstrument also derart verwenden, als sei das Bildgebungsinstrument bereits ohne das Vorsehen des Datenübertragungsadapters zur kabellosen Datenübertragung eingerichtet.

Zudem kann der Datenübertragungsadapter ein Bedienelement umfassen, mittels dessen der Datenübertragungsadapter in einen Kopplungsmodus zur Herstellung einer Kopplung mit einer Anzeigevorrichtung versetzbar ist. Eine Benutzerfreundlichkeit kann dadurch verbessert werden und der Datenübertragungsadapter und/oder das Bildgebungsinstrument leichter bedienbar sein. Das Bedienelement kann beispielsweise einen Druckknopf, einen Drehknopf, ein insbesondere berührungssensitives Bedienfeld wie etwa ein Touchscreen und/oder ein Touchpad, umfassen. In dem Kopplungsmodus kann eine Kopplung initiierbar, insbesondere ausgehend von dem Datenübertragungsadapter, und/oder bestätigbar sein. Beispielsweise kann der Datenübertragungsadapter eine Kopplung anfragen und/oder der Datenübertragungsadapter kann nach einer Bestätigung zur Durchführung einer Kopplung gefragt werden. Zur Bestätigung kann ausreichen, dass der Benutzer das Bedienelement drückt. Ferner kann, um eine Fehlbedienung zu verhindern, erforderlich sein, dass das Bedienelement über mehrere Sekunden gedrückt werden muss, um eine Kopplung herzustellen, zu initiieren und/oder zu bestätigen. Das Bedienelement kann zumindest im Wesentlichen ein Teil der/einer Kopplungseinheit und/oder der/einer Sicherheitseinheit sein.

Außerdem kann das medizinische System ein weiteres medizinisches Bildgebungsinstrument umfassen, wobei der Datenübertragungsadapter wahlweise mit dem Bildgebungsinstrument oder dem weiteren Bildgebungsinstrument verbindbar ist. In einfacher Weise können verschiedene Bildgebungsinstrumente zur kabellosen Datenübertragung vorgesehen werden. Dadurch muss nicht je Bildgebungsinstrument ein Datenübertragungsadapter vorgesehen werden. Insbesondere ein standardisierter Steckeranschluss kann eine breite Kompatibilität des Datenübertragungsadapters gewährleisten.

Außerdem kann das Verhindern der Übertragung der Bilddaten ein Lösen der Kopplung des Bildgebungsinstruments und der Anzeigevorrichtung umfassen. Vorteilhafterweise kann dadurch erreicht werden, dass eine Kopplung eines anderen Bildgebungsinstruments nicht verhindert und/oder blockiert wird und zugleich kann sichergestellt werden, dass Bilddaten nicht fälschlicherweise übertragen werden. Die Kopplung kann gelöst werden, nachdem Geräteinformation und/oder Information über einen Kopplungsversuch übertragen wurde. Ferner kann an der Anzeigevorrichtung eine Benachrichtigung einblendbar sein, die den Benutzer über einen Kopplungsversuch informiert.

Ferner kann die Sicherheitseinheit dazu eingerichtet sein, eine Aufforderung an den Benutzer zur Bestätigung der Kopplung zu erstellen. Dadurch kann ein bediensicherer und einfacher Kopplungsvorgang bereitgestellt werden. Die Aufforderung kann grundsätzlich jede Information und/oder jeden Hinweis an den Benutzer umfassen, dass er die Bestätigung durchführen soll. Beispielsweise kann ein Vibrationsalarm, ein Ton, ein Blinken einer LED, ein Farbwechsel einer Leuchtanzeige, ein Aufforderungstext in einem Bedienfeld, insbesondere in dem Bedienfeld des Datenübertragungsadapters, und/oder dergleichen von der Aufforderung umfasst sein.

Ferner kann die Anzeigevorrichtung dazu eingerichtet sein, die Aufforderung dem Benutzer anzuzeigen. Vorteilhafterweise hat der Benutzer sofort eine Rückmeldung darüber, mit welcher spezifischen Anzeigevorrichtung sich das Bildgebungsinstrument koppelt. Es kann ein besonders bediensicheres medizinisches System bereitgestellt werden. Ferner hat der Benutzer ohnehin häufig die Anzeigevorrichtung, mit der er das Bildgebungsinstrument koppeln möchte, im Blick. Dadurch ist das Koppeln intuitiv, einfach und sicher, da der Benutzer seinen Blick nicht von der Anzeigevorrichtung abwenden muss. Es kann eine Aufforderungsdarstellung an der Anzeigevorrichtung erzeugt werden und/oder eine Einblendung einer Darstellung der Anzeigevorrichtung überlagert werden. Die Aufforderung kann ferner eine Information über das Bildgebungsinstrument, das gekoppelt werden soll, anzeigen. Beispielsweise kann ein Bildgebungsinstrumentenname, ein das Bildgebungsinstrument kennzeichnendes Symbol, eine Identifizierungsnummer und/oder dergleichen gemeinsam mit der Aufforderung einblendbar sein.

Zudem kann das medizinische Bildgebungsinstrument ein Bedienelement umfassen und die Bestätigung der Kopplung ein Betätigen des Bedienelements umfassen. Eine für den Benutzer einfache und komfortable Durchführung der Kopplung kann erreicht werden. Das Bedienelement kann zumindest im Wesentlichen ähnliche Merkmale wie das Bedienelement des Datenübertragungsadapters aufweisen. Ferner kann das Bedienelement das Bedienelement des Datenübertragungsadapters umfassen. Das kann bedeuten, dass wenn der Datenübertragungsadapter angeschlossen ist, das Bedienelement des Datenübertragungsadapters das Bedienelement des Bildgebungsinstrument umfasst. Das Betätigen kann ein Drehen, Drücken, Tippen und/oder dergleichen umfassen.

Außerdem kann die Bestätigung der Kopplung ein Betätigen des Bedienelements über einen vorgegeben minimalen Bestätigungszeitabschnitt umfassen, wobei der Bestätigungszeitabschnitt vorzugsweise zumindest drei Sekunden und bevorzugt zumindest fünf Sekunden lang ist. Eine unbeabsichtigte Kopplung kann dadurch zumindest im Wesentlich verringert werden. Zumindest jedoch kann die Wahrscheinlichkeit einer unbeabsichtigten Kopplung verringert werden.

Ferner kann eine Anleitung zur Bestätigung gemeinsam mit der Aufforderung einblendbar sein und/oder die Aufforderung die Anleitung umfassen. Beispielsweise kann eine Information über den minimalen Bestätigungszeitabschnitt eingeblendet werden. Alternativ oder zusätzlich kann die Bestätigung der Kopplung ein Betätigen des Bedienelements über eine Betätigungsabfolge umfassen, für die eine Anleitung eingeblendet werden kann. Die Bestätigung kann beispielsweise ein dreimaliges Drücken des Bedienelements umfassen.

Zudem kann die Sicherheitseinheit dazu eingerichtet sein, bei einem Vorliegen der Bestätigung der Kopplung ein Übertragen der Bilddaten von dem Bildgebungsinstrument an die Anzeigevorrichtung zu gestatten. Es kann sichergestellt werden, dass Bilddaten erst nach der Bestätigung übertragen werden und somit der Benutzer kontrollieren und sicherstellen kann, dass Bilddaten lediglich an die richtige Anzeigevorrichtung übertragen werden. Es kann also die Bestätigung zur Bedingung dafür gestellt werden, dass Bilddaten übertragen werden.

Zudem kann die Anzeigevorrichtung ein Erkennungsmerkmal aufweisen und anhand des Erkennungsmerkmals identifizierbar sein und die Bestätigung der Kopplung kann ein Erfassen eines Bilds des Erkennungsmerkmals mittels des Bildgebungsinstruments umfassen. Vorteilhafterweise hat der Benutzer dadurch eine direkte Bestätigung, dass die richtige Anzeigevorrichtung gekoppelt wird. Ferner kann dadurch von einem Vorhersehen eines Bedienelements abgesehen werden, da die Kamera des Bildgebungsinstruments für das Bestätigen verwendet werden kann. Eine Kopplung kann besonders zuverlässig, sicher, komfortabel und schnell durchführbar sein. Das Erkennungsmerkmal kann dem Erkennungsmerkmal eines Aspektes der vorliegenden Erfindung entsprechen. Generell können diese Merkmale Bezug auf zumindest einen anderen Aspekt der vorliegenden Erfindung nehmen. Für eine weitreichendere Beschreibung sei auf diesen zumindest einen anderen Aspekt verwiesen.

Ferner kann die Anzeigevorrichtung dazu eingerichtet sein, das Erkennungsmerkmal anzuzeigen. Die Anzeigevorrichtung und das Bildgebungsinstrument können dadurch koppelbar sein, ohne dass bauliche und/oder konstruktive Maßnahmen erforderlich sind. Es können Funktionen der Anzeigevorrichtung und des Bildgebungsinstruments verwendet werden, die ohnehin vorgesehen sind. Das Erkennungsmerkmal kann beispielsweise mittels einer Einblendung anzeigbar sein, welche einer Darstellung überlagerbar ist. Ferner kann eine eigene Darstellung erzeugbar sein, die das Erkennungsmerkmal umfasst. Generell können diese Merkmale Bezug auf zumindest einen anderen Aspekt der vorliegenden Erfindung nehmen. Für eine weitreichendere Beschreibung sei auf diesen zumindest einen anderen Aspekt verwiesen.

Außerdem kann dem Bildgebungsinstrument eine erste Gerätekennung zugeordnet sein, der Anzeigevorrichtung eine zweite Gerätekennung zugeordnet sein, in der Kopplungseinheit eine Zuordnungsregel hinterlegt sein, die Kombinationen unterschiedlicher Gerätekennungen als zulässig oder unzulässig festlegt, und die Kopplungseinheit dazu eingerichtet sein, unter Anwendung der Zuordnungsregel die erste Gerätekennung mit der zweiten Gerätekennung zu vergleichen und eine Kopplung des Bildgebungsinstruments und der Anzeigevorrichtung nach Maßgabe der Anwendung der Zuordnungsregel herzustellen oder zu verhindern. Eine Betriebssicherheit kann dadurch verbessert werden. Es kann sichergestellt werden, dass lediglich gewünschte Paarungen von Bildgebungsinstrumenten und Anzeigevorrichtungen hergestellt werden. Es werden beispielsweise lediglich Paarungen gemäß der Zuordnungsregel eingerichtet. Die Gerätekennung kann dazu eingerichtet sein, ein Gerät eindeutig zuzuordnen und/oder zu identifizieren. Die Gerätekennung kann eine zumindest im Wesentlichen einzigartige Kennzeichnung für ein bestimmtes Gerät aufweisen. Beispielsweise kann die erste und/oder die zweite Gerätekennung eine Geräteidentifikationsnummer umfassen, anhand derer das Bildgebungsinstrument und/oder die Anzeigevorrichtung identifizierbar und/oder zuordenbar ist. Die Zuordnungsregel kann Gerätekennungen in Verhältnis zueinander setzen. Die Zuordnungsregel kann vor jedem Kopplungsvorgang aktualisierbar sein. Beispielsweise kann die Zuordnungsregel vor einem Kopplungsvorgang und/oder während eines Kopplungsvorgangs aus einem Cloudspeicher abrufbar sein. Eine Zuordnung verschiedener Geräte und/oder Instrumente kann dadurch zentral verwaltbar sein. Es kann mittels eines zentralen Datendienstes, insbesondere mittels einer Cloud, eine Zulässigkeit von Kombinationen verwaltbar und/oder steuerbar sein. Es kann beispielsweise in einer Gesundheitseinrichtungsinventursoftware eine Kombination einstellbar sein, welche über den zentralen Datendienst die Kombination an die Kopplungseinheit sendet. Ferner kann der Benutzer anhand einer Listenauswahl die Zuordnungsregel erstellen. Beispielsweise kann der Benutzer an der Anzeigevorrichtung und/oder einem mobilen Endgerät die Zuordnungsregel, insbesondere anhand einer Listenauswahl, erstellen. Diese neu erstellte Zuordnungsregel kann in der Kopplungseinheit hinterlegbar sein und/oder die Zuordnungsregel, die bereits in der Kopplungseinheit hinterlegt ist, kann anhand der neu erstellten Zuordnungsregel aktualisierbar sein. Es kann für ein Bildgebungsinstrument lediglich eine Kombination an Gerätekennungen hinterlegbar sein. Dadurch kann sichergestellt werden, dass nur an eine bestimmte Anzeigevorrichtung Bilddaten übertragbar sind. Alternativ oder zusätzlich können mehrere Kombinationen hinterlegt sein. Dadurch kann etwa ein mehrschichtiges Kopplungssicherheitsverfahren durchführbar sein. Es können bestimmte Kombinationen ausgeschlossen sein, und eine zulässige Kombination kann in einem weiteren Bestätigungsschritt bestätigbar sein.

Außerdem kann die Sicherheitseinheit dazu eingerichtet sein, eine Benutzerberechtigung des Benutzers zu ermitteln, die Benutzerberechtigung mit einer vorgegebenen erforderlichen Berechtigung zu vergleichen, und die Übertragung der Bilddaten von dem Bildgebungsinstrument an die Anzeigevorrichtung zu verhindern, wenn die Benutzerberechtigung die vorgegebene erforderliche Berechtigung nicht erreicht. Dadurch kann eine Benutzung des Bildgebungsinstruments für unbefugte Benutzer verhindert werden. Ferner kann eine Benutzerberechtigung zentral freischaltbar sein und dadurch beispielsweise ein benutzungsbasiertes Bezahlsystem und/oder Registriersystem implementierbar sein. Der Benutzer kann beispielsweise eine Benutzung entgeltlich freischalten lassen und/oder über ein Abonnement über einen Zeitraum für die Benutzung des Bildgebungsinstrument befugt sein. Ein Erwerben und/oder ein Freischalten der Benutzerberechtigung kann beispielsweise über ein mobiles Endgerät wie ein Smartphone durchführbar sein. Der Erwerb und/oder das Freischalten kann etwa in einer Art eines Einkaufs in einem App-Store durchführbar sein. Alternativ oder zusätzlich kann die Berechtigung auch durch einen zentralen Geräteverwaltungsdienst einer Gesundheitseinrichtung durchführbar sein. Dadurch kann eine bessere Kontrolle und/oder Übersicht über den Einsatz von Geräten, insbesondere von Bildgebungsinstrumenten, erreicht werden. Unter "nicht erreicht" kann verstanden werden, dass die Berechtigung eine Berechtigung über einzelne Funktionen umfasst. Es kann beispielsweise eine Berechtigung für einzelne Funktionen des Bildgebungsinstruments erwerbbar sein. Ein Benutzer kann etwa eine Berechtigung für die Erzeugung und/oder Übertragung von SD-Bilddaten haben, jedoch eine Erzeugung und/oder Übertragung von HD-Bilddaten wünschen. In diesem Falle erreicht die Berechtigung die vorgegebene erforderliche Berechtigung nicht. Der Benutzer kann die erforderliche Berechtigung jedoch erwerben, beispielsweise wieder mittels des mobilen Endgeräts in der Art eines Einkaufs in einem App-Store.

Ferner kann das medizinische System eine Speichereinheit, in der Information über die Kopplung des Bildgebungsinstruments mit der Anzeigevorrichtung und/oder die Bestätigung der Kopplung durch den Benutzer hinterlegt ist, umfassen. Dadurch kann nachvollziehbar sein, welches Bildgebungsinstrument mit welcher Anzeigevorrichtung gekoppelt wurde. Die Information kann beispielsweise einen Kopplungsversuch, zumindest eine Gerätekennung, eine Benutzerkennung, anhand der der Benutzer des Bildgebungsinstruments identifizierbar ist, eine Kopplungsdauer, eine Benutzungsdauer, eine Bilddatenmenge, eine Benutzungsart und/oder dergleichen umfassen. Die Speichereinheit kann eine bereits beschriebene Speichereinheit zumindest teilweise umfassen. In einer Ausführungsform kann die Speichereinheit eine cloudbasierte Speichereinheit umfassen und/oder mit einer cloudbasierten Speichereinheit in Verbindung stehen. Dadurch kann die Information zentral speicherbar und/oder zugänglich sein. Es kann etwa zu Analysezwecken und/oder Abrechnungszecken die Information, insbesondere statistisch, auswertbar sein.

Zudem kann das Objekt eine Anzeigevorrichtung umfassen, die dazu eingerichtet ist, Bilddaten zu empfangen und eine Darstellung für einen Benutzer zu erzeugen. Eine Anzeigevorrichtung ist üblicherweise zum Betrieb des Bildgebungsinstruments vorgesehen. Vorteilhafterweise kann die Anzeigevorrichtung in den Aktivierungsprozess eingebunden werden. Die Anzeigevorrichtung kann der bereits beschriebenen Anzeigevorrichtung eines Aspektes der vorliegenden Erfindung entsprechen. Generell können diese Merkmale Bezug auf zumindest einen anderen Aspekt der vorliegenden Erfindung nehmen. Für eine weitreichendere Beschreibung sei auf diesen zumindest einen anderen Aspekt verwiesen.

Außerdem kann die Anzeigevorrichtung dazu eingerichtet sein, das Erkennungsmerkmal anzuzeigen. Vorteilhafterweise kann bei Bedarf das Erkennungsmerkmal erzeugt und/oder angezeigt werden. Es ist eine flexible Aktivierung durchführbar. Das Erkennungsmerkmal kann insbesondere bedarfsweise anzeigbar sein. Es wird lediglich angezeigt und/oder ist lediglich vorhanden, wenn es benötigt wird. Es muss kein Bauraum für das Erkennungsmerkmal bereitgestellt werden und es kann aktuellen Anforderungen genügen. Beispielsweise kann das Erkennungsmerkmal anwendungsspezifisch und/oder bildgebungsinstrumentenspezifisch vorgesehen werden. Es kann bedarfsweise erzeugbar sein und zumindest ein für einen Aktivierungsvorgang spezifisches Merkmal aufweisen. Das bedeutet, dass, wenn eine Aktivierung durchgeführt werden soll, ein Erkennungsmerkmal generierbar ist, wobei das Erkennungsmerkmal spezifisch für die durchzuführende Aktivierung vorgesehen ist und/oder das spezifische Merkmal aufweist. Beispielsweise kann, wenn das Erkennungsmerkmal einen Zahlencode umfasst, der Zahlencode spezifisch für einen bestimmten Aktivierungsvorgang generierbar sein. Das Erkennungsmerkmal kann mittels einer Einblendung anzeigbar sein, die einer anderen Darstellung überlagerbar ist. Ferner kann das Erkennungsmerkmal eine Darstellung umfassen, die zumindest im Wesentlichen einen gesamten Anzeigeabschnitt wie etwa den Bildschirm der Anzeigevorrichtung ausfüllt.

Ferner kann die auf das Objekt bezogene Aktion eine Kopplung des Bildgebungsinstruments mit der Anzeigevorrichtung sein. Dadurch kann die Kopplung besonders einfach, schnell, zuverlässig, sicher und/oder komfortabel durchführbar sein. Ferner kann für die Kopplung lediglich auf Komponenten zurückgegriffen werden, die ohnehin vorhanden sind. Es kann vorteilhafterweise ausgenutzt werden, dass das Bildgebungsinstrument ohnehin eine Kamera aufweist und die Anzeigevorrichtung für die Anzeige von Darstellungen ausgebildet ist. Es kann ein gemeinsames Wirken von Merkmalen der Anzeigevorrichtung und/oder des Bildgebungsinstruments vorsehbar sein. Eine Konstruktion und/oder Herstellung kann kostengünstiger durchführbar sein. Will der Benutzer beispielsweise das Bildgebungsinstrument mit einer bestimmten Anzeigevorrichtung koppeln, kann er veranlassen, dass auf der zu koppelnden bestimmten Anzeigevorrichtung das Erkennungsmerkmal angezeigt wird. Dieses Erkennungsmerkmal kann er mit dem zu koppelnden Bildgebungsmerkmal erfassen. Dadurch kann sichergestellt werden, dass die beiden richtigen Geräte miteinander gekoppelt werden. Der Benutzer hat sofort eine Rückmeldung darüber, dass die beiden richtigen Geräte miteinander gekoppelt werden. Bleibt beispielsweise das Anzeigen des Erkennungsmerkmals auf der bestimmten Anzeigevorrichtung aus, kann die Kopplung nicht durchgeführt werden und/oder hat der Benutzer sofort Rückmeldung über ein Problem bei der Kopplung und/oder Zuordnung von Anzeigevorrichtungen.

Zudem kann das medizinische Bildgebungsinstrument dazu eingerichtet sein, in einem Erkennungsmodus das Bild des Erkennungsmerkmals des Objekts derart zu erfassen und/oder zugehörige Bilddaten derart zu erzeugen, dass zumindest Bildbereiche, die das Erkennungsmerkmal nicht enthalten, unkenntlich sind. Es kann sichergestellt werden, dass lediglich scharfe und/oder detaillierte Bilder des Erkennungsmerkmals erfassbar sein können. Die Umgebung des Erkennungsmerkmals und/oder der Anzeigevorrichtung, auf der das Erkennungsmerkmal anzeigbar sein kann, kann unkenntlich sein. Wenn beispielsweise ein Patient innerhalb eines Aufnahmebereichs der Kamera des Bildgebungsinstruments ist, kann sichergestellt werden, dass der Patient nicht anhand des Bildes des Erkennungsmerkmal identifizierbar ist. Dies kann beispielsweise durch rechtliche Bestimmungen notwendig sein, insbesondere wenn das Bild des Erkennungsmerkmals weiterverarbeitet wird und/oder zentral, insbesondere in einer Cloud, gespeichert wird. Gemäß einigen Ausführungsformen sind zumindest im Wesentlichen alle Bildbereiche des Bilds unkenntlich und/oder unscharf. Mit "unkenntlich" kann gemeint sein, dass zumindest im Wesentlichen keine Details, insbesondere gegenständliche und/oder körperliche Details, zumindest für das menschliche Auge erkennbar sind. Unkenntlich kann beispielsweise unscharf, verwischt, unterbelichtet und/oder überbelichtet umfassen. Diese Merkmale grenzen sich von einer nachträglichen Unkenntlichmachung der Bildbereiche ab, die das Erkennungsmerkmal nicht enthalten. Beispielsweise ein nachträgliches Anwenden eines Filters auf diese Bildbereiche ist durch diese Merkmale nicht umfasst. Die Bilddaten können derart erzeugbar sein, dass die Bildbereiche gemäß der ursprünglichen Bilddaten bereits unkenntlich sind. Die Erkennungseinheit kann dazu eingerichtet sein, das Erkennungsmerkmal in einem unkenntlichen Bild des Erkennungsmerkmal zu erkennen.

Außerdem können die Bildbereiche aufgrund einer Defokussierung der Kamera unkenntlich sein. In einfacher und kostengünstiger Weise kann sichergestellt werden, dass keine kenntlichen Bilder von Patienten aufgenommen werden. Mit der Defokussierung kann insbesondere gemeint sein, dass die Kamera derart defokussiert wird, dass unter üblichen Umständen und bei gängigem Gebrauch des Bildgebungsinstruments ein kenntliches Bild eines Patienten erzeugt würde. Beispielsweise kann die Kamera auf einen besonders kleinen und/oder auf einen besonders großen Brennpunkt fokussiert werden. Besonders klein und/oder groß ist im Zusammenhang mit einem üblichen Brennpunkt zu sehen. Der Brennpunkt kann beispielsweise dreimal, viermal, fünfmal, zehnmal und/oder zwanzigmal so klein und/oder groß sein, wie ein Minimalwert und/oder ein Maximalwert des Brennpunkts während eines üblichen Einsatzes des Bildgebungsinstruments etwa zur Durchführung einer therapeutischen und/oder diagnostischen Aktion. Die Kamera kann in dem Kopplungsmodus defokussierbar sein. Wird das Bildgebungsinstrument beispielsweise in den Kopplungsmodus versetzt, wird die Defokussierung automatisch eingestellt. In diesem Zustand kann der Benutzer keine kenntlichen Bilder des Patienten aufnehmen.

Außerdem kann das Erkennungsmerkmal derart beschaffen sein, dass es in einem Bild des Erkennungsmerkmals erkennbar ist, das in einem defokussierten Zustand der Kamera aufgenommen ist. Vorteilhafterweise muss nicht sichergestellt werden, dass ein kenntliches Bild des Erkennungsmerkmals erfasst wird. Auch mittels unkenntlicher Bilder kann eine Kopplung und/oder Aktivierung durchführbar sein. Eine technischer und/oder konstruktiver Anspruch an das medizinische System kann dadurch verringert werden. Das medizinische System kann wesentlich weniger komplex sein und gleichzeitig zuverlässiger und/oder bediensicherer. Das Erkennungsmerkmal kann beispielsweise derart beschaffen sein, dass für ein Erkennen kein kenntliches Bild, insbesondere Bild aufweisend gegenständliche und/oder körperliche Details, notwendig ist. Der defokussierte Zustand kann der Defokussierung entsprechen.

Ferner kann das Erkennungsmerkmal einen Farbcode umfassen. Ein Farbcode kann derart beschaffen sein, dass er in einem Bild des Erkennungsmerkmals erkennbar ist, das in dem defokussierten Zustand der Kamera aufgenommen ist. Es muss folglich kein kenntliches Bild des Erkennungsmerkmals erfasst werden. Der Farbcode kann einen Streifencode, (verschieden-)farbige Kacheln und/oder ein farbiges Muster umfassen.

Ferner kann das Erkennungsmerkmal eine zeitliche Farbabfolge umfassen. Eine Farbabfolge kann derart beschaffen sein, dass sie in einem Bild des Erkennungsmerkmals erkennbar ist, das in dem defokussierten Zustand der Kamera aufgenommen ist. Es muss folglich kein kenntliches Bild des Erkennungsmerkmals erfasst werden. Die Farbabfolge kann ein aufeinanderfolgendes Einblenden einer monofarbigen Darstellung in verschiedenen Farben umfassen. Das Einblenden kann eine Darstellung über zumindest im Wesentlichen den gesamten Anzeigeabschnitt der Anzeigevorrichtung umfassen. Eine Farbabfolge kann beispielsweise zumindest zwei, drei, vier, fünf, sechs und/oder sieben Farben und/oder farbige Darstellungen umfassen. Ein Beispiel für eine Farbabfolge kann Grün-Rot-Grün, Rot-Blau-Gelb-Rot und/oder Blau-Rot-Rot-Blau sein. Die Darstellungen können mit Kadenz von einer Darstellung pro Sekunde, pro halber Sekunde und/oder pro viertel Sekunde anzeigbar sein. Gemäß einigen Ausführungsformen kann die Kadenz einer Bildwiederholrate der Anzeigevorrichtung entsprechen.

Ferner kann das Erkennungsmerkmal eine zeitliche Hell-Dunkel-Abfolge umfassen. Eine Hell-Dunkel-Abfolge kann derart beschaffen sein, dass sie in einem Bild des Erkennungsmerkmals erkennbar ist, das in dem defokussierten Zustand der Kamera aufgenommen ist. Es muss folglich kein kenntliches Bild des Erkennungsmerkmals erfasst werden. Die Hell-Dunkel-Abfolge kann ein aufeinanderfolgendes Einblenden einer monofarbigen Darstellung, insbesondere einer Weißen und/oder einer Schwarzen Darstellung, umfassen. Die Darstellung kann in unterschiedlichen Helligkeitswerten erzeugbar sein. Insbesondere kann die zeitliche Hell-Dunkel-Abfolge das Einblenden einer gleichen Darstellung mit unterschiedlichen Helligkeitswerten umfassen. Eine Farbabfolge kann beispielsweise zumindest zwei, drei, vier, fünf, sechs und/oder sieben unterschiedlich helle Darstellungen umfassen. Die Hell-Dunkel-Abfolge kann pulsierend sein. Das kann bedeuten, dass es einen weichen Übergang zwischen hellen und dunklen Darstellungen geben kann. Die Zeitabfolge des Helligkeitswerts kann beispielsweise mittels einer Kurvenfunktion, insbesondere einer Sinus-Funktion, beschreibbar sein. Die Darstellungen können mit Kadenz von einer Darstellung pro Sekunde, pro halber Sekunde und/oder pro viertel Sekunde anzeigbar sein. Gemäß einigen Ausführungsformen kann die Kadenz einer Bildwiederholrate der Anzeigevorrichtung entsprechen.

Außerdem kann das Erkennungsmerkmal einen Barcode umfassen. Barcodes haben sich als zuverlässige und günstige Erkennungsmerkmale erwiesen. Der Barcode kann erzeugbar und/oder anzeigbar sein. Mittels des Barcodes kann zusätzliche Information übertragbar sein, wenn ein Bild des Barcodes erzeugt und/oder analysiert wird.

Außerdem kann das Erkennungsmerkmal einen QR-Code umfassen. QR-Codes haben sich als zuverlässige und günstige Erkennungsmerkmale erwiesen. Der QR-Code kann erzeugbar und/oder anzeigbar sein. Mittels des QR-Codes kann zusätzliche Information übertragbar sein, wenn ein Bild des QR-Codes erzeugt und/oder analysiert wird.

Zudem kann das Erkennungsmerkmal mit einer Instrumentenkennung verknüpft sein. Wird das Erkennungsmerkmal erkannt, kann anhand der Instrumentenkennung sicher und zuverlässig auf ein bestimmtes Bildgebungsinstrument geschlossen werden. Die Instrumentenkennung erlaubt ein zumindest im Wesentlichen lückenloses Nachverfolgen von Einsätzen des Bildgebungsinstruments und/oder von Funktionen des Bildgebungsinstruments.

Ferner kann das Objekt eine Patientenakte umfassen. Dadurch kann etwa auf einen bestimmten Patienten rückschließbar sein und/oder ein Einsatz eines Bildgebungsinstruments im Zusammenhang mit einem bestimmten Patienten rückverfolgbar sein. Es kann etwa ein Barcode und/oder ein QR-Code in der Patientenakte abgedruckt sein, der eindeutig dem Patienten zugeordnet ist und anhand dessen der Patient identifizierbar ist. Wenn mittels der Kamera ein Bild des Erkennungsmerkmals erfasst wird, welches mittels der Erkennungseinheit erkannt wird, kann die Steuereinheit eine scharfe, fokussierte Bildaufnahmefunktion freischalten und beispielsweise erzeugte Bilddaten eindeutig dem Patienten zuordnen. Die erzeugten Bilddaten und/oder auf den Bilddaten beruhende Information kann zuverlässig und sicher einer elektronischen Patientenakte des Patienten zuordenbar sein und in dieser hinterlegbar sein.

Außerdem kann das Objekt ein Kleidungsstück umfassen. Anhand des Kleidungsstücks kann auf die Identität des Benutzers und/oder des Patienten rückgeschlossen werden. Beispielsweise kann ein Namensschild, ein Barcode, ein QR-Code und/oder dergleichen an dem Kleidungsstück befestigt und/oder in dieses integriert sein. Wenn das Erkennungsmerkmal identifiziert wird, kann auf den Benutzer rückgeschlossen werden und/oder es kann hinterlegbar sein, welcher Benutzer, insbesondere wann und/oder wo, das Bildgebungsinstrument verwendet hat. Ferner können Maßnahmen ergreifbar sein wie etwa im Zusammenhang mit der Patientenakte.

Ferner kann das Verfahren zum Betrieb eines medizinischen Systems den Schritt eines Erkennens des Vorhandenseins des Erkennungsmerkmals in dem mittels der Kamera aufgenommenen Bild des Erkennungsmerkmals, den Schritt eines Erzeugens eines Erkennungssignals nach Maßgabe der Erkennung des Vorhandenseins des Erkennungsmerkmals und den Schritt eines Aktivierens zumindest einer auf das Objekt bezogenen Funktion des medizinischen Systems nach Maßgabe des Erkennungssignals umfassen.

Wenn das Objekt eine Anzeigevorrichtung umfasst, die dazu eingerichtet ist, Bilddaten zu empfangen und eine Darstellung für einen Benutzer zu erzeugen, kann das Verfahren zum Betrieb eines medizinischen Systems ferner den Schritt eines Anzeigens des Erkennungsmerkmals auf der Anzeigevorrichtung umfassen.

Zudem kann die Gestenerkennungseinheit an der Anzeigevorrichtung angeordnet sein. Vorteilhafterweise kann ein kompaktes medizinisches System bereitgestellt werden, das insbesondere bedarfsweise und/oder zügig in einem neuen Behandlungszimmer aufbaubar ist. Die Gestenerkennungseinheit kann beispielsweise ein anbaubares und/oder hinzuschaltbares Modul umfassen, das an einer bestehenden Anzeigevorrichtung angeordnet werden kann. Ein medizinisches System kann dadurch erweiterbar sein.

Außerdem kann das medizinische System eine weitere Anzeigevorrichtung umfassen, die dazu eingerichtet ist, Bilddaten zu empfangen und eine Darstellung für einen Benutzer zu erzeugen, wobei die weitere Anzeigevorrichtung mit dem medizinischen Bildgebungsinstrument gekoppelt ist und wobei die Gestenerkennungseinheit dazu eingerichtet ist, die auf die Anzeigevorrichtung gerichtete Geste des Benutzers zu erkennen und nach Maßgabe der Erkennung der Geste eine Kopplung zwischen dem Bildgebungsinstrument und der Anzeigevorrichtung zusätzlich zu der Kopplung mit der weiteren Anzeigevorrichtung derart herzustellen, dass auf den Bilddaten beruhende Darstellungen sowohl auf der Anzeigevorrichtung als auch auf der weiteren Anzeigevorrichtung anzeigbar sind. Vorteilhafterweise kann die Anzeigevorrichtung hinzuschaltbar sein, wobei die weitere Anzeigevorrichtung bereits gekoppelt ist. Die weitere Anzeigevorrichtung kann beispielsweise ein mobiler tragbarer Monitor für das Bildgebungsinstrument sein, auf dem der Benutzer eine Aktion beobachten kann. Dieser Monitor kann klein sein, was bedeutet, dass er dazu vorgesehen ist, von lediglich einem Benutzer benutzt zu werden. Damit weitere Benutzer und/oder Beobachter die Aktion beobachten können, kann die Anzeigevorrichtung mittels der Geste des Benutzers hinzuschaltbar sein. Die Anzeigevorrichtung kann in dem Fall etwa ein großer Bildschirm in einem Operationssaal sein. Auf beiden Anzeigevorrichtungen kann zumindest im Wesentlichen dieselbe Darstellung darstellbar sein. Die Darstellung kann zentral generierbar sein und Daten an die Anzeigevorrichtungen übertragbar sein, anhand derer die Anzeigevorrichtung die Darstellung erzeugt. Beispielsweise können beide Anzeigevorrichtungen mit einer zentralen Recheneinheit verbunden sein, welche dazu eingerichtet ist, die Darstellung zu generieren.

Zudem kann die Gestenerkennungseinheit an der weiteren Anzeigevorrichtung angeordnet sein. Vorteilhafterweise kann eine Flexibilität erhöhbar sein und das medizinische System als ein Modulsystem bereitstellbar sein. Zusätzliche Rechenkapazitäten und/oder dergleichen können hinzuschaltbar sein. Die Gestenerkennungseinheit kann gemeinsam an der Anzeigevorrichtung und der weiteren Anzeigevorrichtung angeordnet sein.

Zudem kann das Bildgebungsinstrument und die Anzeigevorrichtung derart koppelbar sein, dass die Bilddaten und/oder die Darstellung betreffende Information von der weiteren Anzeigevorrichtung an die Anzeigevorrichtung übertragbar sind. Vorteilhafterweise kann ein Zusammenwirken von den Anzeigevorrichtungen erreicht werden. Nicht jede Anzeigevorrichtung muss ausreichend Rechenkapazitäten, insbesondere graphische Rechenkapazitäten, aufweisen, um die Darstellung zu erzeugen. Es kann beispielsweise eine zentrale Anzeigevorrichtung vorgesehen werden, welche die Darstellung erzeugt und diese und/oder die Darstellung betreffende Information und/oder Daten an die anderen Anzeigevorrichtungen überträgt. In einfacher und/oder effizienter Weise können Anzeigevorrichtungen hinzuschaltbar sein. Es kann beispielsweise eine Darstellung dupliziert werden und/oder gespiegelt werden.

Zudem kann die Gestenerkennungseinheit zumindest ein Gestenerkennungsmodul umfassen und/oder durch Zuschalten weiterer Gestenerkennungsmodule erweiterbar sein. Vorteilhafterweise kann das medizinische System modular bereitstellbar sein und/oder effizient erweiterbar sein. Zum Beispiel kann das medizinische System schnell und einfach an eine neue Arbeitsumgebung wie etwa einem neuen Behandlungszimmer angepasst werden. Es kann auf räumliche Begebenheiten Rücksicht genommen werden. Ferner kann, wenn der Benutzer etwa teilweise durch eine weitere medizinische Vorrichtung, beispielsweise einem OP-Roboter, für das Gestenerkennungsmodul verdeckt ist, kann einfach und schnell ein weiteres Gestenerkennungsmodul hinzugeschaltet werden. Ein Gestenerkennungsmodul kann etwa eine Kamera, einen elektromagnetischen Sensor und/oder dergleichen umfassen. Das Gestenerkennungsmodul kann dazu eingerichtet sein, eine Bewegung des Benutzers und/oder des Bildgebungsinstruments zu bestimmen. Anhand der Maßgabe der Bewegung kann die Gestenerkennungseinheit dazu eingerichtet sein, die Geste zu erkennen. Gemäß einigen Ausführungsformen können mehrere Gestenerkennungsmodule fest an einer Wand eines Behandlungszimmers befestigt sein. Sollte sich herausstellen, dass die vorhandenen Gestenerkennungsmodule nicht ausreichen, um eine Geste zuverlässig zu erkennen, kann bedarfsweise ein weiteres Gestenerkennungsmodul hinzugeschaltet werden. Ist das Gestenerkennungsmodul eine Kamera, kann diese auf einem tragbaren Stativ angeordnet werden und zur Gestenerkennung ausgerichtet werden.

Ferner kann das medizinische System mehrere Anzeigevorrichtungen umfassen, die jeweils dazu eingerichtet sind, Bilddaten zu empfangen und eine Darstellung für einen Benutzer zu erzeugen, wobei die Gestenerkennungseinheit dazu eingerichtet ist, eine auf eine bestimmte Anzeigevorrichtung der Anzeigevorrichtungen gerichtete Geste des Benutzers zu erkennen und nach Maßgabe der Erkennung der auf die bestimmte Anzeigevorrichtung der Anzeigevorrichtungen gerichteten Geste eine Kopplung zwischen dem Bildgebungsinstrument und der bestimmten Anzeigevorrichtung der Anzeigevorrichtungen herzustellen. Vorteilhafterweise kann die Gestenerkennungseinheit erkennen, auf welche Anzeigevorrichtung genau die Geste gerichtet ist, und diese Anzeigevorrichtung kann hinzugeschaltet werden. Der Benutzer kann sich also entscheiden, welche von mehreren Anzeigevorrichtungen er hinzuschalten möchte. Es ist üblich, dass mehrere Anzeigevorrichtung in Behandlungsräumen angeordnet sind. Daher ist es vorteilhaft, dass die Gestenerkennungseinheit die auf eine bestimmte Anzeigevorrichtung gerichtete Geste erkennt. Es können die Anzeigevorrichtungen nacheinander hinzugeschaltet werden.

Außerdem kann die Gestenerkennungseinheit ein stereoskopisches Kamerasystem umfassen. Vorteilhafterweise kann eine Geste räumlich erfassbar sein und Abstände bestimmbar sein. Eine Zuverlässigkeit und Genauigkeit der Gestenerkennungseinheit werden dadurch verbessert. Mittels des stereoskopischen Kamerasystems können Stereobilder erzeugbar sein. Beispielsweise umfasst das stereoskopische Kamerasystem zumindest zwei Bilderfassungseinheiten, die von dem gleichen Objektbereich Bilder erzeugen. Anhand einer Disparität zwischen den Bildern kann ein räumlich aufgelöstes Bild, insbesondere ein Stereobild, erzeugbar sein. Das stereoskopische Kamerasystem kann etwa durch zumindest zwei Gestenerkennungsmodule ausgebildet sein. Alternativ oder zusätzlich kann ein Gestenerkennungsmodul das stereoskopische Kamerasystem umfassen und/oder ausbilden.

Ferner kann das medizinische System ein Mikrofon umfassen, das dazu eingerichtet ist, ein Audiosignal zu empfangen, wobei die Gestenerkennungseinheit dazu eingerichtet ist, das Audiosignal zu erkennen und nach Maßgabe der Erkennung des Audiosignals die Kopplung zwischen dem Bildgebungsinstrument und der Anzeigevorrichtung zu verhindern oder zu gestatten. Dadurch kann eine Bediensicherheit verbessert werden und eine Gefahr einer ungewollten Kopplung reduziert werden. Das Audiosignal kann ein von dem Benutzer erzeugtes Geräusch umfassen. Beispielsweise kann das Audiosignal ein mittels der Stimmbänder des Benutzers generierte Signal, insbesondere einen Sprachbefehl, umfassen und/oder eine Klanggeste umfassen, etwa ein Klatschen, ein Klopfen, ein Pfeifen und/oder Stampfen des Benutzers. Insbesondere kann die Gestenerkennungseinheit zur Spracherkennung eingerichtet sein. Dadurch kann die Gestenerkennungseinheit ein Audiosignal, das einen Sprachbefehl umfasst, interpretieren. Ein Sprachbefehl kann beispielsweise einen Befehl zur Kopplung umfassen. Durch eine Erkennung des Befehls kann ein Koppelmodus aktivierbar sein, in dem die Kopplung möglich ist.

Im Folgenden wird die vorliegende Erfindung anhand der beigefügten Figuren beispielhaft beschrieben. Die Zeichnung, die Beschreibung und die Ansprüche enthalten zahlreiche Merkmale in Kombination. Der Fachmann wird die Merkmale zweckmäßigerweise auch einzeln betrachten und im Rahmen der Ansprüche sinnvoll in Kombination verwenden.

Falls von einem bestimmten Objekt mehr als ein Exemplar vorhanden ist, ist ggf. nur eines davon in den Figuren und in der Beschreibung mit einem Bezugszeichen versehen. Die Beschreibung dieses Exemplars kann entsprechend auf die anderen Exemplare von dem Objekt übertragen werden. Sind Objekte insbesondere mittels Zahlenwörtern, wie beispielsweise erstes, zweites, drittes Objekt etc. benannt, dienen diese der Benennung und/oder Zuordnung von Objekten. Demnach können beispielsweise ein erstes Objekt und ein drittes Objekt, jedoch kein zweites Objekt umfasst sein. Allerdings könnten anhand von Zahlenwörtern zusätzlich auch eine Anzahl und/oder eine Reihenfolge von Objekten ableitbar sein.

Es zeigen:
- Fig. 1: eine schematische Darstellung eines medizinischen Systems;
- Fig. 2: eine schematische Darstellung einer Ansicht auf einen Datenübertragungsadapter;
- Fig. 3: eine schematische Darstellung eines Ausschnitts des medizinischen Systems;
- Fig. 4: eine schematische Darstellung eines medizinischen Systems;
- Fig. 5: eine schematische Darstellung eines medizinischen Systems;
- Fig. 6: eine weitere schematische Darstellung des medizinischen Systems;
- Fig. 7: eine schematische Darstellung eines Bilds eines Erkennungsmerkmals;
- Fig. 8: ein schematisches Ablaufdiagramm eines Verfahrens;
- Fig. 9: ein schematisches Ablaufdiagramm eines Verfahrens;
- Fig. 10: ein schematisches Ablaufdiagramm eines Verfahrens;
- Fig. 11: ein schematisches Ablaufdiagramm eines Verfahrens; und
- Fig. 12: ein schematisches Ablaufdiagramm eines Verfahrens.

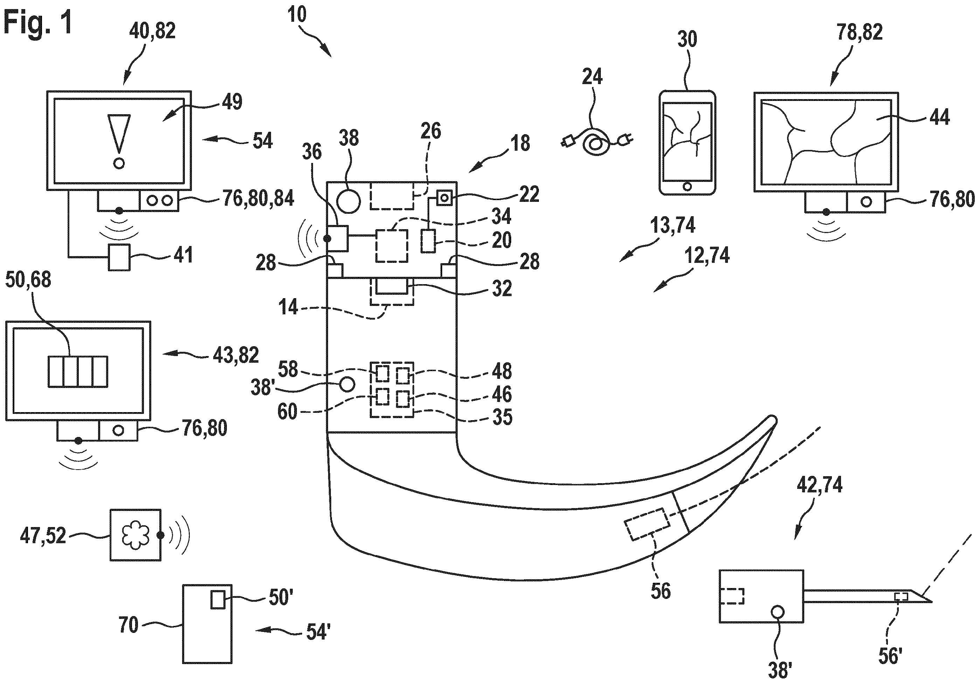

Fig. 1 zeigt ein medizinisches System 10 umfassend mehrere Anzeigevorrichtungen 82, insbesondere eine Anzeigevorrichtung 40, eine weitere Anzeigevorrichtung 43 und eine weitere Anzeigevorrichtung 78, ein mobiles Endgerät 30, einen Datenübertragungsadapter 18, ein medizinisches Bildgebungsinstrument 12, ein weiteres medizinisches Bildgebungsinstrument 42, eine Gestenerkennungseinheit 76 und eine Speichereinheit 52. Die Anzeigevorrichtungen 82 können miteinander austauschbar sein. Das bedeutet, dass sie zumindest im Wesentlichen dieselben Merkmale aufweisen können. Die Zuordnung der Bezugszeichen kann diesbezüglich willkürlich sein.

Das medizinische Bildgebungsinstrument 12 umfasst eine Kamera 56 und einen Steckeranschluss 14, an den üblicherweise ein Datenkabel (nicht dargestellt) angeschlossen wird, um Bilddaten, die mittels der Kamera 56 erzeugt werden, an zumindest eine der mehreren Anzeigevorrichtungen 82 zu übertragen. Statt des Datenkabels ist der Datenübertragungsadapter 18 an den Steckeranschluss 14 angeschlossen. Der Datenübertragungsadapter 18 ist dazu eingerichtet, Bilddaten über den Steckeranschluss zu empfangen und auf den Bilddaten beruhende Daten kabellos zu übertragen. Dadurch ist das Bildgebungsinstrument 12 zur kabellosen Datenübertragung eingerichtet. Wahlweise kann der Datenübertragungsadapter 18 an das weitere medizinische Bildgebungsinstrument 42 angeschlossen werden, um dieses zur kabellosen Datenübertragung einzurichten. Die Bildgebungsinstrumente 12, 42 sind vorliegend Endoskope 72. Das Bildgebungsinstrument 12 ist ein Laryngoskop und das Bildgebungsinstrument 42 ein Endoskop 72 mit starrem Schaft. Der Datenübertragungsadapter 18 kann jedoch grundsätzlich mit beliebigen Bildgebungsinstrumenten kompatibel sein.

Das Bildgebungsinstrument 12 und der Datenübertragungsadapter 18 bilden ein von einem Benutzer als einzelnes Instrument handhabbares Bildgebungsinstrument 13 aus. Das bedeutet, das Bildgebungsinstrument 13 ist derart verwendbar, als hätte es eine integrierte Vorrichtung zur kabellosen Datenübertragung. Das Bildgebungsinstrument 12 ist dadurch vorwärts kompatibel, es kann also mit neuen Anzeigevorrichtungen betrieben werden, die lediglich zur kabellosen Datenübertragung eingerichtet sind. Mittels des Datenübertragungsadapters 18 kann das Datenkabel ersetzt werden. Im Folgenden werden einige Merkmale eines kabellosen Bildgebungsinstruments mit Bezug auf das Bildgebungsinstrument 12 beschrieben. Grundsätzlich kann es unerheblich sein, ob das Bildgebungsinstrument 12 oder ein Bildgebungsinstrument, das ohne Datenübertragungsadapter 18 zu einer kabellosen Datenübertragung eingerichtet ist, gemeint ist. Anders gesagt, kann es unerheblich sein, wie die Daten kabellos übertragen werden. Insbesondere kann mit dem Bildgebungsinstrument 12 ein Bildgebungsinstrument gemeint sein, das in einem verbundenen Zustand mit dem Datenübertragungsadapter 18 ist, also das Bildgebungsinstrument 13.

Ferner können mittels des Datenübertragungsadapters 18 Bilddaten unmittelbar an das mobile Endgerät 30, vorliegend ein Smartphone, übertragen werden. Dadurch kann das Bildgebungsinstrument 12 hochflexibel und/oder mobil eingesetzt werden. Das Smartphone kann entsprechende Software aufweisen, um das Bildgebungsinstrument 12 zu betreiben.

Der Datenübertragungsadapter 18 umfasst ein Anschlusselement 32, eine Dichtvorrichtung 28, eine Ladeschnittstelle 22, einen Energiespeicher 20, eine Steuereinheit 34, eine Datenschnittstelle 36, ein Bedienelement 38 und einen Steckeranschluss 26. Das Anschlusselement 32 ist wie ein Stecker des Datenkabels ausgebildet, der üblicherweise an den Steckeranschluss 14 des Bildgebungsinstruments 12 angeschlossen werden würde. Das Anschlusselement 32 ist steckerartig ausgebildet. Über das Anschlusselement 32 empfängt der Datenübertragungsadapter 18 Bilddaten von dem Bildgebungsinstrument 12 und leitet sie weiter zu der Steuereinheit 34. Mittels der Steuereinheit 34 werden die Bilddaten aufbereitet. Das kann ein Bereinigen, ein Komprimieren, ein Verschlüsseln, ein Verpacken in einzelne Pakete und/oder dergleichen umfassen. Insbesondere werden die Bilddaten derart aufbereitet, dass sie kabellos übertragbar sind. Daten, die auf den Bilddaten beruhen, insbesondere aufbereitete Bilddaten, werden an die Datenschnittstelle 36 geleitet. Mittels der Datenschnittstelle 36 können diese Daten kabellos an eine Empfangseinrichtung kabellos übertragen werden. Beispielsweise werden gemäß Fig. 1 die Daten an die Anzeigevorrichtung 78 übertragen und von dieser eine Darstellung 44, die auf den Daten, insbesondere Bilddaten, beruht, erzeugt. Ferner kann der Datenübertragungsadapter 18 zumindest ein Steuersignal über die Datenschnittstelle 36 kabellos empfangen, mittels der Steuereinheit 34 aufbereiten und über das Anschlusselement 32 an das Bildgebungsinstrument 12 übertragen. Der Benutzer kann etwa einen Befehl zur Bildaufnahme an einer Eingabevorrichtung 41, die an der Anzeigevorrichtung 40 angeordnet ist, erzeugen, der an den Datenübertragungsadapter 18 übertragen wird. Dadurch kann der Benutzer eine Funktion der Kamera 56 aktivieren.

Mittels des Energiespeichers 20 wird das Bildgebungsinstrument 12 während des Betriebs mit Energie, insbesondere elektrischer Energie, über den Steckeranschluss 14 versorgt. Vorliegend ist der Energiespeicher 20 ein wiederaufladbarer Energiespeicher 20, insbesondere ein Akkumulator, der in den Datenübertragungsadapter 18 integriert ist. Insbesondere ist der Energiespeicher 20 derart integriert, dass eine Montagekammer für den Energiespeicher 20 gasdicht und/oder fluiddicht zu der Umgebung verschließbar ist. Um den Energiespeicher 20 wieder aufladen zu können, umfasst der Datenübertragungsadapter 18 die Ladeschnittstelle 22. Die Ladeschnittstelle 22 ist zur induktiven Energieübertragung eingerichtet und zum induktiven Laden mittels einer induktiven Ladestation (nicht dargestellt) ausgebildet. Zusätzlich umfasst die Ladeschnittstelle 22 eine Buchse für einen Stecker eines Ladekabels 24. Das Ladekabel 24 kann wahlweise lösbar mit dem Datenübertragungsadapter 18 verbunden werden, um diesen zu laden.

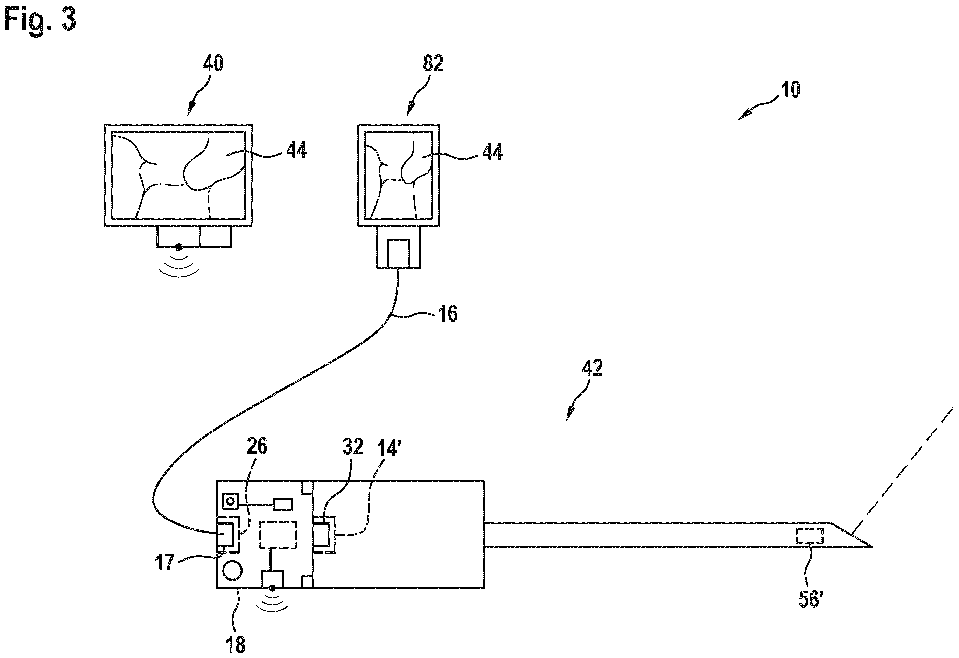

Der Steckeranschluss 26 des Datenübertragungsadapters 18 ist im Wesentlichen gleich wie der Steckeranschluss 14 des Bildgebungsinstruments ausgebildet. Das bedeutet, der Steckeranschluss 26 und der Steckeranschluss 14 haben eine im Wesentlichen gleiche Bauart. An den Steckeranschluss 26 kann das Datenkabel angeschlossen werden, das an den Steckeranschluss 14 angeschlossen werden kann. Dadurch kann der Datenübertragungsadapter 18 mit einer kabelgebundenen Anzeigevorrichtung und einer kabellosen Anzeigevorrichtung gleichzeitig betrieben werden (siehe Fig. 3).

Mittels des Bedienelements 38 lässt sich zudem beispielsweise eine Funktion des Bildgebungsinstruments 12 aktivieren und/oder steuern. Zudem kann mittels des Bedienelements 38 der Datenübertragungsadapter 18 in einen Kopplungsmodus zur Herstellung einer Kopplung mit einer Anzeigevorrichtung versetzbar sein und/oder eine Kopplung bestätigt werden. Darauf wird in dem Folgenden noch weiter eingegangen.

Die Dichtvorrichtung 28 ist dazu vorgesehen, dass der Datenübertragungsadapter 18 in einem an das Bildgebungsinstrument 12 angeschlossenem Zustand gemeinsam mit dem Bildgebungsinstrument 12 desinfiziert und/oder autoklaviert werden kann. In dem angeschlossenen und/oder verbundenen Zustand wird dazu mittels der Dichtvorrichtung 28 der Steckeranschluss 14 gegenüber einer Umgebung fluiddicht und/oder gasdicht abgedichtet. Die Dichtvorrichtung 28 ist als verformbare Gummierung entlang des gesamten Umfangs einer Kontaktfläche zu dem Bildgebungsinstrument 12 des Datenübertragungsadapter 18 vorgesehen. Bei dem Anschließen des Datenübertragungsadapters 18 wird die Gummierung mit einer Kraft beaufschlagt und dadurch verformt, sodass der Steckeranschluss gegenüber der Umgebung abgedichtet ist. Alternativ oder zusätzlich kann etwa auch ein O-Ring an dem Anschlusselement 32 vorgesehen werden.

Gemäß der schematischen Darstellung der Fig. 1 ist die weitere Anzeigevorrichtung 78 mit dem medizinischen Bildgebungsinstrument 12 gekoppelt und es werden auf den Bilddaten beruhende Daten an die Anzeigevorrichtung 78 übertragen. Die Anzeigevorrichtung ist dazu eingerichtet, anhand der Daten eine Darstellung 44 für den Benutzer zu erzeugen. Dadurch kann der Benutzer einen abgebildeten Objektbereich während eines Einsatzes des Bildgebungsinstruments 12 an der Anzeigevorrichtung 78 beobachten. Weitere Anzeigevorrichtungen 40, 43 können wahlweise zusätzlich mit dem Bildgebungsinstrument 12 gekoppelt werden und die Darstellung von diesen erzeugt werden und/oder an diesen angezeigt werden. Es kann also zumindest im Wesentlichen dieselbe Darstellung 44 auf verschiedenen Anzeigevorrichtungen 82 angezeigt werden.

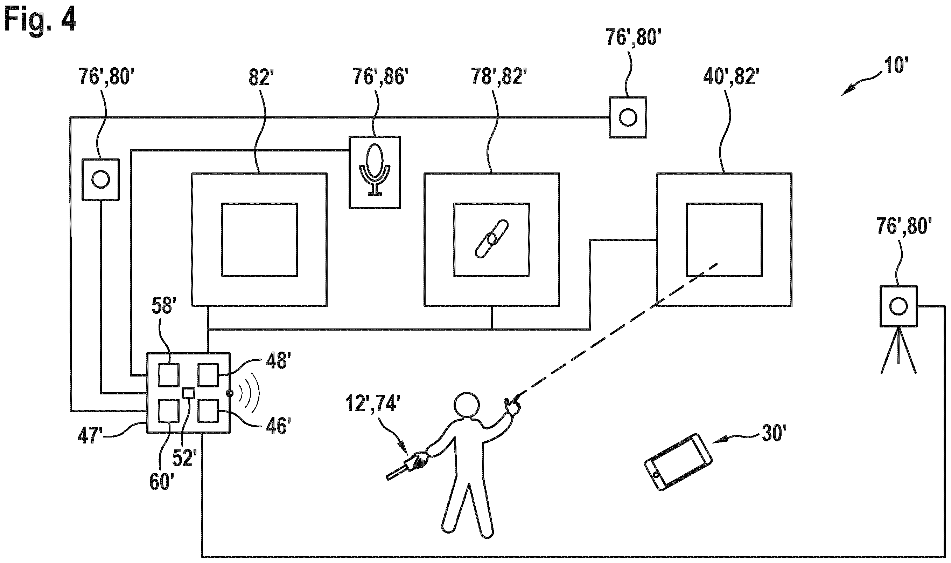

Für ein Koppeln zumindest einer der Anzeigevorrichtungen 40, 43 können verschiedene Verfahren und Methoden vorgesehen sein. Beispielsweise kann der Kopplungsmodus durch eine Betätigung des Bedienelements 38 des Datenübertragungsadapters 18 initiiert werden. Gemäß einer anderen der Methoden ist eine Gestenerkennungseinheit 76 vorgesehen. Diese wird nochmals im Zusammenhang mit der Fig. 4 genauer beschrieben. Die Gestenerkennungseinheit 76 umfasst mehrere Gestenerkennungsmodule 80, die jeweils an einer der Anzeigevorrichtungen 82, 40, 43, 78 angeordnet sind. Die Gestenerkennungsmodule 80 umfassen jeweils eine Kamera, die derart angeordnet sind und dazu eingerichtet sind, den Benutzer bei der Benutzung des Bildgebungsinstruments 12 abzubilden. Das Gestenerkennungsmodul 80 an der Anzeigevorrichtung 40 umfasst ein stereoskopisches Kamerasystem 84. Alternativ oder zusätzlich können die Gestenerkennungsmodule 80 gemeinsam das stereoskopische Kamerasystem 84 ausbilden. Die Gestenerkennungseinheit 76 ist dazu eingerichtet, eine auf die entsprechende Anzeigevorrichtung 40 gerichtete Geste des Benutzers zu erkennen, mit der der Benutzer das Bildgebungsinstrument 12 koppeln möchte, und nach Maßgabe der Erkennung der Geste eine Kopplung zwischen dem Bildgebungsinstrument 12 und der Anzeigevorrichtung 40 herzustellen. Dazu kann der Benutzer beispielsweise mit einer Hand in Richtung der zu koppelnden Anzeigevorrichtung 40 zeigen. Diese Geste wird, insbesondere mittels räumlicher Auflösung, durch die Gestenerkennungseinheit 76 erkannt. In gleicher Weise kann auch die weitere Anzeigevorrichtung 43 hinzukoppelbar sein. Ferner kann die Gestenerkennungseinheit 76 durch ein Hinzuschalten weiterer Gestenerkennungsmodule 80 erweiterbar sein. Diese können ebenfalls an weiteren Anzeigevorrichtungen angeordnet sein und/oder alleinstehende Gestenerkennungsmodule sein.

Gemäß dem Ausführungsbeispiel in der Fig. 1 wird die Kopplung mittels eines zweischrittigen Verfahrens durchgeführt. In dem ersten Schritt wird die Kopplung mittels der Gestenerkennungseinheit 76 initiiert, bzw. das Bildgebungsinstrument 12 und/oder der Datenübertragungsadapter 18 in einen Kopplungsmodus versetzt. Alternativ oder zusätzlich wird die Kopplung mittels der Betätigung des Bedienelements 38 des Datenübertragungsadapters 18 initiiert, bzw. das Bildgebungsinstrument 12 und/oder der Datenübertragungsadapter 18 in den Kopplungsmodus versetzt. In einem zweiten Schritt wird überprüft, ob die initiierte Kopplung eine gewollte Kopplung ist, also, ob tatsächlich das Bildgebungsinstrument 12 mit der Anzeigevorrichtung 40 gekoppelt werden soll. Dazu umfasst das Bildgebungsinstrument 12 eine Kopplungseinheit 46 und eine Sicherheitseinheit 48. Mittels der Kopplungseinheit 46 und der Sicherheitseinheit 48 kann grundsätzlich jede Kopplung überprüft werden, unabhängig davon, ob sie mittels der Gestenerkennungseinheit 76 initiiert wurde.

Die Kopplungseinheit 46 ist dazu eingerichtet, die Kopplung des Bildgebungsinstruments 12 und der Anzeigevorrichtung 40 herzustellen, wobei in einem gekoppelten Zustand die Bilddaten von dem Bildgebungsinstrument 12 an die Anzeigevorrichtung 40 übertragbar sind. Die Sicherheitseinheit 48 ist dazu eingerichtet, eine Bestätigung der Kopplung durch den Benutzer zu erkennen, wobei die Sicherheitseinheit 48 dazu eingerichtet ist, bei einem Fehlen der Bestätigung der Kopplung durch den Benutzer die Übertragung der Bilddaten von dem Bildgebungsinstrument 12 an die Anzeigevorrichtung 40 zu verhindern. Bleibt die Bestätigung durch den Benutzer aus, kann die Kopplung des Bildgebungsinstruments 12 und der Anzeigevorrichtung 40 gelöst werden.

Wenn das Vorliegen eines Kopplungsversuchs, insbesondere mittels der Kopplungseinheit 46, erkannt wird, erstellt die Sicherheitseinheit 48 eine Aufforderung 49 an den Benutzer zur Bestätigung der Kopplung. Gemäß der gezeigten Ausführungsform zeigt die Anzeigevorrichtung 40 dem Benutzer die Aufforderung 49 an. Dies kann ein Einblenden einer Aufforderungsanzeige über eine Darstellung der Anzeigevorrichtung 40 umfassen. Während des Kopplungsvorgangs sind das Bildgebungsinstrument 12 und die Anzeigevorrichtung 40 bereits derart gekoppelt, dass die Aufforderung 49 an die Anzeigevorrichtung 40 übertragbar ist. Zumindest Bilddaten und/oder auf den Bilddaten beruhende Daten werden jedoch nicht übertragen. Alternativ oder zusätzlich kann das Bildgebungsinstrument 12 zumindest eine LED (nicht dargestellt) und/oder einen Lautsprecher (nicht dargestellt) umfassen, mittels derer der Benutzer zur Bestätigung der Kopplung aufgefordert werden kann. Die LED kann etwa, insbesondere in einer Farbe, z.B. in Rot, blinken, um den Benutzer zu Bestätigung aufzufordern. In einem gekoppelten Zustand kann die LED in einer anderen Farbe und/oder Farbmischung leuchten, z.B. in Weiß. Mittels des Lautsprechers kann etwa eine Tonaufnahme einer gesprochenen Aufforderung und/oder ein Signalton ausgegeben werden.

Um die Kopplung zu bestätigen, betätigt der Benutzer das Bedienelement 38 des Datenübertragungsadapters 18 und/oder ein Bedienelement 38' des Bildgebungsinstruments 12. Das Bestätigen umfasst das Betätigen des Bedienelement 38, 38' über einen vorgegeben minimalen Bestätigungszeitabschnitt, wobei der Bestätigungszeitabschnitt vorzugsweise zumindest drei Sekunden und bevorzugt zumindest fünf Sekunden lang ist. Ist die Kopplung bestätigt, ist die Sicherheitseinheit 48 dazu eingerichtet, ein Übertragen der Bilddaten von dem Bildgebungsinstrument 12 an die Anzeigevorrichtung 40 zu gestatten. Der Benutzer hat in diesem Fall bestätigt, dass die richtige Anzeigevorrichtung mit dem richtigen Bildgebungsinstrument 12 gekoppelt wird.

Alternativ oder zusätzlich umfasst die Bestätigung der Kopplung ein Erfassen eines Bilds eines Erkennungsmerkmals 50 mittels des Bildgebungsinstruments 12. In einem solchen Fall erzeugt die Anzeigevorrichtung 40 ein Erkennungsmerkmal, anhand dessen die Anzeigevorrichtung 40 identifizierbar ist. Darauf wird im Folgenden noch genauer eingegangen. Die Darstellung des Erkennungsmerkmals kann nach der Einblendung der Aufforderung 49 erzeugbar sein und/oder gemeinsam mit der Aufforderung eingeblendet werden.

Möchte der Benutzer also das Bildgebungsinstrument 12 mit der Anzeigevorrichtung 40 koppeln, so kann er mittels einer Geste die Anzeigevorrichtung 40 auswählen. Daraufhin wird ihm eine Aufforderung 49 zur Bestätigung der Kopplung an der Anzeigevorrichtung 40 angezeigt. Zusätzlich wird an der Anzeigevorrichtung 40 ein Erkennungsmerkmal eingeblendet, von dem der Benutzer zur Bestätigung der Kopplung ein Bild mittels des Bildgebungsinstruments 12 erfasst. Alternativ kann der Benutzer die Kopplung durch die Betätigung des Bedienelements 38 initiieren und/oder bestätigen.

Alternativ oder zusätzlich kann die Kopplung durch Anwendung einer Zuordnungsregel verhindert und/oder gestattet werden. Dem Bildgebungsinstrument 12 ist dazu eine erste Gerätekennung und der Anzeigevorrichtung 40 eine zweite Gerätekennung zugeordnet. In der Kopplungseinheit 46 ist ferner die Zuordnungsregel hinterlegt, die Kombinationen unterschiedlicher Gerätekennungen als zulässig oder unzulässig festlegt. Alternativ oder zusätzlich kann die Zuordnungsregel auf einer Speichereinheit 52 einer zentralen Recheneinheit 47 hinterlegt sein. Die zentrale Recheneinheit 47 kann gemeinsam mit dem Bildgebungsinstrument 12 die Kopplungseinheit 46 ausbilden. Die Kopplungseinheit 46 ist dazu eingerichtet, unter Anwendung der Zuordnungsregel die erste Gerätekennung mit der zweiten Gerätekennung zu vergleichen und eine Kopplung des Bildgebungsinstruments 12 und der Anzeigevorrichtung 40 nach Maßgabe der Anwendung der Zuordnungsregel herzustellen oder zu verhindern. Wird also mittels der Geste eine der Anzeigevorrichtungen zur Herstellung der Kopplung ausgewählt, kann mittels der Zuordnungsregel überprüft werden, ob die gewünschte Kopplung herzustellen ist oder ob die Kopplung verhindert werden soll. Die Zuordnungsregel kann dazu voreingestellte Gerätekennungen von Bildgebungsinstrumenten und Anzeigevorrichtungen umfassen, die in einem bestimmten Operationssaal und/oder dergleichen vorhanden sind. Wird eine Gerätekennung erkannt, die diesen vorhandenen Geräten zuordenbar ist, wird die Kopplung gestattet. Andernfalls wird sie verhindert.

Ferner ist die Sicherheitseinheit 48 dazu eingerichtet, eine Benutzerberechtigung des Benutzers zu ermitteln, die Benutzerberechtigung mit einer vorgegebenen erforderlichen Berechtigung zu vergleichen und die Übertragung der Bilddaten von dem Bildgebungsinstrument 12 an die Anzeigevorrichtung 40 zu verhindern, wenn die Benutzerberechtigung die vorgegebene erforderliche Berechtigung nicht erreicht. Die Benutzerberechtigung kann beispielsweise auch auf der zentralen Recheneinheit 47 hinterlegt sein. Die zentrale Recheneinheit 47 ist etwa dazu vorgesehen, die Geräte eines Krankenhauses zu verwalten. Durch das Hinterlegen der Benutzerberechtigung kann dadurch im Vorhinein eine gewollte Paarung von Geräten eingestellt werden und/oder durch eine Zahlung freigeschaltet werden. Der Benutzer kann dazu in einer Software eine gewünschte Paarung auswählen und zur Erlangung der Benutzerberechtigung einen Betrag zahlen. Dadurch kann eine Abrechnungsmethode für eine Benutzung von Bildgebungsinstrumenten und Anzeigevorrichtungen implementiert werden, bei der jede Benutzung einzeln abgerechnet wird.

In der Speichereinheit 52 wird zudem Information über die Kopplung des Bildgebungsinstruments 12 mit der Anzeigevorrichtung 40 und/oder die Bestätigung der Kopplung durch den Benutzer hinterlegt. Dadurch kann etwa ermittelt werden, wie lange und wie oft welche Geräte verwendet wurden. Zudem kann Information über erfolglose Kopplungsversuche hinterlegt werden. Die Speichereinheit 52 kann insbesondere eine cloudbasierte Speichereinheit umfassen. Dadurch kann dezentral auf entsprechende Information zugegriffen werden.

Das Bildgebungsinstrument 12 umfasst außerdem eine Erkennungseinheit 58, die dazu eingerichtet ist, das Vorhandensein eines Erkennungsmerkmals 50 in einem mittels der Kamera 56 aufgenommenen Bild des Erkennungsmerkmals 50 zu erkennen und in Abhängigkeit des Erkennungsmerkmals 50 ein Erkennungssignal zu erzeugen, und eine Steuereinheit 60, die dazu eingerichtet ist, das Erkennungssignal zu verarbeiten und zumindest eine Funktion des medizinischen Bildgebungsinstruments 12 nach Maßgabe des Erkennungssignals zu aktivieren.

Die weitere Anzeigevorrichtung 43 ist dazu eingerichtet, das Erkennungsmerkmal 50 anzuzeigen. Das Erkennungsmerkmal 50 umfasst einen Farbcode 68 und ist derart beschaffen, dass es in einem Bild des Erkennungsmerkmals 50 erkennbar ist, das in einem defokussierten Zustand der Kamera 56 aufgenommen ist.

Die auf das Objekt 54 bezogene Funktion ist gemäß dem oben beschriebenen Fall die Kopplung des Bildgebungsinstruments 12 mit der Anzeigevorrichtung 40. Das Objekt 54, entsprechend die Anzeigevorrichtung 40, ist anhand des Erkennungsmerkmals 50 identifizierbar, wenn das Erkennungsmerkmal 50 zur Bestätigung der Kopplung angezeigt wird. Das Erkennungsmerkmal 50 kann in diesem Fall mit der Instrumentenkennung der Anzeigevorrichtung 40 verknüpft sein. Wird das Erkennungsmerkmal 50 also angezeigt, kann der Benutzer zur Durchführung der Kopplung des Bildgebungsinstruments 12, das er mit der Anzeigevorrichtung koppeln will, ein Bild der Erkennungsmerkmals 50 aufnehmen.

Gemäß der folgend beschriebenen Ausführungsform ist die Funktion eine Aktivierung einer scharfen, fokussierten Bildaufnahme mittels der Kamera 56. Vor der Aktivierung können mit der Kamera 56 lediglich Bilder derart aufgenommen werden, dass zumindest im Wesentlichen der gesamte Bildbereich unkenntlich ist. Dies liegt etwa daran, dass die Kamera 56 defokussiert ist und insbesondere auf einen sehr nahen oder sehr fernen Punkt fokussiert ist. Die Aktivierung kann durch eine Betätigung des Bedienelements 38, 38' initiiert werden. Dadurch wird das Anzeigen des Erkennungsmerkmal 50 auf der weiteren Anzeigevorrichtung 43 veranlasst. Der Benutzer kann daraufhin mittels der defokussierten Kamera 56 ein Bild des Erkennungsmerkmals 50 aufnehmen, wobei insbesondere keine scharfen Bilder eines Patienten vor der Aktivierung der scharfen Bildaufnahme erfasst werden. Wird das Erkennungsmerkmal 50 erkannt, wird die scharfe Bildaufnahme aktiviert und es kann eine diagnostische Aktion mittels der Bildgebungsinstruments 12 durchgeführt werden.

Das Objekt 54' kann zudem eine Patientenakte 70 umfassen. Die Patientenakte 70 weist das Erkennungsmerkmal 50', insbesondere einen QR-Code 66 und/oder Barcode 64, auf, anhand dessen die Patientenakte 70 und/oder der zu behandelnde Patient identifiziert werden kann. Wenn der Benutzer mittels der Kamera 56 des Bildgebungsinstruments 12 ein Bild des Erkennungsmerkmals 50' erzeugt, können mittels der Steuereinheit 60 beispielsweise Daten, insbesondere Bilddaten, die daraufhin erzeugt werden, dem Patienten und/oder der Patientenakte 70 zugeordnet werden, was eine auf das Objekt 54' bezogene Funktion ist. Die Patientenakte 70 kann insbesondere auch eine elektronische Patientenakte umfassen und das entsprechende Erkennungsmerkmal 50' an einer der Anzeigevorrichtungen 82 angezeigt werden. Zudem kann an einem Krankenbett des Patienten ein Namensschild (nicht dargestellt) angebracht sein, auf dem ein Erkennungsmerkmal, zum Beispiel ein QR-Code, angeordnet ist. Vor einer Benutzung des Bildgebungsinstruments 12 zur Durchführung einer therapeutischen und/oder diagnostischen Aktion kann der Benutzer ein Bild des QR-Codes erstellen. Darauffolgend durchgeführte Aktionen können dann dem Patienten zugeordnet werden und beispielsweise Information in der, insbesondere cloudbasierten, Speichereinheit 52 hinterlegt werden.

Gemäß dem Ausführungsbeispiel der Fig. 1 umfasst das Bildgebungsinstrument 12 die Erkennungseinheit 58, die Steuereinheit 60, die Kopplungseinheit 46 und die Sicherheitseinheit 48. Diese können gemeinsam auf einer Recheneinheit 35 des Bildgebungsinstruments 12 implementiert sein. Ein solches Bildgebungsinstrument 12 eignet sich insbesondere für mobile Anwendungen, beispielsweise in Verbindung mit dem mobilen Endgerät 30. Dieses kann zumindest teilweise die übrigen beschriebenen Merkmale aufweisen. Alternativ oder zusätzlich können diese Einheiten 46, 48, 58, 60 zumindest teilweise extern von dem Bildgebungsinstrument 12 angeordnet sein. Das weitere Bildgebungsinstrument 42 umfasst beispielsweise keine dieser Einheiten. Wird das weitere Bildgebungsinstrument 42 mittels des Datenübertragungsadapters 18 zur kabellosen Datenübertragung eingerichtet, können diese Einheiten auf einer externen Recheneinheit implementiert sein (siehe z.B. Fig. 4). Ferner versteht es sich, dass die einzelnen vorgehend beschriebenen Merkmale optional sind und eine Kopplung beispielsweise auch ohne die Erkennungseinheit 58 und/oder die Gestenerkennungseinheit 76 durchführbar ist.

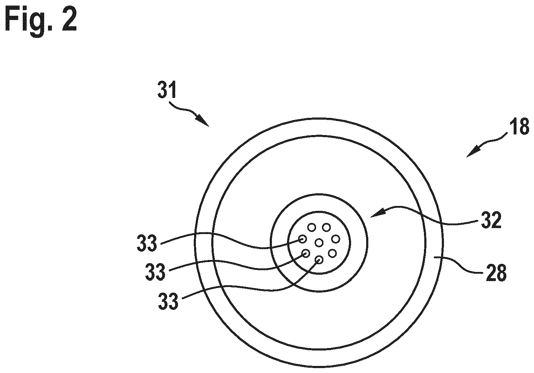

Fig. 2 zeigt eine schematische Darstellung einer Ansicht auf den Datenübertragungsadapter 18. Es ist die Kontaktfläche 31 des Datenübertragungsadapters 18 dargestellt, welche in einem an ein Bildgebungsinstrument angeschlossenen Zustand zumindest teilweise mit einer proximalen Endfläche des Bildgebungsinstruments in Kontakt steht. Entlang des Umfangs der Kontaktfläche 31 ist die Dichtvorrichtung 28 in Form der Gummierung angeordnet. Mittig in der Kontaktfläche 31 erstreckt sich das Anschlusselement 32 von der Kontaktfläche 31 weg. Das Anschlusselement 32 ist wie ein Stecker eines üblichen Datenkabels ausgebildet, das mit dem entsprechenden Bildgebungsinstrument verwendet wird. Beispielhaft ist das Anschlusselement 32 mit acht Pins 33 dargestellt, die jeweils in eine entsprechende Buchse des Steckeranschlusses 14 gesteckt werden, um den Datenübertragungsadapter 18 anzuschließen. Alternativ oder zusätzlich zu den Pins können auch Federkontaktstifte, beispielsweise Pogo-Pins, und entsprechende Kontaktflächen vorgesehen werden. Die Kontur der Kontaktfläche kann derart ausgebildet sein, dass sie einer Kontur der proximalen Endfläche eines Bildgebungsinstruments entspricht.

Fig. 3 zeigt eine schematische Darstellung eines Ausschnitts des medizinischen Systems 10 der Fig. 1. Der Datenübertragungsadapter 18 ist an das weitere medizinische Bildgebungsinstrument 42 angeschlossen. Dazu ist das Anschlusselement 32 des Datenübertragungsadapters in den Steckeranschluss 14' des Bildgebungsinstruments 42 gesteckt. Der Datenübertragungsadapter 18 ist zur kabellosen Datenübertragung mit der Anzeigevorrichtung 40 gekoppelt. Diese erzeugt die Darstellung 44 eines Objektbereichs, welcher mit der Kamera 56' des Bildgebungsinstruments 42 abgebildet wird. Zusätzlich ist eine weitere Anzeigevorrichtung 82 dargestellt. Diese Anzeigevorrichtung 82 ist eine alte, kabelgebundene Anzeigevorrichtung, die üblicherweise mit dem Bildgebungsinstrument 42 verwendet wurde. Das Bildgebungsinstrument 42 wurde jedoch mittels des Datenübertragungsadapters 18 zur kabellosen Datenübertragung eingerichtet. Zusätzlich ist der Datenübertragungsadapter 18 mittels des Datenkabels 16 mit der alten Anzeigevorrichtung 82 verbunden. Das Datenkabel 16, welches einen Stecker 17 umfasst, ist an die Anzeigevorrichtung 82 und mittels des Steckers 17 an den Steckeranschluss 26 Datenübertragungsadapter 18 angeschlossen. Der Stecker 17 ist in seiner Bauart wie das Anschlusselement 32 ausgebildet. Der Stecker 17 kann also auch in den Steckeranschluss 14' gesteckt werden. Ebenso ist der Steckeranschluss 26 in seiner Bauart wie der Steckeranschluss 14' ausgebildet. Auf der Anzeigevorrichtung 82 kann also zumindest im Wesentlichen die gleiche Darstellung 44 angezeigt werden. Eine Auflösung der Darstellung 44 und/oder ein Format der Darstellung 44 kann jedoch unterschiedlich sein. Zumindest beruhen die Darstellungen 44 jeder der Anzeigevorrichtungen 40, 82 auf denselben Bilddaten.

Fig. 4 zeigt eine schematische Darstellung einer weiteren Ausführungsform eines medizinischen Systems 10'. Das medizinische System weist große Gemeinsamkeiten mit dem System 10 auf. Die Bezugszeichen werden mit Hochkommata versehen, um eine Ähnlichkeit zu dem medizinischen System 10 hervorzuheben. Es wird vorrangig auf Unterschiede eingegangen. Die Funktion der einzelnen Einheiten kann gleich sein.

Im Unterschied zu der Fig. 1 sind die Kopplungseinheit 46', die Sicherheitseinheit 48', die Erkennungseinheit 58' und die Steuereinheit 60' in einer zentralen Recheneinheit 47' angeordnet, beziehungsweise auf dieser implementiert. Die Recheneinheit 57' umfasst zudem eine Speichereinheit 52'. Die Recheneinheit 57' ist ein Rechner in einem Operationssaal und/oder alternativ ein Server eines Krankenhauses. Der Rechner und der Server können sich zudem ergänzen und die Einheiten auf Rechenleistung beider zurückgreifen.

Die Recheneinheit 47' ist mit Anzeigevorrichtungen 82', 40' 78' verbunden. Zudem ist die Recheneinheit 47' zur kabellosen Datenübertragung eingerichtet. Beispielsweise können dadurch Steuerbefehle und/oder Signale der Einheiten 46', 48' 58' 60' an ein Bildgebungsinstrument 12' übertragen werden. Das Bildgebungsinstrument 12' ist ein Endoskop 74, das eine integrierte Datenschnittstelle aufweist. Alternativ kann das Bildgebungsinstrument 12' das Bildgebungsinstrument 12 der Fig. 1 sein und mittels des Datenübertragungsadapters 18 zur kabellosen Datenübertragung eingerichtet sein.

Die Anzeigevorrichtungen 82' sind untereinander verbunden und dazu eingerichtet, Daten auszutauschen. Das Bildgebungsinstrument 12' ist mit der Anzeigevorrichtung 78' gekoppelt. Wenn die Anzeigevorrichtung 40' ebenfalls mit dem Bildgebungsinstrument 12' gekoppelt wird, werden Bilddaten und/oder eine Darstellung betreffende Information von der weiteren Anzeigevorrichtung 78' an die Anzeigevorrichtung 40' übertragen. Beispielsweise wird eine Darstellung an der weiteren gekoppelten Anzeigevorrichtung 40 dupliziert. Ferner kann die die Darstellung betreffende Information auch an das mobile Endgerät 30' übertragen werden.

Ferner unterscheidet sich die Gestenerkennungseinheit 76' von der der Fig. 1. Die Gestenerkennungseinheit 76' umfasst mehrere eigenständige Gestenerkennungsmodule 80', die jeweils eine Kamera umfassen. Gemeinsam bilden die Gestenerkennungsmodule 80' ein stereoskopisches Kamerasystem aus. Dadurch kann der Benutzer aus mehreren Richtungen abgebildet werden und eine Geste des Benutzers räumlich interpretiert und/oder erkannt werden. Wenn der Benutzer, wie dargestellt, auf die Anzeigevorrichtung 40' zeigt, wird diese Geste durch die Gestenerkennungseinheit 76' erkannt und nach Maßgabe der Erkennung der Geste eine Kopplung zwischen dem Bildgebungsinstrument 12' und der Anzeigevorrichtung 40' hergestellt. Alternativ oder zusätzlich kann der Benutzer mit dem Bildgebungsinstrument 12' auf die Anzeigevorrichtung 40' zeigen. Wiederum alternativ oder zusätzlich können optische Marker (nicht dargestellt) an dem Bildgebungsinstrument 12' angeordnet sein, mittels derer eine Erkennung der Geste vereinfacht wird. Wiederum alternativ oder zusätzlich kann die Gestenerkennungseinheit 76' eine elektromagnetische Messeinrichtung (nicht dargestellt) und/oder das Bildgebungsinstrument 12' elektromagnetische Marker (nicht dargestellt) umfassen und die Geste anhand einer Erfassung der Position der elektromagnetischen Marker erkannt werden. Die Gestenerkennungseinheit 76' umfasst zudem ein Mikrofon 86', das dazu eingerichtet ist, ein Audiosignal zu empfangen. Die Gestenerkennungseinheit 76' ist dazu eingerichtet, das Audiosignal zu erkennen und nach Maßgabe der Erkennung des Audiosignals die Kopplung zwischen dem Bildgebungsinstrument 12' und der Anzeigevorrichtung 40' zu verhindern oder zu gestatten. Ferner kann ein Kopplungsmodus nach Maßgabe der Erkennung des Audiosignals aktivierbar sein. Der Benutzer kann beispielsweise ein Audiosignal erzeugen, um eine Kopplung zu bestätigen. Wird beispielsweise eine Kopplung durchgeführt, kann mittels der Sicherheitseinheit 60' eine Aufforderung zur Bestätigung der Kopplung erzeugt werden und der Benutzer mittels des Audiosignals die Kopplung bestätigen. Das Audiosignal kann ein Sprachbefehl sein und/oder ein willentlich erzeugtes Signal wie ein Klatschen. In gleicher Weise kann die weitere der Anzeigevorrichtungen 82' gekoppelt werden.

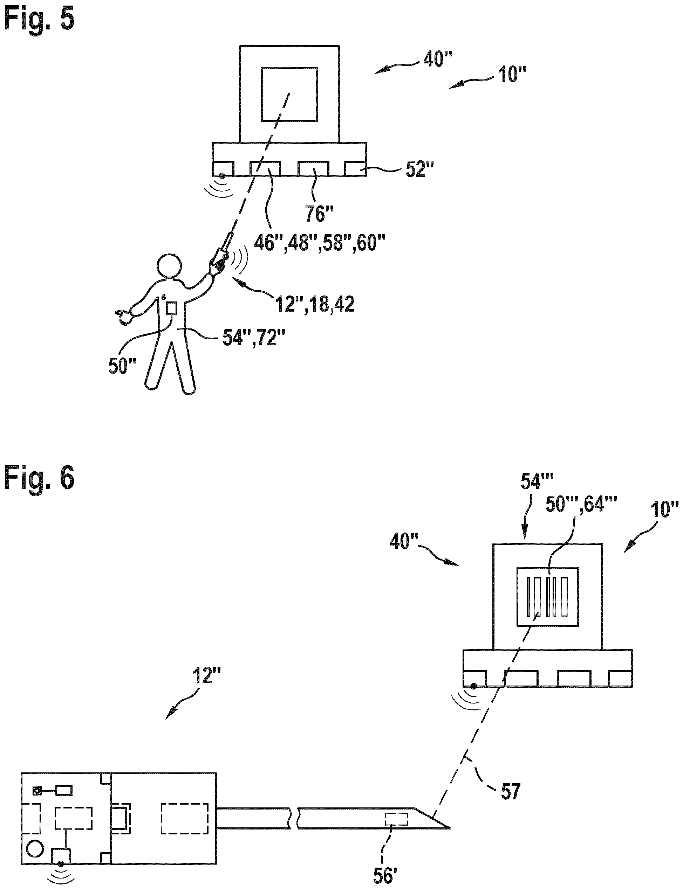

Fig. 5 zeigt eine schematische Darstellung einer weiteren Ausführungsform eines medizinischen Systems 10". Aufgrund der vielen Gemeinsamkeiten zu den medizinischen Systemen 10, 10' wird vorrangig auf Unterschiede eingegangen. Die Speichereinheit 52", die Gestenerkennungseinheit 76", die Kopplungseinheit 56", die Sicherheitseinheit 48", die Erkennungseinheit 5‴ und die Steuereinheit 60" sind an einer Anzeigevorrichtung 40" angeordnet. Ein Benutzer hält ein medizinisches Bildgebungsinstrument 12" in Richtung der Anzeigevorrichtung 40", was einer Geste zur Kopplung entspricht. Dadurch möchte der Benutzer das Bildgebungsinstrument 12" in einen Kopplungsmodus versetzen. Diese Geste wird mittels der Gestenerkennungseinheit 76" erkannt. Das Bildgebungsinstrument 12" ist das Bildgebungsinstrument 42 mit angeschlossenem Datenübertragungsadapter 18. Alternativ kann das Bildgebungsinstrument 12" auch ein anderes Bildgebungsinstrument sein, das zur kabellosen Datenübertragung eingerichtet ist. Zudem trägt der Benutzer ein Kleidungsstück 72", an dem ein Erkennungsmerkmal 50" angeordnet ist. Das Kleidungsstück 72" kann ein Objekt 54" sein und der Kleidungsstück 72" und/oder der Benutzer anhand eines Bilds des Erkennungsmerkmals 50" identifizierbar sein. Das Erkennungsmerkmal 50' ist mit einer Kennung des Benutzers verknüpft. Durch ein Aufnehmen des Bilds des Erkennungsmerkmals 50" kann ermittelt werden, dass der Benutzer das Bildgebungsinstrument 12" benutzt und in der Folge aufgenommene Bilddaten mit dem Benutzer verknüpft werden. Ferner kann eine Benutzerberechtigung zur Benutzung des Bildgebungsinstruments 12" überprüft werden. Information über den Benutzer, das Erkennungsmerkmal 50" und/oder dergleichen wird auf der Speichereinheit 52" hinterlegt.

Fig. 6 zeigt eine weitere schematische Darstellung des medizinischen Systems 10". Ein Objekt 54‴ umfasst die Anzeigevorrichtung 40", auf der ein Erkennungsmerkmal 50‴ in Form eines Barcodes 64‴ angezeigt wird. Alternativ oder zusätzlich kann das Erkennungsmerkmal 50‴ eine zeitliche Farbabfolge und/oder eine zeitliche Hell-Dunkel-Abfolge umfassen. Die Kamera 56' des Bildgebungsinstruments 12" definiert eine Blickrichtung 57. Zur Aktivierung einer auf das Objekt 54'" bezogenen Funktion des medizinischen Systems 10", vorliegend das Aktivieren einer scharfen, fokussierten/fokussierbaren Bildaufnahme mittels der Kamera 56', hält der Benutzer das Bildgebungsinstrument 12" derart, dass das Erkennungsmerkmal 50‴ in etwa in der Blickrichtung 57 des Bildgebungsinstruments 12" angeordnet ist. In diesem Zustand ist die Kamera 56' defokussiert und es ist keine scharfe Bildaufnahme möglich. Insbesondere wird keine auf Bilddaten beruhende Darstellung an einer Anzeigevorrichtung angezeigt. Dieser Zustand kann auch als Erkennungsmodus bezeichnet werden. Das Bildgebungsinstrument 12‴ kann durch eine Betätigung des Bedienelements 38 in diesen Modus versetzbar sein. Alternativ oder zusätzlich kann das Bildgebungsinstrument 12" grundsätzlich nach einem Einschalten in dem Erkennungsmodus sein. Der hat daher keine Rückmeldung, ob er das Bildgebungsinstrument 12" richtig hält. Zweckmäßig weist das Bildgebungsinstrument 12" jedoch eine Fokussierung auf einen sehr weit entfernten Punkt auf, sodass ein großer Bildbereich erfassbar ist. Sobald das Bild des Erkennungsmerkmals erfasst ist, bekommt der Benutzer Rückmeldung darüber, etwa, indem nach Erzeugung des Erkennungssignals eine Erkennungsnachricht in der Anzeigevorrichtung 40" angezeigt wird. Der Benutzer bewegt das Bildgebungsinstrument 12" so lange leicht bezüglich der Anzeigevorrichtung 40", bis die Erkennungsnachricht angezeigt wird. Nach Maßgabe der Erkennung des Vorhandenseins des Erkennungsmerkmals 50‴ wird die scharfe Bildaufnahme aktiviert und ein Patient kann untersucht werden.

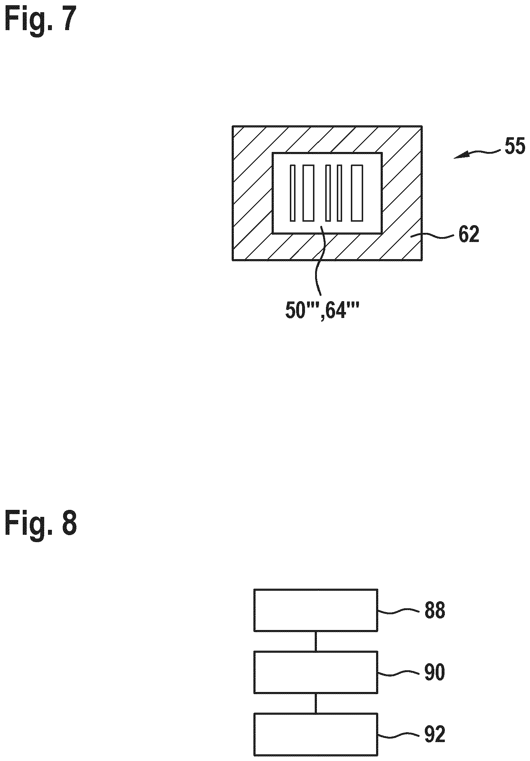

In der Fig. 7 ist eine schematische Darstellung des Bilds 55 des Erkennungsmerkmals 50‴, insbesondere des Barcodes 64‴, gezeigt. Das medizinische Bildgebungsinstrument 12" hat in dem Erkennungsmodus das Bild 55 des Erkennungsmerkmals 50‴ des Objekts 54'" derart erfasst und/oder zugehörige Bilddaten derart erzeugt, dass zumindest der Bildbereich 62, der das Erkennungsmerkmal 50‴ nicht enthält, unkenntlich ist. Insbesondere ist der Bildbereich 62 aufgrund der Defokussierung der Kamera 56' unkenntlich.

Fig. 8 zeigt ein schematisches Ablaufdiagramm eines Verfahrens zum Nachrüsten eines medizinischen Bildgebungsinstruments 12, 42, insbesondere mittels eines erfindungsgemäßen medizinischen Systems 10, 10', 10" und/oder mittels eines erfindungsgemäßen Datenübertragungsadapters 18. Das Verfahren umfasst einen Schritt 88 eines Bereitstellens eines medizinischen Bildgebungsinstruments 12, 42, das dazu eingerichtet ist, Bilddaten zu erzeugen und das einen Steckeranschluss 14 zum Anschließen eines Datenkabels 16 umfasst, einen Schritt 90 eines Bereitstellens eines Datenübertragungsadapters 18, der lösbar mit dem Steckeranschluss 14 verbindbar ist und dazu eingerichtet ist, die Bilddaten über den Steckeranschluss 14 zu empfangen und kabellos zu übertragen, und einen Schritt 92 eines Verbindens des Datenübertragungsadapters 18 mit dem Steckeranschluss 14 des Bildgebungsinstruments 12, 42.

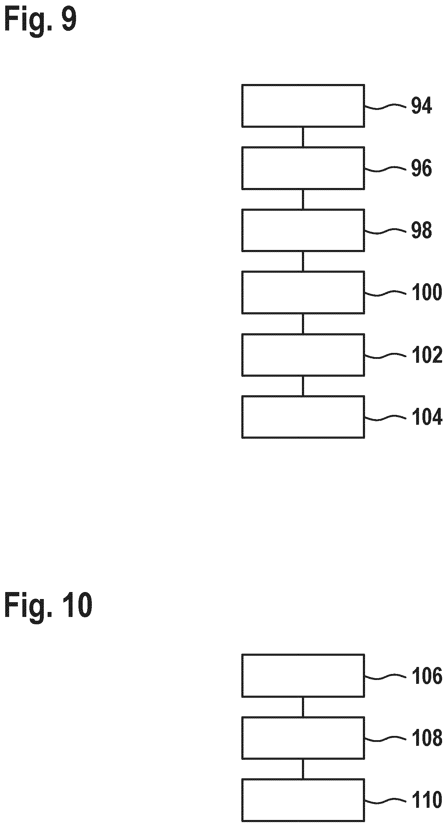

Fig. 9 zeigt ein schematisches Ablaufdiagramm eines Verfahrens zum Übertragen von Bilddaten, insbesondere mittels eines erfindungsgemäßen medizinischen Systems 10, 10', 10" und/oder mittels eines erfindungsgemäßen Datenübertragungsadapters 18. Das Verfahren umfasst einen Schritt 94 eines Bereitstellens eines medizinischen Bildgebungsinstruments 12, das dazu eingerichtet ist, Bilddaten zu erzeugen und das einen Steckeranschluss 14 zum Anschließen eines Datenkabels 16 umfasst, einen Schritt 96 eines Bereitstellens eines Datenübertragungsadapters 18, der lösbar mit dem Steckeranschluss 14 verbindbar ist und dazu eingerichtet ist, die Bilddaten über den Steckeranschluss 14 zu empfangen und kabellos zu übertragen, einen Schritt 98 eines Verbindens des Datenübertragungsadapters 18 mit dem Steckeranschluss 14 des Bildgebungsinstruments 12, einen Schritt 100 eines Erzeugen von Bilddaten mittels des Bildgebungsinstruments 12, einen Schritt 102 eines Übertragens der Bilddaten über den Steckeranschluss 14 zu dem Datenübertragungsadapter 18 und einen Schritt 104 eines kabellosen Übertragens der Bilddaten mittels des Datenübertragungsadapters 18.

Fig. 10 zeigt ein schematisches Ablaufdiagramm eines Verfahrens zur Verbindung eines medizinischen Bildgebungsinstruments 12 mit einer Anzeigevorrichtung 40, insbesondere durchgeführt mittels eines erfindungsgemäßen medizinischen Systems 10, 10', 10" und/oder eines erfindungsgemäßen medizinischen Bildgebungsinstruments 12, wobei das medizinische Bildgebungsinstrument 12 dazu eingerichtet ist, Bilddaten zu erzeugen, und wobei die Anzeigevorrichtung 40 dazu eingerichtet ist, Bilddaten zu empfangen und eine Darstellung 44 für einen Benutzer zu erzeugen. Das Verfahren umfasst einen Schritt 106 eines Herstellens einer Kopplung des Bildgebungsinstruments 12 und der Anzeigevorrichtung 40, wobei in einem gekoppelten Zustand die Bilddaten von dem Bildgebungsinstrument 12 an die Anzeigevorrichtung 40 übertragbar sind, einen Schritt 108 eines Erkennens einer Bestätigung der Kopplung durch den Benutzer und einen Schritt 110 eines Verhinderns der Übertragung der Bilddaten von dem Bildgebungsinstrument 12 an die Anzeigevorrichtung 40 bei einem Fehlen der Bestätigung der Kopplung durch den Benutzer.

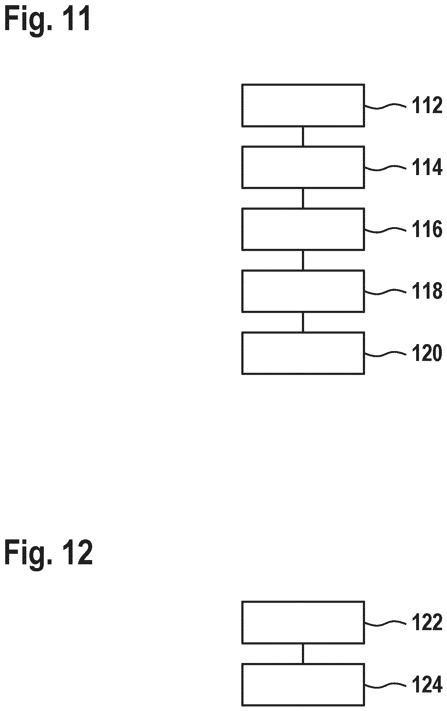

Fig. 11 zeigt ein schematisches Ablaufdiagramm eines Verfahrens mit einem erfindungsgemäßen medizinischen System 10, 10', 10", wobei das medizinische System 10, 10', 10" ein Objekt 54, das ein Erkennungsmerkmal 50 aufweist und das anhand des Erkennungsmerkmals 50 identifizierbar ist, und ein medizinisches Bildgebungsinstrument 12 mit einer Kamera 56, mittels derer Bilder erfassbar und Bilddaten erzeugbar sind, wobei mittels der Kamera 56 ein Bild des Erkennungsmerkmals 50 des Objekts 54 erfassbar ist, umfasst. Zudem kann das Objekt 54 eine Anzeigevorrichtung 40 umfassen, die dazu eingerichtet ist, Bilddaten zu empfangen und eine Darstellung für einen Benutzer zu erzeugen. Das Verfahren umfasst einen Schritt 112 eines Erfassens eines Bilds des Erkennungsmerkmals 50 des Objekts 54 mittels der Kamera 56, einen Schritt 114 eines Erkennens des Vorhandenseins des Erkennungsmerkmals 50 in dem mittels der Kamera 56 aufgenommenen Bild des Erkennungsmerkmals 50, einen Schritt 116 eines Erzeugens eines Erkennungssignals nach Maßgabe der Erkennung des Vorhandenseins des Erkennungsmerkmals 50 und einen Schritt 118 eines Aktivierens zumindest einer auf das Objekt 54 bezogenen Funktion des medizinischen Systems 10 nach Maßgabe des Erkennungssignals. Zudem kann das Verfahren einen Schritt 120 eines Anzeigens des Erkennungsmerkmals 50 auf der Anzeigevorrichtung 40 umfassen.

Fig. 12 zeigt ein schematisches Ablaufdiagramm eines Verfahrens zum Koppeln eines medizinischen Bildgebungsinstruments 12 mit einer Anzeigevorrichtung 40, insbesondere mit einem erfindungsgemäßen medizinischen System 10, 10', 10", wobei das medizinische Bildgebungsinstrument 12 dazu eingerichtet ist, Bilddaten zu erzeugen und die Bilddaten kabellos zu übertragen, und wobei die Anzeigevorrichtung 40 dazu eingerichtet ist, Bilddaten zu empfangen und eine Darstellung 44 für einen Benutzer zu erzeugen. Das Verfahren umfasst einen Schritt 122 eines Erkennens einer auf die Anzeigevorrichtung 40 gerichteten Geste des Benutzers und einen Schritt eines Herstellens einer Kopplung zwischen dem Bildgebungsinstrument 12 und der Anzeigevorrichtung 40 nach Maßgabe der Erkennung der Geste.

### Bezugszeichenliste

- 10: medizinisches System
- 12: medizinisches Bildgebungsinstrument
- 13: handhabbares Bildgebungsinstrument
- 14: Steckeranschluss
- 16: Datenkabel
- 17: Stecker
- 18: Datenübertragungsadapter
- 20: Energiespeicher
- 22: Ladeschnittstelle
- 24: Ladekabel
- 26: Steckeranschluss
- 28: Dichtvorrichtung
- 30: mobiles Endgerät
- 31: Kontaktfläche
- 32: Anschlusselement
- 33: Pin
- 34: Steuereinheit
- 35: Recheneinheit
- 36: Datenschnittstelle
- 38: Bedienelement
- 40: Anzeigevorrichtung
- 41: Eingabevorrichtung
- 42: weiteres medizinisches Bildgebungsinstrument
- 43: weitere Anzeigevorrichtung
- 44: Darstellung
- 46: Kopplungseinheit
- 47: zentrale Recheneinheit
- 48: Sicherheitseinheit
- 49: Aufforderung
- 50: Erkennungsmerkmal
- 52: Speichereinheit
- 54: Objekt
- 56: Kamera
- 57: Blickrichtung
- 58: Erkennungseinheit
- 60: Steuereinheit
- 62: Bildbereiche
- 64: Barcode
- 66: QR-Code
- 68: Farbcode
- 70: Patientenakte
- 72: Kleidungsstück
- 74: Endoskop
- 76: Gestenerkennungseinheit
- 78: weitere Anzeigevorrichtung
- 80: Gestenerkennungsmodul
- 82: Anzeigevorrichtungen
- 84: stereoskopisches Kamerasystem
- 86: Mikrofon
- 88: Schritt
- 90: Schritt
- 92: Schritt
- 94: Schritt
- 96: Schritt
- 98: Schritt
- 100: Schritt
- 102: Schritt
- 104: Schritt
- 106: Schritt
- 108: Schritt
- 110: Schritt
- 112: Schritt
- 114: Schritt
- 116: Schritt
- 118: Schritt
- 120: Schritt
- 122: Schritt
- 124: Schritt

Die Offenbarung betrifft unter anderem die folgenden Aspekte:
Aspekt 1. Medizinisches System (10) umfassend:
   ein medizinisches Bildgebungsinstrument (12), das dazu eingerichtet ist, Bilddaten zu erzeugen und das einen Steckeranschluss (14) zum Anschließen eines Datenkabels (16) umfasst; und
   einen Datenübertragungsadapter (18), der lösbar mit dem Steckeranschluss (14) verbindbar ist und dazu eingerichtet ist, die Bilddaten über den Steckeranschluss (14) zu empfangen und auf den Bilddaten beruhende Daten kabellos zu übertragen.
Aspekt 2. Medizinisches System (10) nach Aspekt 1,
   wobei der Datenübertragungsadapter (18) einen Energiespeicher (20) umfasst, der dazu eingerichtet ist, das medizinische Bildgebungsinstrument (12) während eines Betriebs über den Steckeranschluss (14) mit Energie zu versorgen.
Aspekt 3. Medizinisches System (10) nach Aspekt 2,
   wobei der Datenübertragungsadapter (18) eine Ladeschnittstelle (22) umfasst, mittels derer der Energiespeicher (20) aufladbar ist.
Aspekt 4. Medizinisches System (10) nach Aspekt 3,
   wobei die Ladeschnittstelle (22) zur induktiven Energieübertragung eingerichtet ist.
Aspekt 5. Medizinisches System (10) nach Aspekt 3 oder 4,
   wobei die Ladeschnittstelle (22) zum lösbaren Verbinden eines Ladekabels (24) eingerichtet ist.
Aspekt 6. Medizinisches System (10) nach einem der vorhergehenden Aspekte,
   wobei der Datenübertragungsadapter (18) einen Steckeranschluss (26) zum Anschließen eines Datenkabels (16) umfasst.
Aspekt 7. Medizinisches System (10) nach Aspekt 6,
   wobei der Steckeranschluss (26) des Datenübertragungsadapters (18) in seiner Bauart einer Bauart des Steckeranschlusses (14) des Bildgebungsinstruments (12) entspricht.
Aspekt 8. Medizinisches System (10) nach einem der vorhergehenden Aspekte,
   wobei der Datenübertragungsadapter (18) dazu eingerichtet ist, desinfiziert und/oder autoklaviert zu werden.
Aspekt 9. Medizinisches System (10) nach einem der vorhergehenden Aspekte,
   dazu eingerichtet, in einem Zustand zumindest teilweise desinfiziert und/oder autoklaviert zu werden, in dem der Datenübertragungsadapter (18) mit dem medizinischen Bildgebungsinstrument (12) verbunden ist.
Aspekt 10. Medizinisches System (10) nach einem der vorhergehenden Aspekte,
   wobei der Datenübertragungsadapter (18) eine Dichtvorrichtung (28) umfasst, die dazu eingerichtet ist, in einem Zustand, in dem der Datenübertragungsadapter (18) mit dem medizinischen Bildgebungsinstrument (12) verbunden ist, den Steckeranschluss (14) des Bildgebungsinstruments (12) gegenüber einer Umgebung, insbesondere fluiddicht und/oder gasdicht, abzudichten.
Aspekt 11. Medizinisches System (10) nach einem der vorhergehenden Aspekte,
   wobei der Datenübertragungsadapter (18) dazu eingerichtet ist, die Bilddaten vor dem kabellosen Übertragen aufzubereiten.
Aspekt 12. Medizinisches System (10) nach einem der vorhergehenden Aspekte,
   wobei der Datenübertragungsadapter (18) dazu eingerichtet ist, die Bilddaten unmittelbar an ein mobiles Endgerät (30) kabellos zu übertragen.
Aspekt 13. Medizinisches System (10) nach einem der vorhergehenden Aspekte,
   wobei der Datenübertragungsadapter (18) dazu eingerichtet ist, zumindest ein Steuersignal kabellos zu empfangen und das Steuersignal über den Steckeranschluss (14) an das Bildgebungsinstrument (12) zu übertragen.
Aspekt 14. Medizinisches System (10) nach einem der vorhergehenden Aspekte,
   wobei in einem Zustand, in dem der Datenübertragungsadapter (18) mit dem medizinischen Bildgebungsinstrument (12) verbunden ist, der Datenübertragungsadapter (18) und das Bildgebungsinstrument (12) gemeinsam ein von einem Benutzer als einzelnes Instrument handhabbares Bildgebungsinstrument (13) ausbilden.
Aspekt 15. Medizinisches System (10) nach einem der vorhergehenden Aspekte,
   wobei der Datenübertragungsadapter (18) umfasst:
   ein Anschlusselement (32), das lösbar mit dem Steckeranschluss (14) verbindbar ist;
   eine Steuereinheit (34), die mit dem Anschlusselement (32) verbunden ist und die dazu eingerichtet ist, die Bilddaten von dem Anschlusselement (32) zu empfangen; und
   eine Datenschnittstelle (36), die mit der Steuereinheit (34) verbunden ist und die dazu eingerichtet ist, Daten von der Steuereinheit (34) zu empfangen und Daten kabellos zu übertragen.
Aspekt 16. Medizinisches System (10) nach einem der vorhergehenden Aspekte,
   wobei der Datenübertragungsadapter (18) ein Bedienelement (38) umfasst, mittels dessen der Datenübertragungsadapter (18) in einen Kopplungsmodus zur Herstellung einer Kopplung mit einer Anzeigevorrichtung (40) versetzbar ist.
Aspekt 17. Medizinisches System (10) nach einem der vorhergehenden Aspekte,
   ferner umfassend ein weiteres medizinisches Bildgebungsinstrument (42), wobei der Datenübertragungsadapter (18) wahlweise mit dem Bildgebungsinstrument (12) oder dem weiteren Bildgebungsinstrument (42) verbindbar ist.
Aspekt 18. Datenübertragungsadapter (18) für ein medizinisches System (10) nach einem der vorhergehenden Aspekte.
Aspekt 19. Datenübertragungsadapter (18), insbesondere für ein medizinisches System (10) nach einem der Aspekte 1 bis 17, umfassend:
   ein Anschlusselement (32), das lösbar mit einem Steckeranschluss (14) eines medizinischen Bildgebungsinstruments verbindbar ist;
   eine Steuereinheit (34), die mit dem Anschlusselement (32) verbunden ist und die dazu eingerichtet ist, Bilddaten von dem Anschlusselement (32) zu empfangen; und
   eine Datenschnittstelle (36), die mit der Steuereinheit (34) verbunden ist und die dazu eingerichtet ist, Daten von der Steuereinheit (34) zu empfangen und Daten kabellos zu übertragen.
Aspekt 20. Verfahren zum Nachrüsten eines medizinischen Bildgebungsinstruments (12, 42), insbesondere mittels eines medizinischen Systems (10) nach einem der Aspekte 1 bis 17 und/oder mittels eines Datenübertragungsadapters (18) nach Aspekt 18 oder 19, umfassend:
   Bereitstellen eines medizinischen Bildgebungsinstruments (12, 42), das dazu eingerichtet ist, Bilddaten zu erzeugen und das einen Steckeranschluss (14) zum Anschließen eines Datenkabels (16) umfasst;
   Bereitstellen eines Datenübertragungsadapters (18), der lösbar mit dem Steckeranschluss (14) verbindbar ist und dazu eingerichtet ist, die Bilddaten über den Steckeranschluss (14) zu empfangen und kabellos zu übertragen; und
   Verbinden des Datenübertragungsadapters (18) mit dem Steckeranschluss (14) des Bildgebungsinstruments (12, 42).
Aspekt 21. Verfahren zum Übertragen von Bilddaten, insbesondere mittels eines medizinischen Systems (10) nach einem der Aspekte 1 bis 17 und/oder mittels eines Datenübertragungsadapters (18) nach Aspekt 18 oder 19, umfassend:
   Bereitstellen eines medizinischen Bildgebungsinstruments (12), das dazu eingerichtet ist, Bilddaten zu erzeugen und das einen Steckeranschluss (14) zum Anschließen eines Datenkabels (16) umfasst;
   Bereitstellen eines Datenübertragungsadapters (18), der lösbar mit dem Steckeranschluss (14) verbindbar ist und dazu eingerichtet ist, die Bilddaten über den Steckeranschluss (14) zu empfangen und kabellos zu übertragen;
   Verbinden des Datenübertragungsadapters (18) mit dem Steckeranschluss (14) des Bildgebungsinstruments (12);
   Erzeugen von Bilddaten mittels des Bildgebungsinstruments (12);
   Übertragen der Bilddaten über den Steckeranschluss (14) zu dem Datenübertragungsadapter (18); und
   kabelloses Übertragen der Bilddaten mittels des Datenübertragungsadapters (18).
Aspekt 22. Verfahren zum Betrieb eines medizinischen Systems (10) nach einem der Aspekte 1 bis 17 und/oder eines medizinischen Bildgebungsinstruments (12) nach Aspekt 18 oder 19.
Aspekt 23. Medizinisches System (10) umfassend:
   ein medizinisches Bildgebungsinstrument (12), das dazu eingerichtet ist, Bilddaten zu erzeugen;
   eine Anzeigevorrichtung (40), die dazu eingerichtet ist, Bilddaten zu empfangen und eine Darstellung (44) für einen Benutzer zu erzeugen;
   eine Kopplungseinheit (46), die dazu eingerichtet ist, eine Kopplung des Bildgebungsinstruments (12) und der Anzeigevorrichtung (40) herzustellen, wobei in einem gekoppelten Zustand die Bilddaten von dem Bildgebungsinstrument (12) an die Anzeigevorrichtung (40) übertragbar sind; und
   eine Sicherheitseinheit (48), die dazu eingerichtet ist, eine Bestätigung der Kopplung durch den Benutzer zu erkennen, wobei die Sicherheitseinheit (48) dazu eingerichtet ist, bei einem Fehlen der Bestätigung der Kopplung durch den Benutzer die Übertragung der Bilddaten von dem Bildgebungsinstrument (12) an die Anzeigevorrichtung (40) zu verhindern.
Aspekt 24. Medizinisches System (10) nach Aspekt 23,
   wobei das Verhindern der Übertragung der Bilddaten ein Lösen der Kopplung des Bildgebungsinstruments (12) und der Anzeigevorrichtung (40) umfasst.
Aspekt 25. Medizinisches System (10) nach einem der vorhergehenden Aspekte, insbesondere nach einem der Aspekte 23 bis 24,
   wobei die Sicherheitseinheit (48) dazu eingerichtet ist, eine Aufforderung (49) an den Benutzer zur Bestätigung der Kopplung zu erstellen.
Aspekt 26. Medizinisches System (10) nach Aspekt 25,
   wobei die Anzeigevorrichtung (40) dazu eingerichtet ist, die Aufforderung (49) dem Benutzer anzuzeigen.
Aspekt 27. Medizinisches System (10) nach einem der vorhergehenden Aspekte, insbesondere nach einem der Aspekte 23 bis 26,
   wobei das medizinische Bildgebungsinstrument (12) ein Bedienelement (38) umfasst, und
   wobei die Bestätigung der Kopplung ein Betätigen des Bedienelements (38) umfasst.
Aspekt 28. Medizinisches System (10) nach Aspekt 27,
   wobei die Bestätigung der Kopplung ein Betätigen des Bedienelements (38) über einen vorgegeben minimalen Bestätigungszeitabschnitt umfasst, wobei der Bestätigungszeitabschnitt vorzugsweise zumindest drei Sekunden und bevorzugt zumindest fünf Sekunden lang ist.
Aspekt 29. Medizinisches System (10) nach einem der vorhergehenden Aspekte, insbesondere nach einem der Aspekte 23 bis 28,
   wobei die Sicherheitseinheit (48) dazu eingerichtet ist, bei einem Vorliegen der Bestätigung der Kopplung ein Übertragen der Bilddaten von dem Bildgebungsinstrument (12) an die Anzeigevorrichtung (40) zu gestatten.
Aspekt 30. Medizinisches System (10) nach einem der vorhergehenden Aspekte, insbesondere nach einem der Aspekte 23 bis 29,
   wobei die Anzeigevorrichtung (40) ein Erkennungsmerkmal (50) aufweist und anhand des Erkennungsmerkmals (50) identifizierbar ist,
   wobei die Bestätigung der Kopplung ein Erfassen eines Bilds des Erkennungsmerkmals (50) mittels des Bildgebungsinstruments (12) umfasst.
Aspekt 31. Medizinisches System (10) nach Aspekt 30,
   wobei die Anzeigevorrichtung (40) dazu eingerichtet ist, das Erkennungsmerkmal (50) anzuzeigen.
Aspekt 32. Medizinisches System (10) nach einem der vorhergehenden Aspekte, insbesondere nach einem der Aspekte 23 bis 31,
   wobei dem Bildgebungsinstrument (12) eine erste Gerätekennung zugeordnet ist, wobei der Anzeigevorrichtung (40) eine zweite Gerätekennung zugeordnet ist, wobei in der Kopplungseinheit (46) eine Zuordnungsregel hinterlegt ist, die Kombinationen unterschiedlicher Gerätekennungen als zulässig oder unzulässig festlegt, und wobei die Kopplungseinheit (46) dazu eingerichtet ist, unter Anwendung der Zuordnungsregel die erste Gerätekennung mit der zweiten Gerätekennung zu vergleichen und eine Kopplung des Bildgebungsinstruments (12) und der Anzeigevorrichtung (40) nach Maßgabe der Anwendung der Zuordnungsregel herzustellen oder zu verhindern.
Aspekt 33. Medizinisches System (10) nach einem der vorhergehenden Aspekte, insbesondere nach einem der Aspekte 23 bis 32,
   wobei die Sicherheitseinheit (48) dazu eingerichtet ist, eine Benutzerberechtigung des Benutzers zu ermitteln, die Benutzerberechtigung mit einer vorgegebenen erforderlichen Berechtigung zu vergleichen, und die Übertragung der Bilddaten von dem Bildgebungsinstrument (12) an die Anzeigevorrichtung (40) zu verhindern, wenn die Benutzerberechtigung die vorgegebene erforderliche Berechtigung nicht erreicht.
Aspekt 34. Medizinisches System (10) nach einem der vorhergehenden Aspekte, insbesondere nach einem der Aspekte 23 bis 33,
   ferner umfassend eine Speichereinheit (52), in der Information über die Kopplung des Bildgebungsinstruments (12) mit der Anzeigevorrichtung (40) und/oder die Bestätigung der Kopplung durch den Benutzer hinterlegt ist.
Aspekt 35. Medizinisches Bildgebungsinstrument (12) für ein medizinisches System (10) nach einem der vorhergehenden Aspekte, insbesondere nach einem der Aspekte 23 bis 34.
Aspekt 36. Medizinisches Bildgebungsinstrument (12), insbesondere für ein medizinisches System (10) nach einem der Aspekte 23 bis 34, umfassend:
   eine Kopplungseinheit (46), die dazu eingerichtet ist, eine Kopplung des Bildgebungsinstruments (12) mit einer Anzeigevorrichtung (40) herzustellen, wobei in einem gekoppelten Zustand die Bilddaten von dem Bildgebungsinstrument (12) an die Anzeigevorrichtung (40) übertragbar sind; und
   eine Sicherheitseinheit (48), die dazu eingerichtet ist, eine Bestätigung der Kopplung durch den Benutzer zu erkennen, wobei die Sicherheitseinheit (48) dazu eingerichtet ist, bei einem Fehlen der Bestätigung der Kopplung durch den Benutzer die Übertragung der Bilddaten von dem Bildgebungsinstrument (12) an die Anzeigevorrichtung (40) zu verhindern.
Aspekt 37. Verfahren zum Betrieb eines medizinischen Systems (10) nach einem der Aspekte 23 bis 34 und/oder eines medizinischen Bildgebungsinstruments (12) nach Aspekt 35 oder 36.
Aspekt 38. Verfahren zur Verbindung eines medizinischen Bildgebungsinstruments (12) mit einer Anzeigevorrichtung (40), insbesondere durchgeführt mittels eines medizinischen Systems (10) nach einem der Aspekte 23 bis 34 und/oder eines medizinischen Bildgebungsinstruments (12) nach Aspekt 35 oder 36,
   wobei das medizinische Bildgebungsinstrument (12) dazu eingerichtet ist, Bilddaten zu erzeugen; und
   wobei die Anzeigevorrichtung (40) dazu eingerichtet ist, Bilddaten zu empfangen und eine Darstellung (44) für einen Benutzer zu erzeugen;
   umfassend die Schritte:
      Herstellen einer Kopplung des Bildgebungsinstruments (12) und der Anzeigevorrichtung (40), wobei in einem gekoppelten Zustand die Bilddaten von dem Bildgebungsinstrument (12) an die Anzeigevorrichtung (40) übertragbar sind;
      Erkennen einer Bestätigung der Kopplung durch den Benutzer; und
      Verhindern der Übertragung der Bilddaten von dem Bildgebungsinstrument (12) an die Anzeigevorrichtung (40) bei einem Fehlen der Bestätigung der Kopplung durch den Benutzer.
Aspekt 39. Medizinisches System (10) umfassend:
   ein Objekt (54), das ein Erkennungsmerkmal (50) aufweist und das anhand des Erkennungsmerkmals (50) identifizierbar ist;
   ein medizinisches Bildgebungsinstrument (12) mit einer Kamera (56), mittels derer Bilder erfassbar und Bilddaten erzeugbar sind, wobei mittels der Kamera (56) ein Bild des Erkennungsmerkmals (50) des Objekts (54) erfassbar ist;
   eine Erkennungseinheit (58), die dazu eingerichtet ist, das Vorhandensein des Erkennungsmerkmals (50) in dem mittels der Kamera (56) aufgenommenen Bild des Erkennungsmerkmals (50) zu erkennen und nach Maßgabe der Erkennung des Vorhandenseins des Erkennungsmerkmals (50) ein Erkennungssignal zu erzeugen; und
   eine Steuereinheit (60), die dazu eingerichtet ist, das Erkennungssignal zu verarbeiten und zumindest eine auf das Objekt (54) bezogene Funktion des medizinischen Systems (10) nach Maßgabe des Erkennungssignals zu aktivieren.
Aspekt 40. Medizinisches System (10) nach Aspekt 39,
   wobei das Objekt (54) eine Anzeigevorrichtung (40) umfasst, die dazu eingerichtet ist, Bilddaten zu empfangen und eine Darstellung (44) für einen Benutzer zu erzeugen.
Aspekt 41. Medizinisches System (10) nach Aspekt 40,
   wobei die Anzeigevorrichtung (40) dazu eingerichtet ist, das Erkennungsmerkmal (50) anzuzeigen.
Aspekt 42. Medizinisches System (10) nach einem der vorhergehenden Aspekte, insbesondere nach einem der Aspekte 39 bis 41,
   wobei die auf das Objekt (54) bezogene Aktion eine Kopplung der Bildgebungsinstruments (12) mit der Anzeigevorrichtung (40) ist.
Aspekt 43. Medizinisches System (10) nach einem der vorhergehenden Aspekte, insbesondere nach einem der Aspekte 39 bis 42,
   wobei das medizinische Bildgebungsinstrument (12) dazu eingerichtet ist, in einem Erkennungsmodus das Bild (55) des Erkennungsmerkmals (50) des Objekts (54) derart zu erfassen und/oder zugehörige Bilddaten derart zu erzeugen, dass zumindest Bildbereiche (62), die das Erkennungsmerkmal (50) nicht enthalten, unkenntlich sind.
Aspekt 44. Medizinisches System (10) nach einem der vorhergehenden Aspekte, insbesondere nach einem der Aspekte 39 bis 43,
   wobei die Bildbereiche (62) aufgrund einer Defokussierung der Kamera (56) unkenntlich sind.
Aspekt 45. Medizinisches System (10) nach einem der vorhergehenden Aspekte, insbesondere nach einem der Aspekte 39 bis 44,
   wobei das Erkennungsmerkmal (50) derart beschaffen ist, dass es in einem Bild des Erkennungsmerkmals (50) erkennbar ist, das in einem defokussierten Zustand der Kamera (56) aufgenommen ist.
Aspekt 46. Medizinisches System (10) nach einem der vorhergehenden Aspekte, insbesondere nach einem der Aspekte 39 bis 45,
   wobei das Erkennungsmerkmal (50) einen Barcode (64) umfasst.
Aspekt 47. Medizinisches System (10) nach einem der vorhergehenden Aspekte, insbesondere nach einem der Aspekte 39 bis 46,
   wobei das Erkennungsmerkmal (50) einen QR-Code (66) umfasst.
Aspekt 48. Medizinisches System (10) nach einem der vorhergehenden Aspekte, insbesondere nach einem der Aspekte 39 bis 47,
   wobei das Erkennungsmerkmal (50) einen Farbcode (68) umfasst.
Aspekt 49. Medizinisches System (10) nach einem der vorhergehenden Aspekte, insbesondere nach einem der Aspekte 39 bis 48,
   wobei das Erkennungsmerkmal (50) eine zeitliche Farbabfolge umfasst.
Aspekt 50. Medizinisches System (10) nach einem der vorhergehenden Aspekte, insbesondere nach einem der Aspekte 39 bis 49,
   wobei das Erkennungsmerkmal (50) eine zeitliche Hell-Dunkel-Abfolge umfasst.
Aspekt 51. Medizinisches System (10) nach einem der vorhergehenden Aspekte, insbesondere nach einem der Aspekte 39 bis 50,
   wobei das Erkennungsmerkmal (50) mit einer Instrumentenkennung verknüpft ist.
Aspekt 52. Medizinisches System (10) nach einem der vorhergehenden Aspekte, insbesondere nach einem der Aspekte 39 bis 51,
   wobei das Objekt (54) eine Patientenakte (70) umfasst.
Aspekt 53. Medizinisches System (10) nach einem der vorhergehenden Aspekte, insbesondere nach einem der Aspekte 39 bis 52,
   wobei das Objekt (54) ein Kleidungsstück (72) umfasst.
Aspekt 54. Medizinisches Bildgebungsinstrument (12) für ein medizinisches System (10) nach einem der vorhergehenden Aspekte, insbesondere nach einem der Aspekte 39 bis 53.
Aspekt 55. Medizinisches Bildgebungsinstrument (12), insbesondere für ein medizinisches System (10) nach einem der Aspekte 39 bis 53, insbesondere Endoskop (74), umfassend:
   eine Kamera (56), mittels derer Bilder erfassbar und Bilddaten erzeugbar sind;
   eine Erkennungseinheit (58), die dazu eingerichtet ist, das Vorhandensein eines Erkennungsmerkmals (50) in einem mittels der Kamera (56) aufgenommenen Bilds des Erkennungsmerkmals (50) zu erkennen und in Abhängigkeit des Erkennungsmerkmals (50) ein Erkennungssignal zu erzeugen; und
   eine Steuereinheit (60), die dazu eingerichtet ist, das Erkennungssignal zu verarbeiten und zumindest eine Funktion des medizinischen Bildgebungsinstruments (12) nach Maßgabe des Erkennungssignals zu aktivieren.
Aspekt 56. Verfahren zum Betrieb eines medizinischen Systems (10) nach einem der Aspekte 39 bis 53 und/oder eines medizinischen Bildgebungsinstruments (12) nach Aspekt 54 oder 55.
Aspekt 57. Verfahren mit einem medizinischen System (10), insbesondere nach einem der Aspekte 39 bis 53,
   wobei das medizinische System (10) umfasst:
   ein Objekt (54), das ein Erkennungsmerkmal (50) aufweist und das anhand des Erkennungsmerkmals (50) identifizierbar ist; und
   ein medizinisches Bildgebungsinstrument (12) mit einer Kamera (56), mittels derer Bilder erfassbar und Bilddaten erzeugbar sind, wobei mittels der Kamera (56) ein Bild des Erkennungsmerkmals (50) des Objekts (54) erfassbar ist;
   umfassend den Schritt:
      Erfassen eines Bilds des Erkennungsmerkmals (50) des Objekts (54) mittels der Kamera (56).
Aspekt 58. Verfahren nach Aspekt 57,
   ferner umfassend die Schritte:
   Erkennen des Vorhandenseins des Erkennungsmerkmals (50) in dem mittels der Kamera (56) aufgenommenen Bild des Erkennungsmerkmals (50);
   Erzeugen eines Erkennungssignals nach Maßgabe der Erkennung des Vorhandenseins des Erkennungsmerkmals (50); und
   Aktivieren zumindest einer auf das Objekt (54) bezogenen Funktion des medizinischen Systems (10) nach Maßgabe des Erkennungssignals.
Aspekt 59. Verfahren nach Aspekt 57 oder 58,
   wobei das Objekt (54) eine Anzeigevorrichtung (40) umfasst, die dazu eingerichtet ist, Bilddaten zu empfangen und eine Darstellung für einen Benutzer zu erzeugen;
   ferner umfassend den Schritt:
      Anzeigen des Erkennungsmerkmals (50) auf der Anzeigevorrichtung (40).
Aspekt 60. Medizinisches System (10) umfassend:
   ein medizinisches Bildgebungsinstrument (12), das dazu eingerichtet ist, Bilddaten zu erzeugen und die Bilddaten kabellos zu übertragen;
   eine Anzeigevorrichtung (40), die dazu eingerichtet ist, Bilddaten zu empfangen und eine Darstellung (44) für einen Benutzer zu erzeugen; und
   eine Gestenerkennungseinheit (76), die dazu eingerichtet ist, eine auf die Anzeigevorrichtung (40) gerichtete Geste des Benutzers zu erkennen und nach Maßgabe der Erkennung der Geste eine Kopplung zwischen dem Bildgebungsinstrument (12) und der Anzeigevorrichtung (40) herzustellen.
Aspekt 61. Medizinisches System (10) nach Aspekt 60,
   wobei die Gestenerkennungseinheit (76) an der Anzeigevorrichtung (40) angeordnet ist.
Aspekt 62. Medizinisches System (10) nach Aspekt 60 oder 61, ferner umfassend:
   eine weitere Anzeigevorrichtung (78), die dazu eingerichtet ist, Bilddaten zu empfangen und eine Darstellung (44) für einen Benutzer zu erzeugen, wobei die weitere Anzeigevorrichtung (78) mit dem medizinischen Bildgebungsinstrument (12) gekoppelt ist,
   wobei die Gestenerkennungseinheit (76) dazu eingerichtet ist, die auf die Anzeigevorrichtung (40) gerichtete Geste des Benutzers zu erkennen und nach Maßgabe der Erkennung der Geste eine Kopplung zwischen dem Bildgebungsinstrument (12) und der Anzeigevorrichtung (40) zusätzlich zu der Kopplung mit der weiteren Anzeigevorrichtung (78) derart herzustellen, dass auf den Bilddaten beruhende Darstellungen (44) sowohl auf der Anzeigevorrichtung (40) als auch auf der weiteren Anzeigevorrichtung (78) anzeigbar sind.
Aspekt 63. Medizinisches System (10) nach Aspekt 62,
   wobei die Gestenerkennungseinheit (76) an der weiteren Anzeigevorrichtung (78) angeordnet ist.
Aspekt 64. Medizinisches System (10) nach Aspekt 62 oder 63,
   wobei das Bildgebungsinstrument (12) und die Anzeigevorrichtung (40) derart koppelbar sind, dass die Bilddaten und/oder die Darstellung (44) betreffende Information von der weiteren Anzeigevorrichtung (78) an die Anzeigevorrichtung (40) übertragbar sind.
Aspekt 65. Medizinisches System (10) nach einem der vorhergehenden Aspekte, insbesondere nach einem der Aspekte 60 bis 64,
   wobei die Gestenerkennungseinheit (76) zumindest ein Gestenerkennungsmodul (80) umfasst und/oder durch Zuschalten weiterer Gestenerkennungsmodule (80) erweiterbar ist.
Aspekt 66. Medizinisches System (10) nach einem der vorhergehenden Aspekte, insbesondere nach einem der Aspekte 60 bis 65, umfassend:
   mehrere Anzeigevorrichtungen (82), die jeweils dazu eingerichtet sind, Bilddaten zu empfangen und eine Darstellung (44) für einen Benutzer zu erzeugen,
   wobei die Gestenerkennungseinheit (76) dazu eingerichtet ist, eine auf eine bestimmte Anzeigevorrichtung (40) der Anzeigevorrichtungen (82) gerichtete Geste des Benutzers zu erkennen und nach Maßgabe der Erkennung der auf die bestimmte Anzeigevorrichtung (40) der Anzeigevorrichtungen (82) gerichteten Geste eine Kopplung zwischen dem Bildgebungsinstrument (12) und der bestimmten Anzeigevorrichtung (40) der Anzeigevorrichtungen (82) herzustellen.
Aspekt 67. Medizinisches System (10) nach einem der vorhergehenden Aspekte, insbesondere nach einem der Aspekte 60 bis 66,
   wobei die Gestenerkennungseinheit (76) ein stereoskopisches Kamerasystem (84) umfasst.
Aspekt 68. Medizinisches System (10) nach einem der vorhergehenden Aspekte, insbesondere nach einem der Aspekte 60 bis 67, ferner umfassend:
   ein Mikrofon (86), das dazu eingerichtet ist, ein Audiosignal zu empfangen,
   wobei die Gestenerkennungseinheit (76) dazu eingerichtet ist, das Audiosignal zu erkennen und nach Maßgabe der Erkennung des Audiosignals die Kopplung zwischen dem Bildgebungsinstrument (12) und der Anzeigevorrichtung (40) zu verhindern oder zu gestatten.
Aspekt 69. Verfahren zum Betrieb eines medizinischen Systems (10) nach einem der vorhergehenden Aspekte, insbesondere nach einem der Aspekte 60 bis 68.
Aspekt 70. Verfahren zum Koppeln eines medizinischen Bildgebungsinstruments (12) mit einer Anzeigevorrichtung (40), insbesondere mit einem medizinischen System (10) nach einem der Aspekte 60 bis 68,
   wobei das medizinische Bildgebungsinstrument (12) dazu eingerichtet ist, Bilddaten zu erzeugen und die Bilddaten kabellos zu übertragen; und
   wobei die Anzeigevorrichtung (40) dazu eingerichtet ist, Bilddaten zu empfangen und eine Darstellung (44) für einen Benutzer zu erzeugen;
   umfassend die Schritte:
      Erkennen einer auf die Anzeigevorrichtung (40) gerichteten Geste des Benutzers; und
      Herstellen einer Kopplung zwischen dem Bildgebungsinstrument (12) und der Anzeigevorrichtung (40) nach Maßgabe der Erkennung der Geste.

## Patentansprüche

1. Medizinisches System (10) umfassend:
ein medizinisches Bildgebungsinstrument (12), das dazu eingerichtet ist, Bilddaten zu erzeugen und die Bilddaten kabellos zu übertragen;
eine Anzeigevorrichtung (40), die dazu eingerichtet ist, Bilddaten zu empfangen und eine Darstellung (44) für einen Benutzer zu erzeugen; und
eine Gestenerkennungseinheit (76), die dazu eingerichtet ist, eine auf die Anzeigevorrichtung (40) gerichtete Geste des Benutzers zu erkennen und nach Maßgabe der Erkennung der Geste eine Kopplung zwischen dem Bildgebungsinstrument (12) und der Anzeigevorrichtung (40) herzustellen.

2. Medizinisches System (10) nach Anspruch 1,
wobei die Gestenerkennungseinheit (76) an der Anzeigevorrichtung (40) angeordnet ist.

3. Medizinisches System (10) nach Anspruch 1 oder 2, ferner umfassend:
eine weitere Anzeigevorrichtung (78), die dazu eingerichtet ist, Bilddaten zu empfangen und eine Darstellung (44) für einen Benutzer zu erzeugen, wobei die weitere Anzeigevorrichtung (78) mit dem medizinischen Bildgebungsinstrument (12) gekoppelt ist,
wobei die Gestenerkennungseinheit (76) dazu eingerichtet ist, die auf die Anzeigevorrichtung (40) gerichtete Geste des Benutzers zu erkennen und nach Maßgabe der Erkennung der Geste eine Kopplung zwischen dem Bildgebungsinstrument (12) und der Anzeigevorrichtung (40) zusätzlich zu der Kopplung mit der weiteren Anzeigevorrichtung (78) derart herzustellen, dass auf den Bilddaten beruhende Darstellungen (44) sowohl auf der Anzeigevorrichtung (40) als auch auf der weiteren Anzeigevorrichtung (78) anzeigbar sind.

4. Medizinisches System (10) nach Anspruch 3,
wobei die Gestenerkennungseinheit (76) an der weiteren Anzeigevorrichtung (78) angeordnet ist.

5. Medizinisches System (10) nach Anspruch 3 oder 4,
wobei das Bildgebungsinstrument (12) und die Anzeigevorrichtung (40) derart koppelbar sind, dass die Bilddaten und/oder die Darstellung (44) betreffende Information von der weiteren Anzeigevorrichtung (78) an die Anzeigevorrichtung (40) übertragbar sind.

6. Medizinisches System (10) nach einem der vorhergehenden Ansprüche,
wobei die Gestenerkennungseinheit (76) zumindest ein Gestenerkennungsmodul (80) umfasst und/oder durch Zuschalten weiterer Gestenerkennungsmodule (80) erweiterbar ist.

7. Medizinisches System (10) nach einem der vorhergehenden Ansprüche, umfassend:
mehrere Anzeigevorrichtungen (82), die jeweils dazu eingerichtet sind, Bilddaten zu empfangen und eine Darstellung (44) für einen Benutzer zu erzeugen,
wobei die Gestenerkennungseinheit (76) dazu eingerichtet ist, eine auf eine bestimmte Anzeigevorrichtung (40) der Anzeigevorrichtungen (82) gerichtete Geste des Benutzers zu erkennen und nach Maßgabe der Erkennung der auf die bestimmte Anzeigevorrichtung (40) der Anzeigevorrichtungen (82) gerichteten Geste eine Kopplung zwischen dem Bildgebungsinstrument (12) und der bestimmten Anzeigevorrichtung (40) der Anzeigevorrichtungen (82) herzustellen.

8. Medizinisches System (10) nach einem der vorhergehenden Ansprüche,
wobei die Gestenerkennungseinheit (76) ein stereoskopisches Kamerasystem (84) umfasst.

9. Medizinisches System (10) nach einem der vorhergehenden Ansprüche, ferner umfassend:
ein Mikrofon (86), das dazu eingerichtet ist, ein Audiosignal zu empfangen,
wobei die Gestenerkennungseinheit (76) dazu eingerichtet ist, das Audiosignal zu erkennen und nach Maßgabe der Erkennung des Audiosignals die Kopplung zwischen dem Bildgebungsinstrument (12) und der Anzeigevorrichtung (40) zu verhindern oder zu gestatten.

10. Verfahren zum Betrieb eines medizinischen Systems (10) nach einem der vorhergehenden Ansprüche.

11. Verfahren zum Koppeln eines medizinischen Bildgebungsinstruments (12) mit einer Anzeigevorrichtung (40), insbesondere mit einem medizinischen System (10) nach einem der Ansprüche 1 bis 9,
wobei das medizinische Bildgebungsinstrument (12) dazu eingerichtet ist, Bilddaten zu erzeugen und die Bilddaten kabellos zu übertragen; und
wobei die Anzeigevorrichtung (40) dazu eingerichtet ist, Bilddaten zu empfangen und eine Darstellung (44) für einen Benutzer zu erzeugen;
umfassend die Schritte:
Erkennen einer auf die Anzeigevorrichtung (40) gerichteten Geste des Benutzers; und
Herstellen einer Kopplung zwischen dem Bildgebungsinstrument (12) und der Anzeigevorrichtung (40) nach Maßgabe der Erkennung der Geste.
